# EUROPEAN PATENT SPECIFICATION

(11) **EP 4 426 684 B1**
(45) Date of publication and mention of the grant of the patent: **17.09.2025**
(21) Application number: 22813491.2
(22) Date of filing: 02.11.2022
(51) Int. Cl.: C07D 317/26, A61K 8/49, C07D 319/06, A61Q 13/00, C07D 263/06, C07D 265/06

(54) **CYCLIC ACETALS AND KETALS FOR THE LIGHT-INDUCED RELEASE OF ACTIVE ALDEHYDES AND KETONES**
CYCLISCHE ACETALE UND KETALE ZUR LICHTINDUZIERTEN FREISETZUNG VON AKTIVEN ALDEHYDEN UND KETONEN
ACÉTALS ET CÉTALS CYCLIQUES POUR LA LIBÉRATION DE D'ALDÉHYDES ET DE CÉTONES ACTIFS INDUITE PAR LA LUMIÈRE

(30) Priority: 03.11.2021 US 202163275186 P; 11.11.2021 EP 21207675
(43) Date of publication of application: 11.09.2024
(73) Proprietor: Firmenich SA, 1242 Satigny (CH)
(72) Inventor: WOMACK, Gary, Plainsboro, New Jersey 08536 (US); HERRMANN, Andreas, 1242 Satigny (FR)
(74) Representative: Strych, Sebastian
(86) International application number: PCT/EP2022/080523
(87) International publication number: WO 2023/078909

(56) References cited:
- EP-A1- 1 262 473
- WO-A2-01/96272
- DERRER SAMUEL ET AL: "Applied Photochemistry - Light Controlled Perfume Release", vol. 61, no. 10, 24 October 2007 (2007-10-24), CH, pages 665, XP055916491, ISSN: 0009-4293, Retrieved from the Internet <URL:https://chimia.ch/chimia/article/download/4387/3677> DOI: 10.2533/chimia.2007.665
- LAN YUN ET AL: "An iron-catalyzed hydroalkylation reaction of [alpha],[beta]-unsaturated ketones with ethers", vol. 53, no. 91, 1 January 2017 (2017-01-01), UK, pages 12353 - 12356, XP055916357, ISSN: 1359-7345, Retrieved from the Internet <URL:https://pubs.rsc.org/en/content/articlepdf/2017/cc/c7cc07235j> DOI: 10.1039/C7CC07235J
- RUEPING MAGNUS ET AL: "Effective synthesis of 2,5-disubstituted tetrahydrofurans from glycerol by catalytic alkylation of ketones", vol. 14, no. 1, 1 January 2012 (2012-01-01), GB, pages 55 - 57, XP055916432, ISSN: 1463-9262, Retrieved from the Internet <URL:https://pubs.rsc.org/en/content/articlepdf/2012/gc/c1gc15764g> DOI: 10.1039/C1GC15764G

## Description

### Technical field

The present invention relates to compounds of formula (I), a delivery system based on photoresponsive cyclic acetal or ketal compounds capable of liberating in a controlled manner active volatile carbonyl compounds into the surrounding upon exposure to light. Furthermore, the present invention relates to the use of said compounds in perfumery, as well as the perfuming compositions or perfumed consumer products comprising the invention's compounds.

### Background

The perfumery industry has a particular interest for compositions or additives which are capable of prolonging or enhancing the perfuming effect of a mixture of several fragrances at the same time over a certain period of time. It is particularly desirable to obtain long-lasting properties for standard perfumery raw materials which are too volatile or have a poor substantivity by themselves, or which are only deposited in a small amount onto the surface of the final application. Furthermore, some of the perfumery ingredients, especially aldehydes, are unstable and need to be protected against slow degradation prior to their use. Long-lasting perfumes are desirable for various applications, as for example fine or functional perfumery or cosmetic preparations. The washing and softening of textiles is a particular field in which there is a constant quest to enable the effect of active substances, in particular perfumes, to be effective for a certain period of time after washing, softening and drying. Indeed, many substances having odors which are particularly suitable for this type of application are known to lack tenacity on laundry, or do not remain on the laundry when rinsed, with the result that their perfuming effect is experienced only briefly and not very intensely. Given the importance of this type of application in the perfume industry, research in this field has been sustained, in particular with the aim of finding new, and more effective solutions to the aforementioned problems.

It has now been surprisingly found that the photolabile cyclic acetals or ketals according to the present invention solve the above-mentioned problems and are capable of efficiently liberating several active volatile carbonyl compounds upon exposure to light in numerous practical applications. To the best of our knowledge, none of the prior art documents suggests, or allows to expect, that the photosensitive acetal and ketal compounds of formula (I) could indeed be suitable as delivery systems for the controlled release of volatile compounds.

### Brief Description of the Drawings

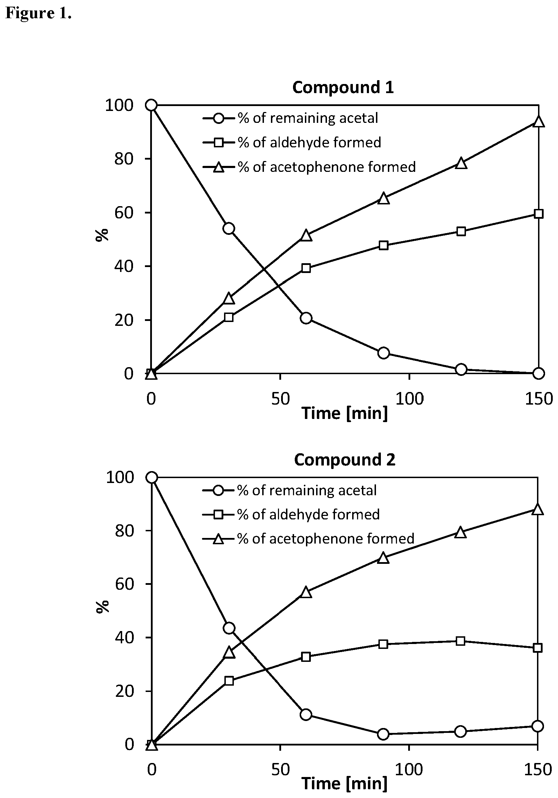

Figure 1: Light-induced release of a phenyl ketone derivative of formula (III) and a carbonyl compound of formula (II) (aldehyde or ketone) from a cyclic acetal or ketal according to formula (I) upon photoirradiation with a xenon lamp followed by ¹H-NMR spectroscopy. Compound 1 (top) releases acetophenone and benzaldehyde, Compound 2 (bottom) releases acetophenone and undecanal.

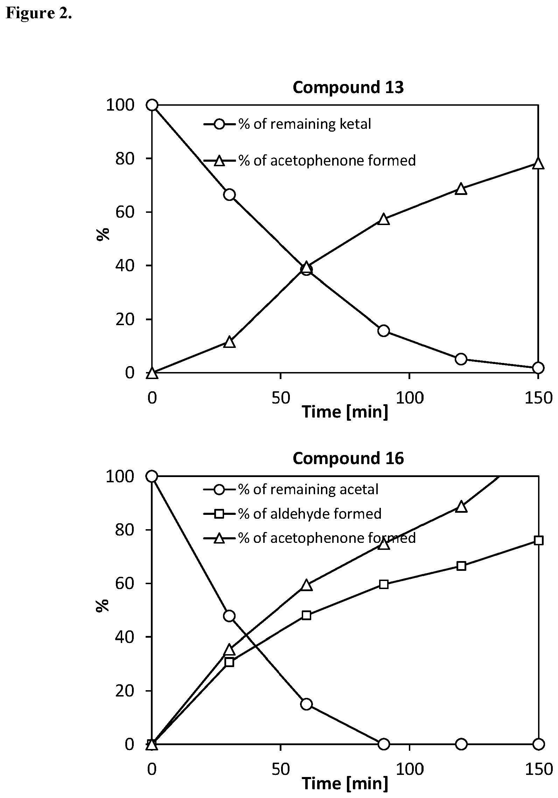

Figure 2: Light-induced release of a phenyl ketone derivative of formula (III) and a carbonyl compound of formula (II) (aldehyde or ketone) from a cyclic acetal or ketal according to formula (I) upon photoirradiation with a xenon lamp followed by ¹H-NMR spectroscopy. Compound 13 (top) releases two molecules of acetophenone (the recorded ¹H-NMR integrals of the acetophenone released were adjusted to correspond to two molar equivalents), Compound 16 (bottom) releases acetophenone and benzaldehyde.

### Description of the invention

It has now been discovered that the compounds of formula (I) can advantageously be employed as a delivery system to release an active volatile aldehyde or ketone together with an active volatile phenyl ketone derivative from a given surface into the surrounding environment upon exposure to light.

Therefore, a first object of the present invention is a compound of formula 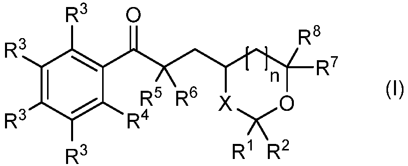 in the form of any one of its stereoisomers or a mixture thereof, and wherein
n is 0 or 1;
X is an oxygen atom or a NR⁹ group wherein R⁹ is a hydrogen atom or a methyl group;
R¹ represents a C₁-₁₈ hydrocarbon group, optionally comprising one to three oxygen atoms and/or one to two nitrogen atoms and/or one sulfur atom; R² represents, a hydrogen atom or a R¹ group; or R¹ and R², when taken together, form a C₅₋₁₆ cycloalkyl, C₅₋₁₆ cycloalkenyl, C₄₋₁₄ heterocycloalkyl or C₄₋₁₄ heterocycloalkenyl group, each optionally substituted with one or more of a C₁₋₁₅ alkyl, C₂₋₁₅ alkenyl, C₁₋₁₅ alkoxy, C₃₋₁₅ cycloalkyl, C₅₋₁₅ cycloalkenyl, C₆₋₁₀ aryl and/or C₆₋₁₀ aryloxy group, each optionally substituted with one or more of a C₁₋₈ alkyl, C₁₋₈ alkoxy, carboxylic acid and/or C₁₋₄ carboxylic ester group, wherein the heteroatom represents one or more of an oxygen atom;
R³ and R⁴ represent, independent of each other, a hydrogen atom, a C₁₋₆ alkoxy group or a C₁₋₁₂ alkyl group, optionally substituted by a hydroxy, C₁₋₆ alkoxy or oxo group, or, two adjacent R³ groups, when taken together, are a C₃₋₈ linear alkanediyl group optionally substituted by one or more of a hydroxy, C₁₋₃ alkyl and/or C₁₋₃ alkoxy group;
R⁵ represents a hydrogen atom or a C₁₋₆ hydrocarbon group, or R⁴ and R⁵, when taken together, represent a C₁₋₄ linear, branched or cyclic alkanediyl group, optionally comprising one oxygen atom;
R⁶ represents a hydrogen atom or a methyl group;
R⁷ and R⁸ represent, independent of each other, a hydrogen atom or a C₁₋₆ alkyl group; and
the groups R¹ and R² have in total at least 4 carbon atoms.

For the sake of clarity, by the expression "any one of its stereoisomers or a mixture thereof", or the similar, it is meant the normal meaning understood by a person skilled in the art, i.e. that the compound of formula (I) can be a pure enantiomer (if optically active) or a diastereoisomer. In other words, the compound of formula (I) may possess several stereocenters and each of said stereocenter can have two different stereochemistries (e.g. R or S). The compound of formula (I) may even be in the form of a pure enantiomer or in the form of a mixture of enantiomers or diastereoisomers. The compound of formula (I) can be in a racemic or scalemic form. Therefore, the compound of formula (I) can be one stereoisomer or in the form of a composition of matter comprising, or consisting of, various stereoisomers.

It is understood that by "... hydrocarbon group ..." it is meant that said group consists of hydrogen and carbon atoms and can be in the form of an aliphatic hydrocarbon, i.e. linear or branched saturated hydrocarbon (e.g. alkyl group), a linear or branched unsaturated hydrocarbon (e.g. alkenyl or alkynyl group), a saturated cyclic hydrocarbon (e.g. cycloalkyl) or an unsaturated cyclic hydrocarbon (e.g. cycloalkenyl or cycloalkynyl), or can be in the form of an aromatic hydrocarbon, i.e. aryl group, or can also be in the form of a mixture of said type of groups, e.g. a specific group may comprise a linear alkyl, a branched alkenyl (e.g. having one or more carbon-carbon double bonds), a (poly)cycloalkyl and an aryl moiety, unless a specific limitation to only one type is mentioned. Similarly, in all the embodiments of the invention, when a group is mentioned as being in the form of more than one type of topology (e.g. linear, cyclic or branched) and/or being saturated or unsaturated (e.g. alkyl, aromatic or alkenyl), it is also meant a group which may comprise moieties having any one of said topologies or being saturated or unsaturated, as explained above. Similarly, in all the embodiments of the invention, when a group is mentioned as being in the form of one type of saturation or unsaturation, (e.g. alkyl), it is meant that said group can be in any type of topology (e.g. linear, cyclic or branched) or having several moieties with various topologies.

It is understood that with the term "... a hydrocarbon group, optionally comprising ..." it is meant that said hydrocarbon group optionally comprises alcohol, ketone, aldehyde, ether, thioether, ester, carboxylic acid, amine, amide, carbamate, nitrile or thiol groups. These groups can either substitute a hydrogen atom of the hydrocarbon group and thus be laterally attached to said hydrocarbon, or substitute a carbon atom (if chemically possible) of the hydrocarbon group and thus be inserted into the hydrocarbon chain. For example, a -CH₂-CH₂-CHOH-CH₂- group represents a C₄ hydrocarbon group comprising an alcohol group (substitution of a hydrogen atom), i.e. a C₄ hydrocarbon comprising an oxygen atom, a -CH₂-CH₂-COO-CH₂-CH₂-CH₂-CH₂- group represents a C₆ hydrocarbon group comprising one ester group (substitution of carbon atoms/insertion into the hydrocarbon chain), i.e. a C₇ hydrocarbon comprising two oxygen atoms and, similarly, a -CH₂-CH₂-O-CH₂-CH₂-O-CH₂-CH₂- group represents a C₆ hydrocarbon group comprising two ether groups, i.e. a C₆ hydrocarbon comprising two oxygen atoms.

The term "optionally" is understood that a certain group to be optionally substituted or optionally comprising can or cannot be substituted with a certain functional group or can or cannot comprise a certain atom. The term "one or more" is understood as being substituted with 1 to 7, preferably 1 to 5 and more preferably 1 to 3 of a certain functional group.

The terms "alkyl" and "alkenyl" are understood as comprising branched and linear alkyl and alkenyl groups. The terms "alkenyl", "cycloalkenyl" and "heterocycloalkenyl" is understood as comprising 1, 2 or 3 olefinic double bonds, preferably 1 or 2 olefinic double bonds. The terms "cycloalkyl", "cycloalkenyl", "heterocycloalkyl", "heterocycloalkenyl" and "heterocyclic" are understood as comprising a monocyclic or fused, spiro and/or bridged bicyclic or tricyclic cycloalkyl, cycloalkenyl, heterocycloalkyl, heterocycloalkenyl and heterocyclic groups, preferably monocyclic cycloalkyl, cycloalkenyl, heterocycloalkyl and heterocycloalkenyl groups.

The term "aryl" is understood as comprising any group comprising at least one aromatic group such as phenyl, indenyl, indanyl, benzodioxolyl, dihydrobenzodioxinyl, tetrahydronaphthalenyl or naphthalenyl group.

The term "oxo group" is understood as comprising any group of formula =O; i.e. such as a ketone or an aldehyde. In other words, a C₁₋₆ alkyl group optionally substituted by an oxo group is an alkyl group having from 1 to 6 carbon atoms and one of these carbon atoms, even the terminal carbon, may be substituted by a =O group instead of two hydrogen atoms.

The term "the groups R¹ and R² have in total at least 4 carbon atoms" is understood as the sum of the carbon atom(s) of the R¹ group and the carbon atom(s) of the R² group being equal to or above 4; i.e. when the R² group is a hydrogen atom, then the R¹ group is a C₄-₁₈ hydrocarbon group such as a butyl group; or when the R¹ group is a methyl group, then the R² group is a C₃₋₁₅ hydrocarbon group such as a propyl group.

According to any embodiment of the invention, R⁷ may be a hydrogen atom or a C₁₋₄ alkyl group. Particularly, R⁷ may be a hydrogen atom or a C₁₋₃ alkyl group. Particularly, R⁷ may be a hydrogen atom or a methyl or ethyl group. Particularly, R⁷ may be a hydrogen atom or a methyl group. Even more particularly, R⁷ may be a hydrogen atom.

According to any embodiment of the invention, R⁸ may be a hydrogen atom or a C₁₋₄ alkyl group. Particularly, R⁸ may be a hydrogen atom or a C₁₋₃ alkyl group. Particularly, R⁸ may be a hydrogen atom or a methyl or ethyl group. Particularly, R⁸ may be a hydrogen atom or a methyl group. Even more particularly, R⁸ may be a hydrogen atom.

According to any embodiment of the invention, R⁶ may be a hydrogen atom.

According to any embodiment of the invention, R⁵ may be a hydrogen atom or a C₁₋₆ linear or branched alkyl group or a C₂₋₆ linear or branched alkenyl group. Particularly, R⁵ may be a hydrogen atom or a C₁₋₄ linear or branched alkyl group or a C₂₋₄ linear or branched alkenyl group. Particularly, R⁵ may be a hydrogen atom or a C₁₋₃ linear or branched alkyl group or a C₂₋₃ linear alkenyl group. Particularly, R⁵ may be a hydrogen atom or a methyl, ethyl, propyl, isopropyl, butyl, tert-butyl, isobutyl, sec-butyl or allyl group. Particularly, R⁵ may be a hydrogen atom or a methyl group. Even more particularly, R⁵ may be a hydrogen atom.

According to any embodiment of the invention, R⁹ may be a hydrogen atom.

According to any embodiment of the invention, X may be an oxygen atom or an NH group. Particularly, X may be an oxygen atom.

According to any embodiment of the invention, n may be 0.

According to any embodiment of the invention, the invention's compound is a compound of formula 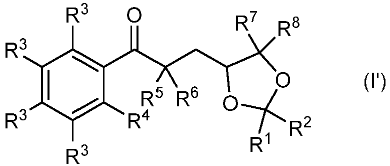 in the form of any one of its stereoisomers or a mixture thereof, and wherein R¹, R²,
R³, R⁴, R⁵, R⁶, R⁷ and R⁸ have the same meaning as defined above.

According to any embodiment of the invention, the invention's compound is a compound of formula 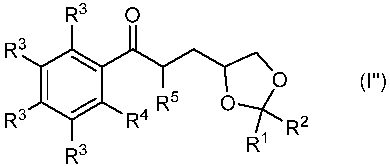 in the form of any one of its stereoisomers or a mixture thereof, and wherein R¹, R²,
R³, R⁴ and R⁵ have the same meaning as defined above.

According to any embodiment of the invention, each R³ may be, independently of each other, a hydrogen atom, a C₁₋₅ alkoxy group or a C₁₋₁₀ alkyl group, optionally substituted by a hydroxy, C₁₋₃ alkoxy or oxo group. Particularly, each R³ may be, independently of each other, a hydrogen atom, a C₁₋₄ alkoxy group or a C₁₋₈ alkyl group, optionally substituted by a hydroxy, C₁₋₃ alkoxy or oxo group. Particularly, each R³ may be, independently of each other, a hydrogen atom, a C₁₋₃ alkoxy group or a C₁₋₆ alkyl group, optionally substituted by a hydroxy, C₁₋₃ alkoxy or oxo group. Particularly, each R³ may be, independently of each other, a hydrogen atom, a methoxy group or a C₁₋₄ alkyl group. Particularly, one or two R³ may be, independently of each other, a hydrogen atom, a methoxy group or a C₁₋₄ alkyl group and the other are a hydrogen atom. Particularly, one or two R³ may be, independently of each other, a hydrogen atom, a methoxy, methyl, ethyl, propyl, isopropyl, butyl, tert-butyl, isobutyl or sec-butyl group and the other are a hydrogen atom. Particularly, one or two R³ may be, independently of each other, a hydrogen atom, a methoxy, methyl, ethyl, isopropyl, tert-butyl group and the other are a hydrogen atom. Particularly, one R³ may be, independently of each other, a hydrogen atom, a methoxy or methyl group and the other R³ are a hydrogen atom. Even more particularly, the R³ in meta position of R⁴ may be a hydrogen atom, a methoxy or methyl group and the other R³ are a hydrogen atom.

According to any embodiment of the invention, two adjacent R³ groups, when taken together, may be a C₃₋₆ linear alkanediyl group, optionally substituted by one or more of a hydroxy, C₁₋₃ alkyl and/or C₁₋₃ alkoxy group. Particularly, two adjacent R³ groups, when taken together, may be a C₃₋₄ linear alkanediyl group, optionally substituted by one or more of a hydroxy and/or C₁₋₃ alkyl group. Particularly, two adjacent R³ groups, when taken together, may be a C₃₋₄ linear alkanediyl group, optionally substituted by one or more of a C₁₋₃ alkyl group. Even more particularly, two adjacent R³ groups, when taken together, may be a C₃₋₄ linear alkanediyl group, optionally substituted by one, two, three, four or five of a methyl, ethyl or isopropyl group.

According to any embodiment of the invention, R⁴ may be a hydrogen atom, a C₁₋₄ alkoxy group or a C₁₋₈ alkyl group, optionally substituted by a hydroxy, C₁₋₃ alkoxy or oxo group. Particularly, R⁴ may be a hydrogen atom, a C₁₋₃ alkoxy group or a C₁₋₆ alkyl group, optionally substituted by a hydroxy, C₁₋₃ alkoxy or oxo group. Particularly, R⁴ may be a hydrogen atom, a methoxy group or a C₁₋₄ alkyl group. Particularly, R⁴ may be a hydrogen atom, a methoxy group or a C₁₋₃ alkyl group. Particularly, R⁴ may be a hydrogen atom, a methoxy or a methyl group. Even more particularly, R⁴ may be a hydrogen atom.

According to any embodiment of the invention, R⁴ and R⁵ may be taken together and represent a C₁ to C₃ linear or branched alkanediyl group. Particularly, R⁴ and R⁵ may be taken together and represent a methanediyl or propane-2,2-diyl group.

According to any embodiment of the invention, R¹ and R² are derived from an active aldehyde of formula R¹CHO (i.e. R² is a hydrogen atom) or ketone of formula (R¹)(R²)C=O; said aldehyde or ketone having a molecular weight comprised between 80 and 230 g/mol and being a C₅ to C₁₈ compound.

According to any embodiment of the invention, R¹ may be a C₁₋₁₅ hydrocarbon group, optionally comprising one to three oxygen atoms and/or one to two nitrogen atoms and/or one sulfur atom. Particularly, R¹ may be a C₁₋₁₂ hydrocarbon group, optionally comprising one to three oxygen atoms. Particularly, R¹ may be a C₁ to C₁₀ hydrocarbon group, optionally comprising one to three oxygen atoms. Particularly, R¹ may be a C₁ to C₉ hydrocarbon group, optionally comprising one to three oxygen atoms. Even more particulraly, R¹ may be a C₁ to C₈ hydrocarbon group, optionally comprising one to three oxygen atoms.

According to any embodiment of the invention, R¹ and R², when taken together, form a C₅₋₁₆ cycloalkyl or C₅₋₁₆ cycloalkenyl group, each optionally substituted with one or more of a C₁₋₈ alkyl, C₂₋₈ alkenyl, C₁₋₈ alkoxy, C₃₋₈ cycloalkyl, C₅₋₈ cycloalkenyl, C₆ aryl and/or C₆ aryloxy group, each optionally substituted with one or more of a C₁₋₆ alkyl, C₁₋₆ alkoxy, carboxylic acid and/or C₁₋₄ carboxylic ester group. Particularly, R¹ and R², when taken together, form a C₅₋₁₆ cycloalkyl or C₅₋₁₆ cycloalkenyl group, each optionally substituted with one or more of a C₁₋₆ alkyl, C₂₋₆ alkenyl, C₁₋₆ alkoxy, C₃₋₆ cycloalkyl, C₅₋₆ cycloalkenyl, C₆ aryl and/or C₆ aryloxy group, each optionally substituted with one or more of a C₁₋₄ alkyl, C₁₋₄ alkoxy, carboxylic acid and/or C₁₋₃ carboxylic ester group. Even more particulraly, R¹ and R², when taken together, form a C₅₋₁₆ cycloalkyl or C₅₋₁₆ cycloalkenyl group, each optionally substituted with one or more of a C₁₋₄ alkyl, C₂₋₄ alkenyl, C₁₋₄ alkoxy, C₃₋₆ cycloalkyl, C₅₋₆ cycloalkenyl, C₆ aryl and/or C₆ aryloxy group, each optionally substituted with one or more of a C₁₋₃ alkyl, C₁₋₃ alkoxy, carboxylic acid and/or C₁₋₂ carboxylic ester group.

According to any one of the above embodiments, said compound of formula (I) may be 1-phenyl-3-(2-phenyl-1,3-dioxan-4-yl)propan-1-one, 3-(2-decyl-1,3-dioxolan-4-yl)-1-phenylpropan-1-one, 1-phenyl-3-(2-(2-phenylpropyl)-1,3-dioxolan-4-yl)propan-1-one, 1-phenyl-3-(2-(undecan-2-yl)-1,3-dioxolan-4-yl)propan-1-one, 3-(2-(dec-9-en-1-yl)-1,3-dioxolan-4-yl)-1-phenylpropan-1-one, 3-(2-(2,4-dimethylcyclohex-3-en-1-yl)-1,3-dioxolan-4-yl)-1-phenylpropan-1-one, 3-(2-(2-(4,4-dimethylcyclohex-1-en-1-yl)ethyl)-1,3-dioxolan-4-yl)-1-phenylpropan-1-one, 1-phenyl-3-(2-(undec-3-en-1-yl)-1,3-dioxolan-4-yl)propan-1-one, 3-(2-(2-(4-methylcyclohex-3-en-1-yl)propyl)-1,3-dioxolan-4-yl)-1-phenylpropan-1-one, 3-(2-(6-methylhept-5-en-2-yl)-1,3-dioxolan-4-yl)-1-phenylpropan-1-one, (E/Z)-3-(2-(4,8-dimethylnon-3-en-1-yl)-1,3-dioxolan-4-yl)-1-phenylpropan-1-one, (E/Z)-3-(2-(5-cyclohexyl-4-methylpent-4-en-2-yl)-1,3-dioxolan-4-yl)-1-phenylpropan-1-one, 3-(2-methyl-2-phenyl-1,3-dioxolan-4-yl)-1-phenylpropan-1-one, 3-(2-methyl-2-phenethyl-1,3-dioxolan-4-yl)-1-phenylpropan-1-one, 3-(6-pentyl-1,4-dioxaspiro[4.4]nonan-2-yl)-1-phenylpropan-1-one, 1-phenyl-3-(2-phenyl-1,3-dioxolan-4-yl)propan-1-one, 3-(2-decyl-1,3-dioxolan-4-yl)-1-(4-methoxyphenyl)propan-1-one, 3-(2-decyl-1,3-dioxolan-4-yl)-1-(p-tolyl)propan-1-one, 3-(2-(4-methoxyphenyl)-1,3-dioxan-4-yl)-1-phenylpropan-1-one, 3-(2-(4-ethylphenyl)-1,3-dioxan-4-yl)-1-phenylpropan-1-one, 3-(2-decyl-1,3-dioxan-4-yl)-1-phenylpropan-1-one, 3-(2-decyl-1,3-dioxan-4-yl)-1-phenylpropan-1-one, 2-methyl-3-(2-phenethyl-1,3-dioxolan-4-yl)-1-phenylpropan-1-one, 2,2-dimethyl-3-(2-phenethyl-1,3-dioxolan-4-yl)-1-phenylpropan-1-one, 3-(2-phenethyl-1,3-dioxolan-4-yl)-1-(5,6,7,8-tetrahydronaphthalen-2-yl)propan-1-one, 3-(5-methyl-2-phenethyl-1,3-dioxolan-4-yl)-1-phenylpropan-1-one or 3-(5,5-dimethyl-2-phenethyl-1,3-dioxolan-4-yl)-1-phenylpropan-1-one, in the form of any one of its stereoisomers.

In a particular embodiment of the invention, the following compounds are excluded from formula (I): methyl 3-methoxy-2-(2-methyl-5-(4-(3-oxo-3-phenylpropyl)-1,3-dioxolan-2-yl)benzyl)acrylate, methyl 3-methoxy-2-(methyl(3-(4-(3-oxo-3-phenylpropyl)-1,3-dioxolan-2-yl)phenyl)amino)acrylate, methyl 2-(methoxyimino)-3-(2-methyl-5-(4-(3-oxo-3-phenylpropyl)-1,3-dioxolan-2-yl)phenyl)propanoate, 2-(methoxyimino)-N-methyl-3-(2-methyl-5-(4-(3-oxo-3-phenylpropyl)-1,3-dioxolan-2-yl)phenyl)propenamide and 3-(2-(3-(2-(5,6-dihydro-1,4,2-dioxazin-3-yl)-2-(methoxyimino)ethyl)-4-methylphenyl)-1,3-dioxolan-4-yl)-1-phenylpropan-1-one.

According to any of the embodiments, the compounds of formula (I) are non-volatile and essentially odorless. At the same time, they are relatively stable in perfuming compositions or perfumed consumer product.

Non-volatile and essentially odorless compounds are advantageously characterized by a vapor pressure below 2.0 Pa, as obtained by calculation using the software EPIwin v. 3.10 (2000, available at the US Environmental Protection Agency). Preferably, said vapor pressure is below 0.2 Pa, or even more preferably below 0.02 Pa.

The compounds according to formula (I) are capable of releasing, via a decomposition reaction, an active carbonyl compound of formula 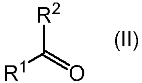 wherein R¹ and R² have the same meaning as described above;
together with a phenyl ketone derivative of formula 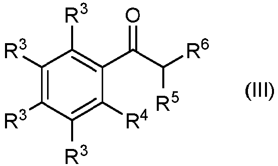 wherein R³ to R⁶ have the same meaning as described above.

The term "carbonyl" designates an aldehyde or a ketone depending on the meaning of the R² group; i.e. the carbonyl compound of formula (II) is an aldehyde when R² represents a hydrogen atom or the carbonyl compound of formula (II) is a ketone when R² is not a hydrogen atom.

The decomposition reaction, which leads to the release of the compound of formula (II) and the compound of formula (III), is believed to be triggered by light, in particular by light at a wavelength above 300, preferably above 330 nm, even more preferably above 350 nm. It is believed that upon exposure to light the compounds of formula (I) degrade to form a phenyl ketone derivative of formula (III) and a 4-methylene-1,3-dioxolane derivative (n = 0 and X = O), a 4-methyleneoxazolidine derivative (n = 0 and X = NR⁹), a 4-methylene-1,3-dioxane derivative (n = 1 and X = O) or a 4-methylene-1,3-oxazinane derivative (n = 1 and X = NR⁹). The respective dioxolane, oxazolidine, dioxane or oxazinane derivatives are hydrolytically unstable and hydrolyze to form a carbonyl compound of formula (II). The cleavage of the hydrolytically stable cyclic acetals or ketals or oxazolidines or 1,3-oxazinanes of the invention is thus induced by light to form a hydrolytically labile intermediate that releases the target compounds in a two-step sequence. Depending on the structure, all but three (if n = 0 and R⁷ and R⁸ are hydrogen atoms) or four (if n = 1 and R⁷ and R⁸ are hydrogen atoms) carbon atoms are transformed into active compounds, which makes the delivery systems according to the present invention highly atom-economic.

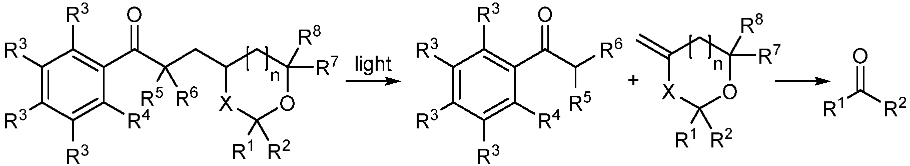

According to any of the embodiments, the compound of formula (II) and the compound of formula (III) are advantageously characterized by a vapor pressure above 1.0 Pa, as obtained by calculation using the software EPIwin v. 3.10 (2000, available at the US Environmental Protection Agency). According to another embodiment, said vapor pressure is above 5.0, or even above 7.0 Pa.

In a particular embodiment, the compound of formula (II) wherein R² is a hydrogen atom; i.e. aldehydes of formula R¹CHO, may be selected from the group consisting of benzaldehyde, 3-(1,3-benzodioxol-5-yl)-2-methylpropanal, 3-butoxybenzaldehyde, 5-cyclohexyl-2,4-dimethylpent-4-enal, decanal, 2,4-decadienal, 2-decenal, 4-decenal, 8-decenal, 9-decenal, 3-(6,6-dimethyl-bicyclo[3.1.1]hept-2-en-2-yl)propanal, 2,4-dimethyl-3-cyclohexene-1-carbaldehyde (Triplal^{®}, origin: International Flavors & Fragrances, New York, USA), 3,5-dimethyl-3-cyclohexene-1-carbaldehyde, 1-(3,3-dimethyl-1-cyclohexyl)-1-ethanone, 3-(4,4-dimethylcyclohex-1-enyl)propanal, 5,9-dimethyl-4,8-decadienal, 4,8-dimethyl-4,9-decadienal, 5,9-dimethyldec-4-enal, 2,6-dimethyl-5-heptenal (melonal), (E/Z)-3,7-dimethyl-2,6-octadienal (citral, neral), 3,7-dimethyloctanal, 3,7-dimethyl-6-octenal (citronellal), (3,7-dimethyl-6-octenyl)acetaldehyde, dodecanal, 2-dodecenal, 3-dodecenal, 4-dodecenal, 3-ethoxy-4-hydroxybenzaldehyde (ethyl vanillin), 4-ethyl benzaldehyde, 3-(2- and 4-ethylphenyl)-2,2-dimethylpropanal, 2-furancarbaldehyde (furfural), 2,4-heptadienal, 4-heptenal, 2-hexenal, 3-hexenal, 2-hydroxybenzaldehyde, 7-hydroxy-3,7-dimethyloctanal (hydroxycitronellal), 4-hydroxy-3-methoxybenzaldehyde (vanillin), 4- and 3-(4-hydroxy-4-methylpentyl)-3-cyclohexene-1-carbaldehyde (Lyral^{®}, origin: International Flavors and Fragrances, New York, USA), 3-(4-isobutyl-2-methylphenyl)propanal, 3-(4-isobutylphenyl)propanal, 4-isopropylbenzaldehyde (cuminaldehyde), 3-(4-isopropylcyclohex-1-en-1-yl)propanal, 3-(3-isopropylphenyl)butanal, 3-(4-isopropylphenyl)-2-methylpropanal, 2-(4-isopropylphenyl)propanal, 4-methoxybenzaldehyde (anisaldehyde), 6-methoxy-2,6-dimethylheptanal (methoxymelonal), 8(9)-methoxy-tricyclo[5.2.1.0.(2,6)]decane-3(4)-carbaldehyde (Scentenal^{®}, origin: Firmenich SA, Geneva, Switzerland), 4-methylbenzaldehyde, 3-(4-methylcyclohex-3-en-1-yl)butanal, 2-methyldecanal, 2-(4-methylenecyclohexyl)propanal, 1-methyl-4-(4-methyl-3-pentenyl)-3-cyclohexen-1-carbaldehyde (Precyclemone^{®} B, origin: International Flavors & Fragrances, New York, USA), 4-(4-methyl-3-pentenyl)-3-cyclohexene-1-carbaldehyde (Empetal, origin: Givaudan-Roure SA., Vernier, Switzerland), (4-methylphenoxy)acetaldehyde, (4-methylphenyl)acetaldehyde, 3-methyl-5-phenylpentanal (Phenexal^{®}, origin: Firmenich SA, Geneva, Switzerland), 3-methyl-3-phenylpropanal, 2-methylundecanal, 2,4-nonadienal, 2,6-nonadienal, 2-nonenal, 3-nonenal, 6-nonenal, 8-nonenal, 4-(octahydro-5H-4,7-methanoinden-5-ylidene)butanaloctanal, 2-octenal, phenoxyacetaldehyde, phenylacetaldehyde, 3-phenylbutanal (Trifernal^{®}, origin: Firmenich SA, Geneva, Switzerland), 2-phenylpropanal (hydratropaldehyde), 3-phenylpropanal, 3-(4-tert-butylphenyl)-2-methylpropanal (Lilial^{®}, origin: Givaudan-Roure SA, Vernier, Switzerland), 3-(4-tert-butylphenyl)propanal (Bourgeonal^{®}, origin: Quest International, Naarden, Netherlands), tricyclo[5.2.1.0(2,6)]decane-4-carbaldehyde, exo-tricyclo[5.2.1.0(2,6)]decane-8exo-carbaldehyde (Vertral^{®}, origin: Symrise, Holzminden, Germany), 2,6,6-trimethyl-bicyclo[3.1.1]heptane-3-carbaldehyde (formyl pinane), 2,6,6-trimethylcyclohexa-1,3-diene-1-carbaldehyde (safranal), 2,4,6- and 3,5,6-trimethyl-3-cyclohexene-1-carbaldehyde, 2,2,3-trimethyl-3-cyclopentene-1-acetaldehyde (campholenic aldehyde), 2,6,10-trimethyl-2,6,9,11-dodecatetraenal, 2,5,6-trimethyl-4-heptenal, 3,5,5-trimethylhexanal, 2,6,10-trimethyl-9-undecenal, undecanal, 2-undecenal, 10-undecenal or 9-undecenal and their mixtures such as Intreleven aldehyde (origin: International Flavors & Fragrances, New York, USA) and Aldehyde Supra (origin: Firmenich SA, Geneva, Switzerland) and 4-vinylcyclohex-1-ene-1-carbaldehyde; wherein the underlined compounds represent, in a preferred embodiment of the invention, particularly useful aldehydes.

In a particular embodiment, the compound of formula (II) wherein R² is not a hydrogen atom; i.e. ketone of formula (R¹)(R²)C=O, may be selected from the group consisting of 4-(1,3-benzodioxol-5-yl)-2-butanone, 2-butanone, (4E/Z,8E/Z)-cyclododeca-4,8-dien-1-one, 2-cyclohexyl-4-methyl-2-pentanone, cyclopentadecanone, (Z)-cyclopentadec-4-en-1-one, (Z)-cycloheptadec-9-en-1-one, 1-(3,5-diisopropylphenyl)ethan-1-one, 1-(3,3-dimethylcyclohexyl)ethan-1-one, 1-[2,6-dimethyl-4-(2-methyl-2-propanyl)phenyl]ethanone, 2,5-dimethyl-2-octen-6-one, 4,7-dimethyl-6-octen-3-one, 2,6-dimethyl-7-octen-4-one (dihydrotagetone), 4-(1,1-dimethylpropyl)cyclohexan-1-one, (5-E/Z)-6,10-dimethylundeca-5,9-dien-2-one, 2-ethyl-4,4-dimethylcyclohexan-1-one, 4-ethyl-8-methyloctahydronaphthalen-1(2H)-one, 1-(4-ethylphenyl)ethan-1-one, 1-(3-ethyl-1,1,3,6-tetramethyl-2,3-dihydro-1H-inden-5-yl)ethanone, 2-heptanone, 3-heptanone, 2-heptylcyclopentan-1-one, 4,4a,6,7,8,8a-hexahydro-1,4-methanonaphthalen-5(1H)-one, 1-(1,1,2,3,3,6-hexamethyl-2,3-dihydro-1H-inden-5-yl)ethanone (Phantolid^{®}, origin: PFW Aroma Chemicals, Barnevald, The Netherlands), 1-(3,5,5,6,8,8-hexamethyl-5,6,7,8-tetrahydro-2-naphthalenyl)ethan-1-one (Fixolide^{®}, origin: Givaudan SA, Vernier, Switzerland), 2-hexanone, 2-(5-hexen-1-yl)cyclopentan-1-one, 4-(4-hydroxyphenyl)-2-butanone, 1-isopropyl-4-methylbicyclo[3.1.0]hexan-3-one, 5-isopropyl-2-methylcyclohexan-1-one, 2-isopropyl-5-methylcyclohexan-1-one (menthone), 1-(5-isopropyl-2-methylcyclohex-2-en-1-yl)propan-1-one, 1-(3-isopropyl-1,1,2,6-tetramethyl-5-indanyl)ethan-1-one, 4-(4-methoxyphenyl)-2-butanone, 1-(4-methoxyphenyl)ethan-1-one (Acetanisole, origin: Givaudan SA, Vernier, Switzerland), 1-(2-methoxyphenyl)propan-1-one, 7-methyl-2H-benzo[b][1,4]dioxepin-3(4H)-one, 2-(2-(4-methylcyclohex-3-en-1-yl)propyl)cyclopentan-1-one, 3-methylcyclopentadecan-1-one, 3-methylcyclopentadec-4-en-1-one, 3-methylcyclopentadec-5-en-1-one, 5-methyl-3-heptanone, 6-methyl-5-hepten-2-one, 7-methyloctahydro-1,4-methanonaphthalen-6(2H)-one, methyl (Z)-2-(3-oxo-2-(pent-2-en-1-yl)cyclopentyl)acetate (methyl jasmonate), methyl 2-(3-oxo-2-pentylcyclopentyl)acetate, 1-(4-methyl-1-phenoxy)-2-propanone, 3-methyl-1-phenylbutan-1-one, 1-(4-methylphenyl)ethan-1-one, 2-methyl-1-phenylpropan-1-one, 1-(4-methylphenyl)propan-1-one, 1-[4-(2-methyl-2-propanyl)phenyl]ethan-1-one, 2-(1-methylpropyl)cyclohexan-1-one, 2-nonanone, 4-nonanone, 1-(octahydro-2,3,8,8-tetramethyl-2-naphthalenyl)-1-ethanone (isomeric mixture, Iso E Super^{®}, origin: International Flavors & Fragrances, New York, USA), 2-octanone, 3-octanone, oct-2-en-4-one, 2-pentadecanone, 2-pentanone, 2-pentylcyclopentan-1-one (Delphone, origin: Firmenich SA, Geneva, Switzerland), 1-phenylbutan-1-one, 4-phenyl-2-butanone, 1-phenylethan-1-one (acetophenone), 1-phenylhexan-1-one, 1-phenylpentan-1-one, 1-phenyl-4-penten-1-one (Lavonax, origin: International Flavors & Fragrances, New York, USA), 1-phenylpropan-1-one (propiophenone), 7-propyl-2H-benzo[b][1,4]dioxepin-3(4H)-one, 1-(5-propylbenzo[d][1,3]dioxol-2-yl)ethan-1-one, 2-(tert-butyl)cyclohexan-1-one, 4-(tert-butyl)cyclohexan-1-one, 1-(6-tert-butyl-1,1-dimethyl-4-indanyl)-1-ethanone (Crysolide, Givaudan SA, Vernier, Switzerland), 1-(5,6,7,8-tetrahydro-2-naphthalenyl)ethan-1-one (Florantone^{®}, origin: Takasago Corporation, Tokyo, Japan), 3,6,8,8-tetramethylhexahydro-1H-3a,7-methanoazulen-5(4H)-one, 1,1,5,5-tetramethylhexahydro-2H-2,4a-methanonaphthalen-8(5H)-one (iso-longifolanone), 2,4a,8,8-tetramethyloctahydrocyclopropa[d]naphthalen-3(1H)-one (thujopsan-4-one), 2,2,7,9-tetramethylspiro[5.5]undec-7-en-1-one, 2-tridecanone, 1,3,3-trimethylbicyclo[2.2.1]heptan-2-one, 1,7,7-trimethylbicyclo[2.2.1]heptan-2-one, 2,2,4-trimethylbicyclo[3.1.1]heptan-3-one, 2,6,6-trimethylcycloheptan-1-one, 2,2,6-trimethylcyclohexan-1-one, 4-(2,6,6-trimethylcyclohex-2-en-1-yl)butan-2-one (dihydro-alpha-ionone), 4-(2,6,6-trimethylcyclohex-1-en-1-yl)butan-2-one (dihydro-beta-ionone), 2,3,3-trimethyl-2,3-dihydro-1H-inden-1-one, 2,2,5-trimethyl-5-pentylcyclopentan-1-one, 2-undecanone and 5-undecanone; wherein the underlined compounds represent, in a preferred embodiment of the invention, particularly useful ketones.

In a particular embodiment, the compound of formula (III) may be selected from the group consisting of 1-[2,6-dimethyl-4-(2-methyl-2-propanyl)phenyl]ethanone, 1-(3,5-diisopropylphenyl)ethan-1-one, 1-(4-ethylphenyl)ethan-1-one, 1-(3-ethyl-1,1,3,6-tetramethyl-2,3-dihydro-1H-inden-5-yl)ethanone, 1-(1,1,2,3,3,6-hexamethyl-2,3-dihydro-1H-inden-5-yl)ethanone (Phantolid^{®}, origin: PFW Aroma Chemicals, Barnevald, The Netherlands), 1-(3,5,5,6,8,8-hexamethyl-5,6,7,8-tetrahydro-2-naphthalenyl)ethan-1-one (Fixolide^{®}, origin: Givaudan SA, Vernier, Switzerland), 1-(3-isopropyl-1,1,2,6-tetramethyl-5-indanyl)ethan-1-one, 1-(4-methoxyphenyl)ethan-1-one (Acetanisole, origin: Givaudan SA, Vernier, Switzerland), 1-(2-methoxyphenyl)propan-1-one, 3-methyl-1-phenylbutan-1-one, 1-(4-methylphenyl)ethan-1-one, 2-methyl-1-phenylpropan-1-one, 1-(4-methylphenyl)propan-1-one, 1-[4-(2-methyl-2-propanyl)phenyl]ethan-1-one, 1-phenylbutan-1-one, 1-phenylhexan-1-one, 1-phenylpentan-1-one, 1-phenyl-4-penten-1-one (Lavonax, origin: International Flavors & Fragrances, New York, USA), 1-phenylethan-1-one (acetophenone), 1-phenylpropan-1-one (propiophenone), 1-(6-tert-butyl-1,1-dimethyl-4-indanyl)-1-ethanone (Crysolide, Givaudan SA, Vernier, Switzerland), 1-(5,6,7,8-tetrahydro-2-naphthalenyl)ethan-1-one (Florantone^{®}, origin: Takasago Corporation, Tokyo, Japan), 1-(5,6,7,8-tetrahydronaphthalen-2-yl)ethan-1-one and 2,3,3-trimethyl-2,3-dihydro-1H-inden-1-one; wherein the underlined compounds represent, in a preferred embodiment of the invention, particularly useful phenyl ketone derivatives, with 1-phenylethan-1-one (acetophenone) being the most preferred phenyl ketone derivative.

The present invention also relates to a microcapsule comprising at least one compound of formula (I). In one embodiment, the at least one compound of formula (I) is encapsulated in a core-shell microcapsule wherein the at least one compound of formula (I) is contained in the core surrounded by the shell. In one embodiment, the shell of the microcapsule protects the compound of formula (I) from the environment. The shell is made of material which is able to release the at least one compound of formula (I) and/or the compound of formulas (II) and/or (III). In one embodiment, the shell is made of material which is able to release the compound of formula (I) and/or the compound of formulas (II) and/or (III) upon breakage of the shell and/or by diffusion through the shell. A person skilled in the art is well aware of processes to prepare said microcapsules. So, a microcapsule comprising at least one compound of formula (I) is one object of the present invention.

In a preferred embodiment, encapsulation of a compound of formula (I) may provide an environment within the capsule wherein all, or a portion of the compound of formula (I) may decompose, thereby releasing the individual carbonyl compound of formula (II) and the phenyl ketone (III) into the capsule. In a preferred embodiment, the shell of the microcapsule may act as a permeability barrier, preventing the leakage of the individual carbonyl compound of formula (II) and the phenyl ketone (III) from the capsule.

According to a particular embodiment, the shell of the microcapsule comprises a material selected from the group consisting of polyurea, polyurethane, polyamide, polyester, poly(meth)acrylate (i.e. polyacrylate and/or polymethacrylate), polysiloxane, polycarbonate, polysulfonamide, polymers of urea and formaldehyde, melamine and formaldehyde, melamine and urea, or melamine and glyoxal and mixtures thereof. The shell can also be hybrid, namely organic-inorganic such as a hybrid shell composed of at least two types of inorganic particles that are cross-linked, or yet a shell resulting from the hydrolysis and condensation reaction of a polyalkoxysilane macro-monomeric composition.

According to a particular embodiment, the core-shell microcapsule(s) can be also derived by using different or more than one encapsulation method.

In a preferred embodiment, the shell of the microcapsules may be, each independently, selected from the group of aminoplast, polyamide, polyester, polyurea and polyurethane shells and mixtures thereof.

In a particular embodiment, the shell of the microcapsules comprises an aminoplast copolymer, such as melamine-formaldehyde or urea-formaldehyde or cross-linked melamine formaldehyde or melamine glyoxal.

In a particular embodiment, the shell of the microcapsules is polyurea-based made from, for example but not limited to isocyanate-based monomers and amine-containing crosslinkers such as guanidine carbonate and/or guanazole. Certain polyurea microcapsules comprise a polyurea wall which is the reaction product of the polymerization between at least one polyisocyanate comprising at least two isocyanate functional groups and at least one reactant selected from the group consisting of an amine (for example a water-soluble guanidine salt and guanidine); a colloidal stabilizer or emulsifier; and an encapsulated perfume. However, the use of an amine can be omitted.

In a particular embodiment, the colloidal stabilizer includes an aqueous solution of between 0.1% and 0.4% of polyvinyl alcohol, between 0.6% and 1% of a cationic copolymer of vinylpyrrolidone and of a quaternized vinylimidazol (all percentages being defined by weight relative to the total weight of the colloidal stabilizer). In a particular embodiment, the emulsifier is an anionic or amphiphilic biopolymer, which may be for example chosen from the group consisting of Gum Arabic, soy protein, gelatin, sodium caseinate and mixtures thereof.

In a particular embodiment, the shell of the microcapsules is polyurethane-based made from, for example but not limited to polyisocyanate and polyols, polyamide, polyester, etc.

In a particular embodiment, the microcapsules have a polymeric shell resulting from complex coacervation wherein the shell is possibly cross-linked.

In a particular embodiment of the core-shell microcapsules, the core-shell microcapsules comprise an oil-based core comprising a hydrophobic active, preferably at least one compound of formula (I), and a composite shell comprising a first material and a second material, wherein the first material and the second material are different, the first material is a coacervate, the second material is a polymeric material.

In a particular embodiment, the weight ratio between the first material and the second material is comprised between 50:50 and 99.9:0.1.

In a particular embodiment, the coacervate comprises a first polyelectrolyte, preferably selected among proteins (such as gelatin), polypeptides or polysaccharides (such as chitosan), most preferably gelatin and a second polyelectrolyte, preferably alginate salts, cellulose derivatives, guar gum, pectinate salts, carrageenan, polyacrylic and methacrylic acid or xanthan gum, or yet plant gums such as acacia gum (Gum Arabic), most preferably Gum Arabic.

The first coacervate material can be hardened chemically using a suitable cross-linker such as glutaraldehyde, glyoxal, formaldehyde, tannic acid or genipin or can be hardened enzymatically using an enzyme such as transglutaminase.

The second polymeric material can be selected from the group consisting of polyurea, polyurethane, polyamide, polyester, polyacrylate, polysiloxane, polycarbonate, polysulfonamide, polymers of urea and formaldehyde, melamine and formaldehyde, melamine and urea, or melamine and glyoxal and mixtures thereof, preferably polyurea and/or polyurethane. The second material is preferably present in an amount less than 3% w/w, preferably less than 1% w/w based on the total weight of the microcapsule slurry.

The preparation of an aqueous dispersion/slurry of core-shell microcapsules is well known by a skilled person in the art. In a particular embodiment, the microcapsule wall material may comprise any suitable resin and especially including melamine, glyoxal, polyurea, polyurethane, polyamide, polyester, etc. Suitable resins include the reaction product of an aldehyde and an amine, suitable aldehydes include, formaldehyde and glyoxal. Suitable amines include melamine, urea, benzoguanamine, glycoluril, and mixtures thereof. Suitable melamines include, methylol melamine, methylated methylol melamine, imino melamine and mixtures thereof. Suitable ureas include, dimethylol urea, methylated dimethylol urea, urea-resorcinol, and mixtures thereof. Suitable materials for making may be obtained from one or more of the following companies Solutia Inc. (St Louis, Missouri U.S.A.), Cytec Industries (West Paterson, New Jersey U.S.A.), Sigma-Aldrich (St. Louis, Missouri U.S.A.).

In a particular embodiment of the core-shell microcapsules, the core-shell microcapsule comprises
- an oil-based core comprising a hydrophobic active, preferably comprising at least one compound of formula (I),
- optionally an inner shell made of a polymerized polyfunctional monomer;
- a biopolymer shell comprising a protein, wherein at least one protein is cross-linked.

According to a particular embodiment, the protein is chosen from the group consisting of milk proteins, caseinate salts such as sodium caseinate or calcium caseinate, casein, whey protein, hydrolyzed proteins, gelatins, gluten, pea protein, soy protein, silk protein and mixtures thereof, preferably sodium caseinate.

According to a particular embodiment, the protein comprises sodium caseinate and a globular protein, preferably chosen from the group consisting of whey protein, betalactoglobulin, ovalbumine, bovine serum albumin, vegetable proteins, and mixtures thereof.

The protein is preferably a mixture of sodium caseinate and whey protein.

According to a particular embodiment, the biopolymer shell comprises a crosslinked protein chosen from the group consisting of sodium caseinate and/or whey protein.

According to a particular embodiment, the microcapsule slurry comprises at least one microcapsule made of:
- an oil-based core comprising the hydrophobic active, preferably comprising at least one compound of formula (I);
- an inner shell made of a polymerized polyfunctional monomer; preferably a polyisocyanate having at least two isocyanate functional groups
- a biopolymer shell comprising a protein, wherein at least one protein is cross-linked; wherein the protein contains preferably a mixture comprising sodium caseinate and a globular protein, preferably whey protein.
- optionally at least an outer mineral layer.

According to an embodiment, sodium caseinate and/or whey protein is (are) cross-linked protein(s).

The weight ratio between sodium caseinate and whey protein is preferably comprised between 0.01 and 100, preferably between 0.1 and 10, more preferably between 0.2 and 5.

In a particular embodiment, the microcapsule is a one-shell aminoplast core-shell microcapsule obtainable by a process comprising the steps of:
1) admixing a perfume oil with at least a polyisocyanate having at least two isocyanate functional groups to form an oil phase;
2) dispersing or dissolving into water an aminoplast resin and optionally a stabilizer to form a water phase;
3) preparing an oil-in-water dispersion, wherein the mean droplet size is comprised between 1 and 100 microns, by admixing the oil phase and the water phase;
4) performing a curing step to form the wall of said microcapsule; and
5) optionally drying the final dispersion to obtain the dried core-shell microcapsule.

In a particular embodiment, the core-shell microcapsule is a formaldehyde-free capsule. A typical process for the preparation of an aminoplast formaldehyde-free microcapsule slurry comprises the steps of
1) preparing an oligomeric composition comprising the reaction product of, or obtainable by reacting together:
   a. a polyamine component in the form of melamine or of a mixture of melamine and at least one C₁-C₄ compound comprising two NH₂ functional groups;
   b. an aldehyde component in the form of a mixture of glyoxal, a C₄₋₆ 2,2-dialkoxy-ethanal and optionally a glyoxalate, said mixture having a molar ratio glyoxal/C₄₋₆ 2,2-dialkoxy-ethanal comprised between 1/1 and 10/1; and
   c. a protic acid catalyst;
2) preparing an oil-in-water dispersion, wherein the droplet size is comprised between 1 and 600 microns, and comprising:
   a. an oil;
   b. a water medium:
   c. at least an oligomeric composition as obtained in step 1;
   d. at least a cross-linker selected amongst:
      i. C₄-C₁₂ aromatic or aliphatic di- or tri-isocyanates and their biurets, triurets, trimmers, trimethylol propane-adduct and mixtures thereof; and/or
      ii. a di- or tri-oxiran compounds of formula:

         Q-(oxiran-2-ylmethyl)ₘ

         wherein m stands for 2 or 3 and Q represents a C₂-C₆ group optionally comprising from 2 to 6 nitrogen and/or oxygen atoms;
   e. optionally a C₁-C₄ compound comprising two NH₂ functional groups;
3) heating the dispersion; and
4) cooling the dispersion.

The above process is described in more detail in WO 2013/068255.

In a particular embodiment of the core-shell microcapsules, the core-shell microcapsule is a polyamide core-shell polyamide microcapsule comprising:
- an oil based core comprising an hydrophobic active, preferably comprising at least one compound of formula (I), and
- a polyamide shell comprising or being obtainable from:
   - an acyl chloride,
   - a first amino compound, and
   - a second amino compound.

According to a particular embodiment, the polyamide core-shell microcapsule comprises:
an oil based core comprising an hydrophobic active, preferably comprising at least one compound of formula (I), and
a polyamide shell comprising or being obtainable from:
   - an acyl chloride, preferably in an amount comprised between 5 and 98%, preferably between 20 and 98%, more preferably between 30 and 85% w/w,
   - a first amino compound, preferably in an amount comprised between 1% and 50% w/w, preferably between 7 and 40% w/w;
   - a second amino compound, preferably in an amount comprised between 1% and 50% w/w, preferably between 2 and 25% w/w,
   - a stabilizer, preferably a biopolymer, preferably in an amount comprised between 0 and 90%, preferably between 0.1 and 75%, more preferably between 1 and 70%.

According to a particular embodiment, the polyamide core-shell microcapsule comprises:
- an oil-based core comprising a hydrophobic active, preferably comprising at least one compound of formula (I), and
- a polyamide shell comprising or being obtainable from:
   - an acyl chloride,
   - a first amino-compound being an amino-acid, preferably chosen from the group consisting of L-Lysine, L-Arginine, L-Histidine, L-Tryptophane and/or mixture thereof.
   - a second amino compound chosen from the group consisting of ethylene diamine, diethylene triamine, cystamine and/or mixture thereof, and
   - a biopolymer chosen from the group consisting of casein, sodium caseinate, bovin serum albumin, whey protein, and/or mixture thereof.

The first amino-compound can be different from the second amino-compound. Typically, a process for preparing a polyamide-based micrcoapsule includes the following steps:
a) dissolving at least one acyl chloride in a hydrophobic material, preferably a perfume to form an oil phase;
b) dispersing the oil phase obtained in step a) into a water phase comprising a first amino compound to form an oil-in water emulsion;
c) performing a curing step to form polyamide microcapsules in the form of a slurry;

wherein a stabilizer is added in the oil phase and/or in the water phase, and
wherein at least a second amino-compound is added in the water phase before the formation of the oil-in-water emulsion and/or in the oil-in water emulsion obtained after step b).

In a particular embodiment, the shell of the microcapsule is polyurea-or polyurethane-based. Examples of processes for the preparation of polyurea and polyureathane-based microcapsule slurries are for instance described in WO 2007/004166, EP 2300146, and EP 2579976. Typically, a process for the preparation of polyurea or polyurethane-based microcapsule slurries includes the following steps:
a) dissolving at least one polyisocyanate having at least two isocyanate groups in an oil to form an oil phase;
b) preparing an aqueous solution of an emulsifier or colloidal stabilizer to form a water phase;
c) adding the oil phase to the water phase to form an oil-in-water dispersion, wherein the mean droplet size is comprised between 1 and 500 µm, preferably between 5 and 50 µm; and
d) applying conditions sufficient to induce interfacial polymerization and form microcapsules in form of a slurry.

In a particular embodiment, the microcapsules can be in form of a powder, which in particular may be obtained by submitting the microcapsule slurry to a drying step, like spray-drying, to provide the microcapsules as such, i.e. in a powdery form. It is understood that any standard method known by a person skilled in the art to perform such drying is also applicable. In particular, the slurry may be spray-dried, preferably in the presence of a polymeric carrier material such as polyvinyl acetate, polyvinyl alcohol, dextrins, natural or modified starch, gum Arabic, vegetable gums, pectins, xanthans, alginates, carrageenans or cellulose derivatives to provide microcapsules in a powder form.

However, one may also cite other drying methods such as extrusion, plating, spray granulation, the fluidized bed process, or even drying at room temperature using materials (carriers, desiccants) that meet specific criteria as disclosed in WO 2017/134179.

The compounds of formula (I) can be prepared from phenyl ketone derivatives of formula (III) in a two-step sequence. In the first step the phenyl ketone is alpha-alkylated using (2,2-dimethyl-1,3-dioxolan-4-yl)methanol (solketal) (n =0) or 4-(hydroxymethyl)-2-phenyl-1,3-dioxane (n =1) in the presence of [Ir(cod)Cl]₂ as described in M. Rueping, V. B. Phapale, Green Chemistry, 2012, Vol. 14, pages 55-57. The [Ir(cod)Cl]₂ acts as a hydrogen-transfer catalyst, first dehydrogenating the primary alcohol function to form an aldehyde which undergoes an aldol condensation with the phenyl ketone. The Ir catalyst then hydrogenates the resulting α,β-unsaturated ketone. This results in a phenyl ketone with either 3,4- or 3,5-diol functions that are protected as cyclic acetals. In the second step of the process, the carbonyl compounds protecting the diol functions are replaced with the desired active aldehyde of formula R¹CHO or ketone of formula (R¹)(R²)C=O by means of an acid-catalyzed transacetalization reaction to form a compound of formula (I). An alternate synthetic strategy to obtain compounds of formula (I) (n=0) is to install an allylic group on the alkyl carbon alpha to the carbonyl group in compounds of formula (III). 1,2-Dihydroxylation of the alkene group followed by acetalization with a carbonyl compound of formula (II) would yield a compound of formula (I). Allylation can be achieved by means of a Claisen rearrangement of an in-situ formed allyl enol ether of the carbonyl compounds of formula (I). This was accomplished by heating a mixture of the dimethyl acetal of the phenyl ketones with allylic alcohols in the presence of an acid catalyst. Reduction of the carbonyl group with NaBH₄ to an alcohol and then conversion to an acetate ester masked the carbonyl group during subsequent reactions. The alkene was epoxidized using meta-chloroperbenzoic acid. By treating the resulting epoxide with acetone in the presence of FeCl₃ it was converted to the corresponding acetonide (S. Saha, S. K. Mandal, S. C. Roy, Tetrahedron Letters, 2008, Vol. 49, pages 5928-5930). Acid-catalyzed hydrolysis of the acetonide could yield the 1,2-diol that could be used to prepare cyclic acetals of carbonyl compound of formula (II). Alternatively, this was achieved in one step by the acid-catalyzed transacetalization reaction between the acetonide and the active carbonyl compounds of formula (II). Removal of the acetate group by methanolysis under basic conditions reformed the alcohol function and oxidization of the alcohol to a ketone yielded compounds of formula (I) (n=0).

The invention's compound of formula (I) is a delivery system able to release under certain conditions active compounds.

By the terms "active compounds", "active volatile compounds", "active volatile aldehyde, ketone or phenyl ketone" or the similar, it is meant here that the aldehyde, ketone or phenyl ketone to which it is referred is capable of bringing a benefit or effect into its surrounding environment. In particular, the active compound is selected from the group consisting of a perfuming ingredient, flavoring ingredient, pharmaceutical ingredient, cosmetic ingredient, agrochemical ingredient, malodor counteracting ingredient, antimicrobial ingredient and insect repellent or attractant ingredient. Therefore, to be considered as an "active compound" the compound has to possess at least one property which renders it useful as a perfuming or flavoring ingredient, pharmaceutical ingredient, cosmetic ingredient, agrochemical ingredient, malodor counteracting ingredient, antimicrobial ingredient and insect repellent or attractant ingredient. For a person skilled in the art, it is also evident that said active aldehydes or ketones are inherently volatile compounds.

The term "perfuming ingredient" is understood as a compound which is used, for the primary purpose, as an active ingredient in perfuming preparations or compositions in order to impart a hedonic effect. In other words, a compound to be considered as being a perfuming ingredient, must be recognized by a skilled person in the art of perfumery as being able to impart or modify in a positive or pleasant way the odor of a composition, and not just as having an odor. The perfuming ingredient may impart an additional benefit beyond that of modifying or imparting an odor, such as long-lastingness, blooming, malodor counteraction, a cosmetic effect, an antimicrobial effect, an antiviral effect, microbial stability, or pest control. The term "flavoring ingredient" is understood to as being capable of imparting a taste sensation to the taster's pallet. The term "malodor counteracting ingredient" is understood as being capable of reducing the perception of malodor, i.e. of an odor that is unpleasant or offensive to the human nose. The term "cosmetic ingredient" is understood as being capable of providing a cosmetic effect, such as e.g. a humidifying or skin caring effect. The term "antimicrobial ingredient" is understood as being capable of killing microorganism or reducing or preventing their growth and/or accumulation and include antibacterial, antibiotic, antifungal, antiviral and antiparasitic ingredients. The term "insect attractant or repellent" is understood as a compound having a positive or negative effect on insects. Examples of insect attractant or repellent ingredients can be found in reference texts or in other works of a similar nature as for example: A. M. El-Sayed, The Pherobase 2005, http://www.pherobase.net.

According to all the above and below mentioned embodiments of the invention, the invention's compound of formula (I) being a delivery system is particularly useful when the active volatile aldehyde, ketone or phenyl ketone released is a perfuming ingredient, i.e. a perfuming aldehyde, ketone or phenyl ketone. A "perfuming aldehyde, ketone or phenyl ketone" is a compound, which is of current use in the perfumery industry, i.e. a compound which is used as active ingredient in perfuming preparations or compositions in order to impart a hedonic effect. In other words, such an aldehyde, ketone or phenyl ketone, to be considered as being a perfuming one, must be recognized by a person skilled in the art of perfumery as being able to impart or modify in a positive or pleasant way the odor of a composition, and not just as having an odor. Said perfuming aldehyde, ketone or phenyl ketones can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery.

From now on we will refer to said "perfuming aldehyde, ketone or phenyl ketone" also as "perfuming compounds".

Practically, the invention is carried out exactly in the same manner, independently of the exact properties of the active, ketone or phenyl ketone. Therefore, it is understood that, even if the invention will be further illustrated herein below with a specific reference to "perfuming compounds", the below embodiments are also applicable to other active aldehydes or ketones (i.e. it is possible to replace the expression "perfuming" with "flavoring", "pharmaceutical", "agrochemical", "malodor counteracting", "cosmetic", "antibacterial", "antimicrobrial," "insect attractant" or with "insect repellent" for instance). According to a particular embodiment of the invention, active aldehydes are preferably used.

So, another object of the present invention is the use of the above-described compounds of formula (I) as delivery system to release perfuming compounds; i.e. use of a compound of formula (I) as defined above as perfuming ingredient to provide a long-lasting odor/effect. In other words, it concerns a method to confer, enhance, improve or modify the odor properties of a perfuming composition, the air surrounding the perfuming composition, a surface or a perfumed article, which method comprises adding to said composition, or article, or contacting or treating the surface with an effective amount of at least one compound of formula (I) as defined above. By "use of an invention's compound" it has to be understood here also the use of any composition containing said compounds and which can be advantageously employed in perfumery industry as active ingredients.

The term "surface", as used herein may refer to a user's skin, hair, a textile, or hard surface, on to which, a perfume composition comprising or containing the at least one compound of formula (I) is applied.

For sake of clarity, a long-lasting effect is typically achieved if, after a certain time, e.g. after several hours or days, a given compound emits higher amounts of an odor into the environment than a reference compound.

Said compositions, which in fact can be advantageously employed as perfuming ingredient, are also an object of the present invention.

Therefore, another object of the present invention is a perfuming composition comprising:
i) as perfuming ingredient, at least one of the invention's compounds of formula (I) as defined above;
ii) at least one ingredient selected from the group consisting of a perfumery carrier and a perfumery base; and
iii) optionally at least one perfumery adjuvant.

By "perfumery carrier" it is meant here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples, solvents such as butylene or propylene glycol, glycerol, dipropyleneglycol and its monoether, 1,2,3-propanetriyl triacetate, dimethyl glutarate, dimethyl adipate 1,3-diacetyloxypropan-2-yl acetate, diethyl phthalate, isopropyl myristate, Abalyn^{®} (rosin resins, available from Eastman), benzyl benzoate, benzyl alcohol, 2-(2-ethoxyethoxy)-1-ethano, tri-ethyl citrate or mixtures thereof, which are the most commonly used or also naturally derived solvents like glycerol or various vegetable oils such as palm oil, sunflower oil or linseed oil. For the compositions which comprise both a perfumery carrier and a perfumery base, other suitable perfumery carriers than those previously specified, can be also ethanol, water/ethanol mixtures, limonene or other terpenes, isoparaffins such as those known under the trademark Isopar (origin: Exxon Chemical) or glycol ethers and glycol ether esters such as those known under the trademark Dowanol (origin: Dow Chemical Company), or hydrogenated castor oils such as those known under the trademark Cremophor RH 40 (origin: BASF).

Solid carrier is meant to designate a material to which the perfuming composition or some element of the perfuming composition can be chemically or physically bound. In general, such solid carriers are employed either to stabilize the composition, or to control the rate of evaporation of the compositions or of some ingredients. Solid carrier are of current use in the art and a person skilled in the art knows how to reach the desired effect. However, by way of non-limiting example of solid carriers, one may cite absorbing gums or polymers or inorganic material, such as porous polymers, cyclodextrins, dextrins, maltodextrins, wood-based materials, organic or inorganic gels, clays, gypsum, talc or zeolites.

As other non-limiting examples of solid carriers, one may cite encapsulating materials. Examples of such materials may comprise wall-forming and plasticizing materials, such as glucose syrups, natural or modified starches, hydrocolloids, cellulose derivatives, polyvinyl acetates, polyvinylalcohols, proteins or pectins, plant gums such as acacia gum (Gum Arabic), urea, sodium chloride, sodium sulfate, sodium carbonate, sodium bicarbonate, calcium carbonate, magnesium sulfate, calcium sulfate, magnesium oxide, zinc oxide, titanium dioxide, calcium chloride, potassium chloride, magnesium chloride, zinc chloride, carbohydrates, saccharides such as sucrose, mono-, di-, tri- and polysaccharides and derivatives such as chitosan, starch, cellulose, carboxymethyl methylcellulose, methylcellulose, hydroxyethyl cellulose, ethyl cellulose, propyl cellulose, polyols/sugar alcohols such as sorbitol, maltitol, xylitol, erythritol and isomalt, polyethylene glycol (PEG), polyvinyl pyrrolidin (PVP), polyvinyl alcohol, acrylamides, acrylates, polyacrylic acid and related structures, maleic anhydride copolymers, amine-functional polymers, vinyl ethers, styrenes, polystyrenesulfonates, vinyl acids, ethylene glycol-propylene glycol block copolymers, pectins, xanthanes, alginates, carragenans, citric acid or any water soluble solid acid, fatty alcohols or fatty acids and mixtures thereof, or yet the materials cited in reference texts such as H. Scherz, Hydrokolloide: Stabilisatoren, Dickungs- und Geliermittel in Lebensmitteln, Band 2 der Schriftenreihe Lebensmittelchemie, Lebensmittelqualität, Behr's Verlag GmbH & Co., Hamburg, 1996. The encapsulation is a well-known process to a person skilled in the art, and may be performed, for instance, by using techniques such as spray-drying, agglomeration or yet extrusion; or consists of a coating encapsulation, including coacervation and complex coacervation techniques.

As non-limiting examples of solid carriers, one may cite in particular the core-shell capsules with resins of aminoplast, polyamide, polyester, polyurea or polyurethane type or a mixture thereof (all of said resins are well known to a person skilled in the art) using techniques like phase separation process induced by polymerization, interfacial polymerization, coacervation or altogether (all of said techniques have been described in the prior art), optionally in the presence of a polymeric stabilizer or of a cationic copolymer.

Resins may be produced by the polycondensation of an aldehyde (e.g. formaldehyde, 2,2-dimethoxyethanal, glyoxal, glyoxylic acid or glycolaldehyde and mixtures thereof) with an amine such as urea, benzoguanamine, glycouryl, melamine, methylol melamine, methylated methylol melamine, guanazole and the like, as well as mixtures thereof. Alternatively, one may use preformed resins alkylolated polyamines such as those commercially available under the trademark Urac^{®} (origin: Cytec Technology Corp.), Cymel^{®} (origin: Cytec Technology Corp.), Urecoll^{®} or Luracoll^{®} (origin: BASF).

Other resins are those produced by the polycondensation of an a polyol, like glycerol, and a polyisocyanate, like a trimer of hexamethylene diisocyanate, a trimer of isophorone diisocyanate or xylene diisocyanate or a Biuret of hexamethylene diisocyanate or a trimer of xylene diisocyanate with trimethylolpropane (known with the tradename of Takenate^{®}, origin: Mitsui Chemicals), among which a trimer of xylene diisocyanate with trimethylolpropane and a Biuret of hexamethylene diisocyanate are preferred.

Some of the seminal literature related to the encapsulation of perfumes by polycondensation of amino resins, namely melamine-based resins with aldehydes includes articles such as those published by K. Dietrich et al. Acta Polymerica, 1989, Vol. 40, pages 243, 325 and 683, as well as 1990, Vol. 41, page 91. Such articles already describe the various parameters affecting the preparation of such core-shell microcapsules following prior art methods that are also further detailed and exemplified in the patent literature. US 4'396'670, to the Wiggins Teape Group Limited, is a pertinent early example of the latter. Since then, many other authors have enriched the literature in this field and it would be impossible to cover all published developments here, but the general knowledge in encapsulation technology is very significant. More recent publications of pertinence, which disclose suitable uses of such microcapsules, are represented for example by the article of K. Bruyninckx and M. Dusselier, ACS Sustainable Chemistry & Engineering, 2019, Vol. 7, pages 8041-8054.

By "perfumery base" what is meant here is a composition comprising at least one perfuming co-ingredient.

The perfuming co-ingredient is not a compound according to the invention. Moreover, by the term "perfuming co-ingredient" is meant a perfuming ingredient as defined above.

The nature and type of the perfuming co-ingredients present in the base do not warrant a more detailed description here, which in any case would not be exhaustive, the skilled person being able to select them on the basis of general knowledge and according to intended use or application and the desired organoleptic effect. In general terms, these perfuming co-ingredients belong to chemical classes as varied as alcohols, lactones, aldehydes, ketones, esters, ethers, acetates, nitriles, thiols, terpene hydrocarbons, nitrogenous or sulfurous heterocyclic compounds and essential oils, and the perfuming co-ingredients can be of natural or synthetic origin. In particular, one may cite perfuming co-ingredients which are commonly used in perfume formulations, such as:
- Aldehydic ingredients: decanal, dodecanal, 2-methyl-undecanal, 10-undecenal, octanal, nonanal and/or nonenal;
- Aromatic-herbal ingredients: eucalyptus oil, camphor, eucalyptol, 5-methyltricyclo[6.2.1.0^{2,7}]undecan-4-one, 1-methoxy-3-hexanethiol, 2-ethyl-4,4-dimethyl-1,3-oxathiane, 2,2,7/8,9/10-tetramethylspiro[5.5]undec-8-en-1-one, menthol and/or alpha-pinene;
- Balsamic ingredients: ethylvanillin and/or vanillin;
- Citrus ingredients: dihydromyrcenol, citral, orange oil, linalyl acetate, citronellyl nitrile, orange terpenes, limonene, 1-p-menthen-8-yl acetate and/or 1,4(8)-p-menthadiene;
- Floral ingredients: methyl dihydrojasmonate, linalool, citronellol, phenylethanol, 3-(4-tert-butylphenyl)-2-methylpropanal, benzyl acetate, benzyl salicylate, tetrahydro-2-isobutyl-4-methyl-4(2H)-pyranol, beta ionone, (E)-3-methyl-4-(2,6,6-trimethyl-2-cyclohexen-1-yl)-3-buten-2-one, (1E)-1-(2,6,6-trimethyl-2-cyclohexen-1-yl)-1-penten-3-one, (2E)-1-(2,6,6-trimethyl-2-cyclohexen-1-yl)-2-buten-1-one, (2E)-1-[2,6,6-trimethyl-3-cyclohexen-1-yl]-2-buten-1-one, (2E)-1-(2,6,6-trimethyl-1-cyclohexen-1-yl)-2-buten-1-one, 2,5-dimethyl-2-indanmethanol, 2,6,6-trimethyl-3-cyclohexene-1-carboxylate, 3-(4,4-dimethyl-1-cyclohexen-1-yl)propanal, hexyl salicylate, 3,7-dimethyl-1,6-nonadien-3-ol, 3-(4-isopropylphenyl)-2-methylpropanal, verdyl acetate, geraniol, p-menth-1-en-8-ol, 4-(1,1-dimethylethyl)-1-cyclohexyle acetate, 1,1-dimethyl-2-phenylethyl acetate, 4-cyclohexyl-2-methyl-2-butanol, amyl salicylate , high cis methyl dihydrojasmonate, 3-methyl-5-phenyl-1-pentanol, verdyl proprionate, geranyl acetate, tetrahydro linalool, cis-7-p-menthanol, propyl (S)-2-(1,1-dimethylpropoxy)propanoate, 2,2,2-trichloro-1-phenylethyl acetate, 4/3-(4-hydroxy-4-methylpentyl)-3-cyclohexene-1-carbaldehyde, 8-decen-5-olide, 4-phenyl-2-butanone, isononyle acetate, 4-(1,1-dimethylethyl)-1-cyclohexyl acetate, verdyl isobutyrate and/or mixture of methylionone isomers;
- Fruity ingredients: gamma-undecalactone, 2,2,5-trimethyl-5-pentylcyclopentanone, 2-methyl-4-propyl-1,3-oxathiane, 4-decanolide, ethyl 2-methyl-pentanoate, hexyl acetate, ethyl 2-methylbutanoate, gamma-nonalactone, allyl heptanoate, 2-phenoxyethyl isobutyrate, ethyl 2-methyl-1,3-dioxolane-2-acetate, 3-(3,3/1,1-dimethyl-5-indanyl)propanal, diethyl 1,4-cyclohexanedicarboxylate, 3-methyl-2-hexen-1-yl acetate, 1-[3,3-dimethylcyclohexyl]ethyl [3-ethyl-2-oxiranyl]acetate and/or diethyl 1,4-cyclohexane dicarboxylate;
- Green ingredients: 2-methyl-3-hexanone (E)-oxime, 2,4-dimethyl-3-cyclohexene-1-carbaldehyde, 2-tert-butyl-1-cyclohexyl acetate, styrallyl acetate, allyl (2-methylbutoxy)acetate, 4-methyl-3-decen-5-ol, diphenyl ether, (Z)-3-hexen-1-ol and/or 1-(5,5-dimethyl-1-cyclohexen-1-yl)-4-penten-1-one;
- Musk ingredients: 1,4-dioxa-5,17-cycloheptadecanedione, (Z)-4-cyclopentadecen-1-one, 3-methylcyclopentadecanone, 1-oxa-12-cyclohexadecen-2-one, 1-oxa-13-cyclohexadecen-2-one, (9Z)-9-cycloheptadecen-1-one, 2-{1S)-1-[(1R)-3,3-dimethylcyclohexyl]ethoxyl-2-oxoethyl propionate 3-methyl-5-cyclopentadecen-1-one, 4,6,6,7,8,8-hexamethyl-1,3,4,6,7,8-hexahydrocyclopenta[g]isochromene, (1S,1'R)-2-[1-(3',3'-dimethyl-1'-cyclohexyl)ethoxy]-2-methylpropyl propanoate, oxacyclohexadecan-2-oneand/or (1S,1'R)-[1-(3',3'-dimethyl-1'-cyclohexyl)ethoxycarbonyl]methyl propanoate;
- Woody ingredients: 1-[(1RS,6SR)-2,2,6-trimethylcyclohexyl]-3-hexanol, 3,3-dimethyl-5-[(1R)-2,2,3-trimethyl-3-cyclopenten-1-yl]-4-penten-2-ol, 3,4'-dimethylspiro[oxirane-2,9'-tricyclo[6.2.1.02,7]undec[4]ene, (1-ethoxyethoxy)cyclododecane, 2,2,9,11-tetramethylspiro[5.5]undec-8-en-1-yl acetate, 1-(octahydro-2,3,8,8-tetramethyl-2-naphtalenyl)-1-ethanone, patchouli oil, terpenes fractions of patchouli oil, Clearwood^{®}, (1'R,E)-2-ethyl-4-(2',2',3'-trimethyl-3'-cyclopenten-1'-yl)-2-buten-1-ol, 2-ethyl-4-(2,2,3-trimethyl-3-cyclopenten-1-yl)-2-buten-1-ol, methyl cedryl ketone, 5-(2,2,3-trimethyl-3-cyclopentenyl)-3-methylpentan-2-ol, 1-(2,3,8,8-tetramethyl-1,2,3,4,6,7,8,8a-octahydronaphthalen-2-yl)ethan-1-one and/or isobornyl acetate;
- Other ingredients (e.g. amber, powdery spicy or watery): dodecahydro-3a,6,6,9a-tetramethyl-naphtho[2,1-b]furan and any of its stereoisomers, heliotropin, anisic aldehyde, eugenol, cinnamic aldehyde, clove oil, 3-(1,3-benzodioxol-5-yl)-2-methylpropanal, 7-methyl-2H-1,5-benzodioxepin-3(4H)-one, 2,5,5-trimethyl-1,2,3,4,4a,5,6,7-octahydro-2-naphthalenol, 1-phenylvinyl acetate, 6-methyl-7-oxa-1-thia-4-azaspiro[4.4]nonan and/or 3-(3-isopropyl-1-phenyl)butanal.

A composition according to the invention may not be limited to the above-mentioned perfuming co-ingredients, and many other of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds also known as properfumes or profragrances. Non-limiting examples of suitable properfumes may include 4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, 3-(dodecylthio)-1-(2,6,6-trimethyl-3-cyclohexen-1-yl)-1-butanone, 3-(dodecylsulfonyl)-1-(2,6,6-trimethylcyclohex-3-en-1-yl)butan-1-one, a linear polysiloxane co-polymer of (3-mercaptopropyl)(methyl)dimethoxysilane, 3-(dodecylthio)-1-(6-ethyl-2,6-dimethylcyclohex-3-en-1-yl)butan-1-one, 2-(dodecylthio)octan-4-one, 2-(dodecylsulfonyl)octan-4-one, 4-oxooctan-2-yl dodecanoate, 2-phenylethyl oxo(phenyl)acetate, 3,7-dimethylocta-2,6-dien-1-yl oxo(phenyl)acetate, (Z)-hex-3-en-1-yl oxo(phenyl)acetate, 3,7-dimethyl-2,6-octadien-1-yl hexadecanoate, bis(3,7-dimethylocta-2,6-dien-1-yl) succinate, (2E,6Z)-2,6-nonadienyl hexadecanoate, (2E,6Z)-2,6-nonadien-1-yl tetradecanoate, (2E,6Z)-nona-2,6-dien-1-yl dodecanoate, (2E,6Z)-nona-2,6-dien-1-yl hexadecanoate, (2-((2-methylundec-1-en-1-yl)oxy)ethyl)benzene, 1-methoxy-4-(3-methyl-4-phenethoxybut-3-en-1-yl)benzene, (3-methyl-4-phenethoxybut-3-en-1-yl)benzene, 1-(((Z)-hex-3-en-1-yl)oxy)-2-methylundec-1-ene, (2-((2-methylundec-1-en-1-yl)oxy)ethoxy)benzene, 2-methyl-1-(octan-3-yloxy)undec-1-ene, 1-methoxy-4-(1-phenethoxyprop-1-en-2-yl)benzene, 1-methyl-4-(1-phenethoxyprop-1-en-2-yl)benzene, (2-phenethoxyvinyl)benzene, (2-((2-pentylcyclopentylidene)methoxy)ethyl)benzene, 4-allyl-2-methoxy-1-((2-methoxy-2-phenylvinyl)oxy)benzene, (2-((2-heptylcyclopentylidene)methoxy)ethyl)benzene, 1-methoxy-4-(1-phenethoxyprop-1-en-2-yl)benzene, (2-((2-methyl-4-(2,6,6-trimethylcyclohex-1-en-1-yl)but-1-en-1-yl)oxy)ethyl)benzene, 1-methoxy-4-(2-methyl-3-phenethoxyallyl)benzene, (2-((2-isopropyl-5-methylcyclohexylidene)methoxy)ethyl)benzene, 1-isopropyl-4-methyl-2-((2-pentylcyclopentylidene)methoxy)benzene, 2-methoxy-1-((2-pentylcyclopentylidene)methoxy)-4-propylbenzene, 3-methoxy-4-((2-methoxy-2-phenylvinyl)oxy)benzaldehyde, 1-isopropyl-2-((2-methoxy-2-phenylvinyl)oxy)-4-methylbenzene, 4-((2-(hexyloxy)-2-phenylvinyl)oxy)-3-methoxybenzaldehyde 3-methyl-5-phenylpentyl palmitate, 3-(dodecylthio)-2-methyl-1-(2,6,6-trimethylcyclohex-3-en-1-yl)butan-1-one, 3,5-bis(1-(4-isopropylphenyl)propan-2-yl)dihydro-1H,3H,5H-oxazolo[3,4-c]oxazole, 3,5-di(undecan-2-yl)dihydro-1H,3H,5H-oxazolo[3,4-c]oxazole, 3,5-bis(2,4-dimethylcyclohex-3-en-1-yl)dihydro-1H,3H,5H-oxazolo[3,4-c]oxazole, ethyl 2-acetyl-4-methyltridec-2-enoate, dec-9-en-1-yl (E)-3-(2-hydroxyphenyl)acrylate, 4-(dodecylthio)-4-methylpentan-2-one, methyl or ethyl N,S-bis(4-oxo-4-(2,6,6-trimethylcyclohex-3-en-1-yl)butan-2-yl)-L-cysteinate, 1-butoxy-3-((1E,4Z)-hepta-1,4-dien-1-yl)benzene, 2-methoxy-4-((1E,4Z)-hepta-1,4-dien-1-yl)phenol, 2-ethoxy-4-((1E,4Z)-hepta-1,4-dien-1-yl)phenol or a mixture thereof.

In a particular embodiment, the perfuming composition according to the invention comprises a perfumery adjuvant.

The term "perfumery adjuvant" is understood as an ingredient capable of imparting additional added benefit such as a color, a particular light resistance, chemical stability and etc. A detailed description of the nature and type of adjuvant commonly used in perfuming bases cannot be exhaustive, but it has to be mentioned that the ingredients are well known to a person skilled in the art. However, one may cite as specific non-limiting examples the following: viscosity agents (e.g. surfactants, thickeners, gelling and/or rheology modifiers), stabilizing agents (e.g. preservatives, antioxidants, heat/light and or buffers or chelating agents, such as BHT), coloring agents (e.g. dyes and/or pigments), preservatives (e.g. antibacterial or antimicrobial or antifungal or anti-irritant agents), abrasives, skin cooling agents, fixatives, insect repellants, ointments, vitamins and mixture thereof. By "fixative" also called "modulator", it is understood here an agent having the capacity to affect the manner in which the odor, and in particular the evaporation rate and intensity, of the compositions incorporating said modulator can be perceived by an observer or user thereof, over time, as compared to the same perception in the absence of the modulator. In particular, the modulator allows prolonging the time during which their fragrance is perceived. Non-limiting examples of suitable modulators may include methyl glucoside polyol; ethyl glucoside polyol; propyl glucoside polyol; isocetyl alcohol; PPG-3 myristyl ether; neopentyl glycol diethylhexanoate; sucrose laurate; sucrose dilaurate, sucrose myristate, sucrose palmitate, sucrose stearate, sucrose distearate, sucrose tristearate, hyaluronic acid disaccharide sodium salt, sodium hyaluronate, propylene glycol propyl ether; dicetyl ether; polyglycerin-4 ethers; isoceteth-5; isoceteth-7, isoceteth-10; isoceteth-12; isoceteth-15; isoceteth-20; isoceteth-25; isoceteth-30; disodium lauroamphodipropionate; hexaethylene glycol monododecyl ether; and their mixtures; neopentyl glycol diisononanoate; cetearyl ethylhexanoate; panthenol ethyl ether, DL-panthenol, n-hexadecyl n-nonanoate, noctadecyl n-nonanoate, cyclodextrin, and a combination thereof. At most 20% by weight, based on the total weight of the perfuming composition, of the modulator may be incorporated into the perfumed consumer product.

It is understood that a person skilled in the art is perfectly able to design optimal formulations for the desired effect by admixing the above-mentioned components of a perfuming composition, simply by applying the standard knowledge of the art as well as by trial and error methodologies.

An invention's composition consisting of at least one compound of formula (I) and at least one perfumery carrier consists of a particular embodiment of the invention as well as a perfuming composition comprising at least one compound of formula (I), at least one perfumery carrier, at least one perfumery base, and optionally at least one perfumery adjuvant.

According to a particular embodiment, the compositions mentioned above, comprise more than one compound of formula (I) and enable the perfumer to prepare accords or perfumes possessing the odor tonality of various compounds of the invention, creating thus new building block for creation purposes.

For the sake of clarity, it is also understood that any mixture resulting directly from a chemical synthesis, e.g. a reaction medium without an adequate purification, in which the compound of the invention would be involved as a starting, intermediate or end-product could not be considered as a perfuming composition according to the invention as far as said mixture does not provide the inventive compound in a suitable form for perfumery. Thus, unpurified reaction mixtures are generally excluded from the present invention unless otherwise specified.

The invention's compound can also be advantageously used in all the fields of modern perfumery, i.e. fine or functional perfumery, to positively impart or modify the odor of a consumer product into which said compound (I) is added. Consequently, another object of the present invention consists of a perfumed consumer product comprising, as a perfuming ingredient, at least one compound of formula (I), as defined above.

The invention's compound can be added as such or as part of an invention's perfuming composition.

For the sake of clarity, it has to be mentioned that the term "perfumed consumer product" is understood as a consumer product, which is expected to deliver at least a pleasant perfuming effect to the surface to which it is applied (e.g. skin, hair, textile, or hard surface). In other words, a perfumed consumer product according to the invention is a perfumed consumer product, which comprises the inventive compound or perfuming composition, as well as optionally additional benefit agents, corresponding to the desired consumer product, e.g. a conditioner, a detergent or an air freshener, and an olfactorily effective amount of the perfuming composition according to the invention. For the sake of clarity, the perfuming consumer product is a non-edible product.

The nature and type of the constituents of the perfuming consumer product do not warrant a more detailed description here, which in any case would not be exhaustive, the skilled person being able to select them on the basis of his general knowledge and according to the nature and the desired effect of the product.

In a particular embodiment, the perfumed consumer product is a perfume, a fabric care product, a body-care product, a cosmetic preparation, a skin-care product, an air care product or a home care product.

Non-limiting examples of suitable perfumed consumer products include a perfume, such as a fine perfume, a splash or an eau de parfum, a cologne or a shave or after-shave lotion; a fabric care product, such as a liquid or solid detergent optionally in the form of a pod or tablet, a fabric softener, a liquid or solid scent booster, a dryer sheet, a fabric refresher, an ironing water, a paper, a bleach, a carpet cleaner, a curtain-care product; a body-care product, such as a hair care product (e.g. a shampoo, a leave-on or rinse-off hair conditioner, a coloring preparation or a hair spray, a color-care product, a hair shaping product, a dental care product), a disinfectant, an intimate care product; a cosmetic preparation (e.g. a skin cream or lotion, a vanishing cream or a deodorant or antiperspirant (e.g. a spray or roll on), a hair remover, a tanning or sun or after sun product, a nail product, a skin cleansing, a makeup); or a skin-care product (e.g. a soap, a shower or bath mousse, oil or gel, or a hygiene product or a foot/hand care product); an air care product, such as an air freshener or a "ready to use" powdered air freshener which can be used in the home space (rooms, refrigerators, cupboards, shoes or car) and/or in a public space (halls, hotels, malls, etc..); or a home care product, such as a mold remover, a furniture care product, a wipe, a dish detergent or a hard-surface (e.g. a floor, bath, sanitary or a window-cleaning) detergent; a leather care product; a car care product, such as a car air-freshener, a polish, a wax or a plastic cleaner. Particularly, the perfumed consumer product may be a liquid or solid detergent, a fabric softener, a fabric refresher, an ironing water, a paper, a bleach, a carpet cleaner, a curtain-care product, a disinfectant, a hygiene product, an air freshener, a "ready to use" powdered air freshener, a mold remover, a furnisher care product, a wipe, a dish detergent or a hard-surface detergent, a leather care product or a car care product.

According to a particular embodiment, the invention's perfumed consumer product is in the form of a personal care, a home care or fabric care consumer product comprising ingredients that are common in personal, home or fabric care consumer products, in particular shower gels, shampoos, soaps, fabric detergents or softeners and all-purpose cleaners. The main functional constituents of perfumed consumer products are surfactants and/or softener components capable of cleaning and/or softening fabrics and/or textiles of varied nature, such as clothes, curtain fabrics, carpets and furniture fabrics, etc., or other home surfaces, skin or hair, and typically used in a large amount of water or water-based solvents. These are therefore formulations wherein the amount of water is typically comprised between 50 and 99% by weight of the perfumed consumer product with the exception of soaps or solid detergents, wherein the amount of water is at most 20%.

A more detailed description of such fabric cleaning and/or softening formulations is not warranted here, many descriptions of current liquid formulations can be found in the cleaner/fabric softener's patent and other pertinent literature, such as for example the textbook of Louis Ho Tan Tai, "Détergents et Produits de Soins Corporels, Chapters 1 to 7 in particular, Dunod, Paris, 1999, or any other similar and/or more recent textbooks pertaining to the art of liquid softener and all-purpose cleaners formulations. A patent publication, WO 2010/105873, is also cited by way of example, in as much as it describes typical current ingredients, other than perfumes, of such liquid products, particularly on pages 9 to 21. Of course, many other examples of liquid cleaner and/or fabric softener formulations can be found in the literature. Any such liquid formulations, namely liquid fabric cleaners or conditioners and/or all-purpose cleaners, can be used in the here-described compositions. Other examples of fabric detergents or softener compositions into which the compounds of the invention can be incorporated are described in WO 97/34986 or in US patents 4,137,180 and 5,236,615 or EP 799 885. Other typical detergent and softening compositions which can be used are described in works such as Ullmann's Encyclopedia of Industrial Chemistry, Vol. 20, Wiley-VCH, Weinheim, p. 355-540 (2012); Flick, Advanced Cleaning Product Formulations, Noye Publication, Park Ridge, New Jersey (1989); Showell, in Surfactant Science Series, Vol. 71: Powdered Detergents, Marcel Dekker, New York (1988); Proceedings of the World Conference on Detergents (4th, 1998, Montreux, Switzerland), AOCS print.

According to a particular embodiment of the invention, the invention's perfumed consumer product may be a liquid fabric softener comprising at least one compound of formula (I) and a fabric softener active base in amount comprised between 85 and 100% by weight, based on the total weight of the perfumed consumer product. The main constituent of the fabric softener active base is water or water-based solvents. The fabric softener active base may comprise dialkyl quaternary ammonium salts, dialkyl ester quaternary ammonium salts, Hamburg esterquat, triethanolamine quat, silicones and mixtures thereof. Optionally, component a) of the composition may further comprise a viscosity modifier in an amount comprised between 0.05 and 1% by weight, based on the total weight of the liquid base; preferably chosen from the group consisting of calcium chloride.

According to a particular embodiment of the invention, the invention's consumer product is an all-purpose cleaner comprising at least one compound of formula (I) and an all-purpose cleaner active base in amount comprised between 85 and 100% by weight, based on the total weight of the consumer product. The main constituent of the all-purpose cleaner active base is water or water-based solvents. The all-purpose active base may comprise linear alkylbenzene sulfonates (LAS) in an amount comprised between 0 and 4%, preferably 1 and 2%, nonionic surfactant in an amount comprised between 0 and 8%, preferably 2 and 4% and acid such as citric acid in an amount comprised between 0.1 and 0.5%.

According to a particular embodiment of the invention, the invention's consumer product is a liquid detergent comprising at least one compound of formula (I) and liquid detergent active base in amount comprised between 85 and 100% by weight, based on the total weight of the consumer product. The main constituent of the liquid detergent active base active base is water or water-based solvents. The liquid detergent active base may comprise anionic surfactant such as alkylbenzenesulfonate (ABS), linear alkylbenzene sulfonates (LAS), secondary alkyl sulfonate (SAS), primary alcohol sulfate (PAS), lauryl ether sulfate (LES), sodium lauryl ether sulfate (SLES), methyl ester sulfonate (MES); nonionic surfactant such as alkyl amines, alkanolamide, fatty alcohol poly(ethylene glycol) ether, fatty alcohol ethoxylate (FAE), ethylene oxide (EO) and propylene oxide (PO) copolymers, amine oxides, alkyl polyglucosides, alkyl polyglucosamides; ormixtures thereof.

According to a particular embodiment of the invention, the invention's consumer product is a solid detergent comprising at least one compound of formula (I) and a solid detergent active base in amount comprised between 85 and 100% by weight, based on the total weight of the consumer product. The solid detergent active base may comprise at least one surfactant chosen from the group consisting of anionic, nonionic, cationic, zwiterionic surfactant and mixtures thereof. The surfactant in the solid detergent active base is preferably chosen from the group consisting of linear alkene benzene sulfonate (LABS), sodium laureth sulfate, sodium lauryl ether sulfate, sodium lauryl sulfate (SLS), alpha olefin sulfonate (AOS), methyl ester sulfonates, alkyl polyglyucosides (APG), primary alcohol ethoxylates and in particular lauryl alcohol ethoxylates (LAE), primary alcohol sulfonates, soap and mixtures thereof. The soild detergent active base may comprise a further component, commonly used in powder detergent consumer product, selected from the group consisting of bleaching agents such as TAED (tetraacetylethylenediamine); buffering agent; builders such as zeolites, sodium carbonate or mixture thereof; soil release or soil suspension polymers; granulated enzyme particles such as cellulase, lipase, protease, mannanase, pectinase or mixtures thereof; corrosion inhibitor; antifoaming; sud suppressing agents; dyes; fillers such as sodium silicate, sodium sulfate or mixture thereof; source of hydrogen peroxide such as sodium percarbonate or sodium perborate; and mixtures thereof.

The proportions in which the perfuming composition according to the invention can be incorporated into the various aforementioned articles or compositions vary within a wide range of values. These values are dependent upon the nature of the article or product to be perfumed and on the desired olfactory effect as well as the nature of the co-ingredients in a given composition when the compounds according to the invention are mixed with perfuming co-ingredients, solvents or additives commonly used in the art.

For example, in the case of perfuming compositions, typical concentrations are in the order of 0.001 % to 10 % by weight, or even more, of the compounds of the invention based on the weight of the composition into which they are incorporated. In the case of perfumed consumer product, typical concentrations are in the order of 0.0001 % to 1 % by weight, or even more, of the compounds of the invention based on the weight of the consumer product into which they are incorporated.

Another aspect of the invention concerns the use of a perfuming composition according to the invention for improving, enhancing, conferring and/or modifying the fragrance impression and/or fragrance intensity of a consumer product.

Another aspect of the invention concerns a method for improving, enhancing, conferring and/or modifying the fragrance impression and/or fragrance intensity of a consumer product, comprising the step of adding the perfuming composition according to the invention to a consumer product.

Another aspect of the invention is a method to release from a precursor compound, compounds selected from the group consisting of
a) a carbonyl compound of formula 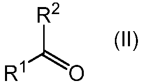 in the form of any one of its stereoisomers or a mixture thereof, and wherein R¹ represents a C₁-₁₈ hydrocarbon group, optionally comprising one to three oxygen atoms and/or one to two nitrogen atoms and/or one sulfur atom; R² represents, a hydrogen atom or a R¹ group; or R¹ and R², when taken together, form a C₅₋₁₆ cycloalkyl, C₅₋₁₆ cycloalkenyl, C₄₋₁₄ heterocycloalkyl or C₄₋₁₄ heterocycloalkenyl group, each optionally substituted with one or more of a C₁₋₁₅ alkyl, C₂₋₁₅ alkenyl, C₁₋₁₅ alkoxy, C₃₋₁₅ cycloalkyl, C₅₋₁₅ cycloalkenyl, C₆₋₁₀ aryl and/or C₆₋₁₀ aryloxy group, each optionally substituted with one or more of a C₁₋₈ alkyl, C₁₋₈ alkoxy, carboxylic acid and/or C₁₋₄ carboxylic ester group, wherein the heteroatom represents one or more of an oxygen atom;
b) a ketone of formula 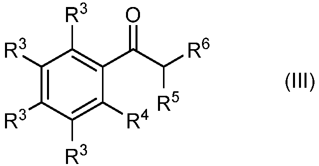 in the form of any one of its stereoisomers or a mixture thereof, and wherein R³ and R⁴ represent, independent of each other, a hydrogen atom, a C₁₋₆ alkoxy group or a C₁₋₁₂ alkyl group, optionally substituted by a hydroxy, C₁₋₆ alkoxy or oxo group, or, two adjacent R³ groups, when taken together, are a C₃₋₈ linear alkanediyl group optionally substituted by one or more of a hydroxyl, C₁₋₃ alkyl and/or C₁₋₃ alkoxy group; R⁵ represents a hydrogen atom or a C₁₋₆ hydrocarbon group, or R⁴ and R⁵, when taken together, represent a C₁₋₄ linear, branched or cyclic alkanediyl group, optionally comprising one oxygen atom; R⁶ represents a hydrogen atom or a methyl group;
   wherein the precursor compound is a compound of formula (I) 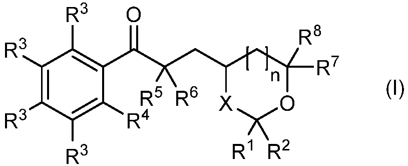 in the form of any one of its stereoisomers or a mixture thereof, and wherein R¹, R², R³, R⁴, R⁵ and R⁶ have the same meaning as defined above; n is 0 or 1, X is an oxygen atom or a NR⁹ group wherein R⁹ is a hydrogen atom or a methyl group and R⁷ and R⁸ represent, independent of each other, a hydrogen atom or a C₁ to C₆ alkyl group;
by exposing the precursor compound of formula (I) to light and to an environment wherein the compound is hydrolyzed.

In a further aspect, the present invention also relates to the use of precursor compounds for releasing compounds selected from the group consisting of
a) a carbonyl compound of formula 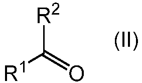 wherein R¹ represents a C₁-₁₈ hydrocarbon group, optionally comprising one to three oxygen atoms and/or one to two nitrogen atoms and/or one sulfur atom; R² represents a hydrogen atom or a R¹ group; or R¹ and R², when taken together, form a C₅₋₁₆ cycloalkyl, C₅₋₁₆ cycloalkenyl, C₄₋₁₄ heterocycloalkyl or C₄₋₁₄ heterocycloalkenyl group, each optionally substituted with one or more of a C₁₋₁₅ alkyl, C₂₋₁₅ alkenyl, C₁₋₁₅ alkoxy, C₃₋₁₅ cycloalkyl, C₅₋₁₅ cycloalkenyl, C₆₋₁₀ aryl and/or C₆₋₁₀ aryloxy group, each optionally substituted with one or more of a C₁₋₈ alkyl, C₁₋₈ alkoxy, carboxylic acid and/or C₁₋₄ carboxylic ester group, wherein the heteroatom represents one or more of an oxygen atom;
b) a ketone of formula 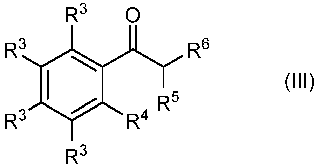 wherein R³ and R⁴ represent, independent of each other, a hydrogen atom, a C₁₋₆ alkoxy group or a C₁₋₁₂ alkyl group, optionally substituted by a hydroxy, C₁₋₆ alkoxy or oxo group, or, two adjacent R³ groups, when taken together, are a C₃₋₈ linear alkanediyl group optionally substituted by one or more of a hydroxyl, C₁₋₃ alkyl and/or C₁₋₃ alkoxy group; R⁵ represents a hydrogen atom or a C₁₋₆ hydrocarbon group, or R⁴ and R⁵, when taken together, represent a C₁₋₄ linear, branched or cyclic alkanediyl group, optionally comprising one oxygen atom; R⁶ represents a hydrogen atom or a methyl group;
   wherein the precursor compound is a compound of formula (I) 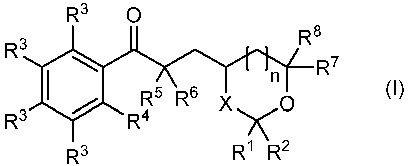 in the form of any one of its stereoisomers or a mixture thereof, and wherein R¹, R², R³, R⁴, R⁵ and R⁶ have the same meaning as defined above; n is 0 or 1; X is an oxygen atom or a NR⁹ group wherein R⁹ is a hydrogen atom or a methyl group; and R⁷ and R⁸ represent, independent of each other, a hydrogen atom or a C₁ to C₆ alkyl group;
by exposing the precursor compound of formula (I) to light and to an environment wherein the compound is hydrolyzed.

Another aspect of the invention is a method for intensifying or prolonging the diffusion effect of the characteristic fragrance of at least one carbonyl compound of formula (II) and of at least one ketone of formula (III) as defined above, on a surface or the air surrounding the perfuming composition, wherein the surface, or the air is treated with at least one compound (I) as defined above, or with a composition or article containing at least one compound (I), under conditions susceptible of allowing the release of at least one ketone or aldehyde formula (II) and of at least one ketone of formula (III) over time.

In a further aspect, the present invention relates to the use of at least one compound of formula (I) as defined above for intensifying or prolonging the diffusion effect, and/or perception of the characteristic fragrance of at least one carbonyl compound formula (II) and/or of at least one phenyl ketone of formula (III) as defined above, on a surface, wherein the surface is treated with at least one compound of formula (I) as defined above, or with a composition or article containing the at least one compound of formula (I), under conditions susceptible of allowing the release of the at least one carbonyl compound formula (II) and/or of at least one phenyl ketone of formula (III) over time.

In a further aspect, the present invention relates to the use of at least one compound of formula (I) as defined above to confer, enhance, improve or modify the odor properties of a perfuming composition, the air surrounding the perfuming composition, a surface, or of a perfumed article, comprising adding to the composition or article or contacting or treating the surface with an effective amount of at least one compound of formula (I) as defined above.

### Examples

The invention is hereafter described in a more detailed manner by way of the following examples, wherein the abbreviations have the usual meaning in the art, temperatures are indicated in degrees centigrade (°C). NMR spectral data were recorded on a Bruker AMX 500 spectrometer in CDCl₃ at 500 MHz for ¹H and at 125.8 MHz for ¹³C if not indicated otherwise, the chemical displacements δ are indicated in ppm with respect to Si(CH₃)₄ as the standard, the coupling constants J are expressed in Hz (br. = broad peak). Reactions were carried out in standard glassware under N₂. Commercially available reagents and solvents were used without further purification if not stated otherwise.

Although specific conformations or configurations are indicated for some of the compounds, this is not meant to limit the use of these compounds to the isomers described. According to the invention, all possible conformation or configuration isomers are expected to have a similar effect.

Typical manners to prepare the invention's compound and to execute the invention's method are reported herein below in the examples.

### Example 1

### Preparation of cyclic acetals or ketals according to formula (I)

### (a) Synthesis of (±)-cis-1-phenyl-3-(2-phenyl-1,3-dioxan-4-yl)propan-1-one (Compound 1)

Toluene (2.5 mL), (±)-cis-4-(hydroxymethyl)-2-phenyl-1,3-dioxane (2.5 g, 12.9 mmol, B. Herradón, S. Valverde, Tetrahedron Asymmetry, 1994, Vol. 5, pages 1479-1500), acetophenone (3.1 g, 25.8 mmol), [Ir(cod)Cl]₂ (0.173 g, 0.26 mmol), triphenylphosphine (0.20 g, 0.77 mmol), and lithium hydroxide monohydrate (0.22 g, 5.2 mmol) were added to a 25 mL round-bottomed flask equipped with reflux condenser and nitrogen bubbler. The dark red solution was heated at 110°C (oil bath) for 23 h. The mixture was diluted with diethyl ether (200 mL) and washed with water (3x 150 mL). The organic phase was dried over Na₂SO₄, filtered and concentrated. The residue was heated (100-120°C) under vacuum (4.7 Pa) to remove excess acetophenone and then subjected to flash chromatography (SiO₂, hexane/ethyl acetate (EtOAc), 100:0 to 80:20) to afford the title compound (0.87 g, 23% yield) as an amber solid.

¹H-NMR: 1.58 (ddt, J=13.3, 2.5, 1.3 Hz, 1H), 1.83-1.92 (m, 1H), 1.96-2.06 (m, 1H), 2.07-2.14 (m, 1H), 3.18 (t, J=7.1 Hz, 2H), 3.90-4.00 (m, 2H), 4.26 (ddd, J=11.5, 5.11, 1.1 Hz, 1H), 5.49 (s, 1H), 7.31-7.38 (m, 3H), 7.41-7.50 (m, 4H), 7.54 (tt, J=7.4, 1.3 Hz, 1H) 7.96-7.99 (m, 2H).

¹³C-NMR: 30.2 (CH₂), 31.4 (CH₂), 33.8 (CH₂), 67.0 (CH₂), 76.1 (CH), 101.1 (CH), 126.0 (CH), 128.0 (CH), 128.2 (CH) 128.6 (CH), 128.7 (CH), 133.0 (CH), 136.9 (C), 138.7 (C), 200.0 (C).

### (b) Synthesis of (±)-3-(2-decyl-1,3-dioxolan-4-yl)-1-phenylpropan-1-one (Compound 2)

*Part 1. Synthesis of (+)-3-(2,2-dimethyl-1,3-dioxolan-4-yl)-1-phenylpropan-1-one* **(Compound 2a**). Toluene (30 mL), (±)-(2,2-dimethyl-1,3-dioxolan-4-yl)methanol (solketal, 20.00 g, 151 mmol), acetophenone (27.3, 227 mmol), [Ir(cod)Cl]₂ (2.0 g, 3.02 mmol), triphenylphosphine (2.37 g, 9.07 mmol), and lithium hydroxide monohydrate (2.55g, 60.7 mmol) was added to a 100 mL round-bottomed flask equipped with reflux condenser and nitrogen bubbler. The dark red solution was heated at 110°C for 21 h. The mixture was diluted with diethyl ether (200 mL) and washed with water (3x 150 mL). The organic phase was dried over Na₂SO₄, filtered and concentrated. Compound 2a (25.1 g, 71% yield) was isolated by Kugelrohr distillation (170°C oven, 3.3 Pa) as a pale-yellow oil.

¹H-NMR: 1.35 (s, 3H), 1.42 (s, 3H), 1.93 (ddt, J=14.0, 8.5, 5.6 Hz, 1H), 2.06 (dddd, 14.0, 10.7, 6.6, 4.1 Hz, 1H), 3.06-3.21 (m, 2H), 3.59 (dd, J=8.0, 7.0 Hz, 1H), 4.09 (dd, J=8.0, 6.1 Hz, 1H), 4.19 (ddt, J=10.7, 6.6, 4.2 Hz, 1H), 7.46 (t, J=7.9 Hz, 2H), 7.56 (t, J=7.5 Hz, 1H), 7.98 (d, J=7.9 Hz, 2H).

¹³C-NMR: 25.6 (CH₃), 27.0 (CH₃), 27.9 (CH₂), 34.7 (CH₂), 69.3 (CH₂), 75.2 (CH), 109.0 (C), 128.1 (CH), 128.6 (CH), 133.1 (CH), 136.8 (C), 199.5 (C).

*Part 2.* A toluene (20 mL) solution of Compound 2a (1.21 g, 5.16 mmol), undecanal (2.64 g, 15.5 mmol, 3 equiv) and para-toluenesulfonic acid monohydrate (pTSA, 0.06 g, 0.32 mmol, 0.06 equiv) was stirred at room temperature (rt) for 3 h. The mixture was diluted with diethyl ether (100 mL) and washed with sat. aqueous Na₂CO₃ and water. The organic phase was dried over Na₂SO₄, filtered, and concentrated. Excess undecanal was removed by Kugelrohr distillation (40°C oven, 6.7 Pa). Flash chromatography (SiO₂, hexane/EtOAc, 100:0 to 93:7) afforded 1.2 g (68% yield) of the title compound as a pale-yellow viscous oil (diastereoisomeric ratio (dr)=67:33).

¹H-NMR (600 MHz): 0.88 (t, J=7.0 Hz, 3H), 1.21-1.35 (m, 14H), 1.35-1.45 (m, 2H), 1.58-1.69 (m, 2H), 1.90-2.01 and 2.07-2.14 (m, 2H), 3.06-3.25 (m, 2H), 3.49-3.54 (m, 0.52H), 3.59 (dd, J=7.7, 6.2 Hz, 0.48H), 3.96 (dd, J=7.7, 6.9 Hz, 0.48H), 4.11-4.20 (m, 1.52H), 4.88 (t, J=4.8 Hz, 0.48H), 4.97 (t, J=4.8 Hz, 0.52H), 7.46 (t, J=7.8 Hz, 2H), 7.56 (t, J=7.4 Hz, 1H), 7.98 (d, J=7.8 Hz, 2H).

¹³C-NMR (151 MHz): 14.1 (CH₃), 22.7 (CH₂), 24.0 (CH₂), 24.1 (CH₂), 27.6 (CH₂), 27.9 (CH₂), 29.3 (CH₂), 29.56 (CH₂), 29.59 (CH₂), 29.6 (CH₂), 31.9 (CH₂), 34.1 (CH₂), 34.2 (CH₂), 34.5 (CH₂), 34.9 (CH₂), 69.5 (CH₂), 70.4 (CH₂), 75.2 (CH), 75.5 (CH), 103.9 (CH), 104.9 (CH), 128.0 (CH), 128.1 (CH), 128.59 (CH), 128.6 (CH), 133.0 (CH), 133.1 (CH), 136.85 (C), 136.87 (C), 199.4 (C), 199.5 (C).

### (c) Synthesis of (±)-1-phenyl-3-(2-(2-phenylpropyl)-1,3-dioxolan-4-yl)propan-1-one (Compound 3)

Following the procedure described for Compound 2, the title compound (1.22 g, 73% yield, colorless liquid) was prepared from Compound 2a (1.21 g, 5.16 mmol) and 3-phenylbutanal (2.3 g, 15.5 mmol) and isolated as a mixture of diastereoisomers.

¹H-NMR: 1.27 and 1.28 (both d, J=7.0 Hz, 3H), 1.81-2.11 (m, 4H), 2.90-3.02 (m, 1H), 3.02-3.19 (m, 2H), 3.40-3.49 (m, 0.25H), 3.56 and 3.57 (both dd, J=7.8, 6.2 Hz, 0.75H), 3.89 and 3.91 (overlapping dd, J=7.8, 6.9 Hz, 0.75H), 4.02-4.19 (m, 1.25H), 4.71 (dd, J=6.4, 4.0 Hz, 0.37H), 4.74 (dd, J=6.0, 4.1 Hz, 0.39H), 4.81 (dd, J=6.3, 4.2 Hz, 0.25H), 7.14-7.22 (m, 3H), 7.24-7.30 (m, 2H), 7.42-7.49 (m, 2H), 7.53-7.59 (m, 1H), 7.93-8.00 (m, 2H).

¹³C-NMR (the two major isomers): 22.8 (CH₃), 22.9 (CH₃), 27.9 (CH₂), 28.1 (CH₂), 34.45 (CH₂), 34.5 (CH₂), 35.8 (CH), 36.0 (CH), 42.2 (CH₂), 42.4 (CH₂), 69.4 (CH₂), 69.5 (CH₂), 75.5 (CH₂), 75.6 (CH₂), 103.48 (CH), 103.54 (CH), 126.0 (CH), 126.1 (CH), 126.93 (CH), 126.96 (CH), 128.04 (CH), 128.06 (CH), 128.38 (CH), 128.39 (CH), 128.57 (CH), 128.59 (CH), 133.1 (CH), 136.84 (C), 136.9 (C), 146.7 (C), 146.8 (C), 199.4 (C), 199.5 (C).

### (d) Synthesis of (±)-1-phenyl-3-(2-(undecan-2-yl)-1,3-dioxolan-4-yl)propan-1-one (Compound 4)

Following the procedure described for Compound 2, the title compound (4.68 g, 38% yield, colorless liquid) was prepared from Compound 2a (8.0 g, 34.1 mmol) and 2-methylundecanal (12.6 g, 68.4 mmol) and isolated as a mixture of diastereoisomers.

¹H-NMR: 0.88 (t, J=7.0 Hz, 3H), 0.90 (d, J=6.8 Hz, 1.1H), 0.93 (d, J=6.8 Hz, 1.9H), 1.09-1.20 (m, 1H), 1.20-1.33 (m, 13H), 1.33-1.43 (m, 1H), 1.43-1.57 (m, 1H), 1.61-1.73 (m, 1H), 1.89-2.00 (m, 1.3H), 2.03-2.13 (m, 0.7H), 3.05-3.24 (m, 2H), 3.49-3.54 (m, 0.34H), 3.55 and 3.56 (overlapping dd, J=7.7, 6.1 Hz, 0.66H), 3.96 (dd, J=7.7, 6.7, 0.66H), 4.10-4.18 (m, 1.34H), 4.72 and 4.73 (overlapping d, J=4.5 Hz, 0.66H), 4.78 and 4.79 (overlapping d, J=4.5 Hz, 0.34H), 7.46 (t, J=7.8 Hz, 2H), 7.56 (t, J=7.5 Hz, 1H), 7.98 (d, J=7.8 Hz, 2H).

¹³C-NMR: 13.6 (CH₃), 13.7 (CH₃), 13.8 (CH₃), 13.82 (CH₃), 14.1 (CH₃), 22.7 (CH₂), 27.0 (CH₂), 27.07 (CH₂), 27.09 (CH₂), 27.1 (CH₂), 27.59 (CH₂), 27.6 (CH₂), 27.77 (CH₂), 27.8 (CH₂), 29.3 (CH₂), 29.6 (CH₂), 29.9 (CH₂), 31.4 (CH₂), 31.5 (CH₂), 31.6 (CH₂), 31.63 (CH₂), 31.9 (CH₂), 34.5 (CH₂), 34.87 (CH₂), 34.9 (CH₂), 36.8 (CH), 36.9 (CH), 37.1 (CH), 37.15 (CH), 69.6 (CH₂), 69.64 (CH₂), 70.5 (CH₂), 75.38 (CH), 75.39 (CH), 75.40 (CH), 75.43 (CH), 107.0 (CH), 107.1 (CH), 107.91 (CH), 107.95 (CH), 128.1 (CH), 128.6 (CH), 133.1 (CH), 136.9 (C), 199.47 (C), 199.49 (C), 199.51 (C).

### (e) Synthesis of (±)-3-(2-(dec-9-en-1-yl)-1,3-dioxolan-4-yl)-1-phenylpropan-1-one (Compound 5)

Following the procedure described for Compound 2, the title compound (3.75 g, 98% yield, colorless liquid) was prepared from Compound 2a (2.5 g, 10.7 mmol) and 10-undecenal (3.6 g, 21.3 mmol) and isolated as a mixture of two diastereoisomers (dr=63:37)

¹H-NMR: 1.24-1.34 (m, 8H), 1.34-1.45 (m, 4H), 1.59-1.69 (m, 2H), 1.89-2.00 (m, 1.35H), 2.06-2.15 (m, 0.65H), 2.02 (q, J=7.3 Hz, 2H), 3.06-3.24 (m, 2H), 3.49-3.54 (m, 0.35H), 3.59 (dd, J=7.7, 6.3 Hz, 0.65H), 3.96 (dd, 7.7, 6.9 Hz, 0.65H), 4.11-4.20 (m, 1.35H), 4.88 (t, J=4.8 Hz, 0.65H), 4.92 (d, J=10.2 Hz, 1H), 4.97 (t, J=4.8 Hz, 0.35H), 4.99 (dq, J=17.0, 1.7 Hz, 1H), 5.80 (ddt, J=17.0, 10.2, 6.7 Hz, 1H), 7.46 (t, J=7.7 Hz, 2H), 7.56 (t, J=7.4 Hz, 1H), 7.98 (d, J=7.7 Hz, 2H).

¹³C-NMR: 24.00 (CH₂), 24.03 (CH₂), 27.6 (CH₂), 27.9 (CH₂), 28.9 (CH₂), 29.1 (CH₂), 29.36 (CH₂), 29.37 (CH₂), 29.5 (CH₂), 29.53 (CH₂), 29.6 (CH₂), 33.8 (CH₂), 34.1 (CH₂), 34.2 (CH₂), 34.5 (CH₂), 34.9 (CH₂), 69.5 (CH₂), 70.4 (CH₂), 75.2 (CH), 75.5 (CH), 103.9 (CH), 104.9 (CH), 114.1 (CH₂), 128.1 (CH), 128.6 (CH), 133.07 (CH), 133.1 (CH), 136.9 (C), 139.2 (CH), 199.4 (C), 199.5 (C).

### (f) Synthesis of (±)-3-(2-(2,4-dimethylcyclohex-3-en-1-yl)-1,3-dioxolan-4-yl)-1-phenylpropan-1-one (Compound 6)

Following the procedure described for Compound 2, the title compound (2.41 g, 72% yield, colorless viscous liquid) was prepared from Compound 2a (2.5 g, 10.7 mmol) and 2,4-dimethylcyclohex-3-ene-1-carbaldehyde (2.95 g, 21.3 mmol) and isolated as a mixture of diastereoisomers.

¹H-NMR: 0.92 (d, J=7.0 Hz, 3H), 1.42-1.54 (m, 1H), 1.63 (s, 3H), 1.67-1.80 (m, 2H), 1.86-2.02 (m, 3H), 2.02-2.15 (m, 1H), 2.30-2.43 (m, 1H), 3.06-3.23 (m, 2H), 3.49-3.55 (m, 0.3H), 3.58 and 3.60 (overlapping dd, J=7.7, 6.3 Hz, 0.7Hz), 3.96 and 3.964 (overlapping dd, J=7.7, 6.7 Hz, 0.7H), 4.10-4.21 (m, 1.3H), 4.74 and 4.742 (overlapping d, J=7.1 Hz, 0.7H), 4.82 and 4.825 (overlapping d, J=7.3 Hz, 0.3H), 5.31-5.37 (overlapping s, 1H), 7.46 (t, J=7.8 Hz, 2H), 7.56 (t, J=7.4 Hz, 1H), 7.98 (d, J=7.8 Hz, 2H).

¹³C-NMR: 15.9 (CH₃), 19.16 (CH₂), 19.22 (CH₂), 23.5 (CH₃), 28.0 (CH₂), 28.2 (CH₂), 30.03 (CH₂), 30.05 (CH₂), 30.1 (CH), 30.2 (CH), 34.47 (CH₂), 34.53 (CH₂), 41.7 (CH), 41.9 (CH), 69.37 (CH₂), 69.40 (CH₂), 75.3 (CH), 75.4 (CH), 106.4 (CH), 106.5 (CH), 127.19 (CH), 127.20 (CH), 128.06 (CH), 128.07 (CH), 128.6 (CH), 132.8 (C), 132.9 (C), 133.06 (CH), 133.08 (CH), 136.9 (C), 199.5 (C), 199.6 (C).

### (g) Synthesis of (±)-3-(2-(2-(4,4-dimethylcyclohex-1-en-1-yl)ethyl)-1,3-dioxolan-4-yl)-1-phenylpropan-1-one (Compound 7)

Following the procedure described for Compound 2, the title compound (1.64 g, 64% yield, amber oil) was prepared from Compound 2a (1.75 g, 7.5 mmol) and 3-(4,4-dimethylcyclohex-1-en-1-yl)propanal (4.2 g, 31.4 mmol) and isolated as a mixture of two diastereoisomers (dr=60:40)

¹H-NMR: 0.88 (s, 6H), 1.35 (t, J=6.5 Hz, 2H), 1.71-1.82 (m, 4H), 1.88-2.00 (m, 3.4H), 2.01-2.15 (m, 2.6), 3.06-3.24 (m, 2H), 3.49-3.55 (m, 0.4H) and 3.59 (dd, J=7.7, 6.4 Hz, 0.6H), 3.96 (dd, J=7.7, 6.9 Hz, 0.6H), 4.11-4.21 (m, 1.4H), 4.89 (t, J=4.8 Hz, 0.6H), 4.98 (t, 4.9 Hz, 0.4H), 5.33 (br. s, 1H), 7.46 (t, J=7.7 Hz, 2H), 7.56 (t, J=7.4 Hz, 1H), 7.98 (d, J=7.7 Hz, 2H).

¹³C-NMR: 26.2 (CH₂), 27.5 (CH₂), 27.9 (CH₂), 28.16 (CH₃), 28.17 (CH₃), 28.2 (CH₃), 28.5 (C), 31.7 (CH₂), 31.8 (CH₂), 32.4 (CH₂), 32.5 (CH₂), 34.5 (CH₂), 34.9 (CH₂), 35.7 (CH₂), 39.3 (CH₂), 69.6 (CH₂), 70.4 (CH₂), 75.2 (CH), 75.6 (CH), 103.7 (CH), 104.6 (CH), 120.0 (CH), 128.0 (CH), 128.1 (CH), 128.6 (CH), 133.08 (CH), 133.1 (CH), 135.4 (C), 136.84 (C), 136.85 (C), 199.4 (C), 199.5 (C).

### (h) Synthesis of (±)-(Z)-1-phenyl-3-(2-(undec-3-en-1-yl)-1,3-dioxolan-4-yl)propan-1-one (Compound 8)

Following the procedure described for Compound 2, the title compound (2.11 g, 79% yield, colorless liquid) was prepared from Compound 2a (1.75 g, 7.5 mmol) and (Z)-4-dodecenal (5.7 g, 31.4 mmol) and isolated as a mixture of two diastereoisomers (dr=76:24)

¹H-NMR (major isomer only): 0.87 (t, J=6.8 Hz, 3H), 1.20-1.43 (m, 10H), 1.59-1.76 (m, 2H), 1.89-2.00 (m, 1H), 2.03 (q, J=6.7 Hz, 2H), 2.06-2.21 (m, 3H), 3.05-3.25 (m, 2H), 3.60 (dd, J=7.7, 6.3 Hz, 1H), 3.96 (dd, J=7.7, 6.7 Hz, 1H), 4.11-4.21 (m, 1H), 4.90 (t, J=4.7 Hz, 1H), 5.32-5.44 (m, 2H), 7.46 (t, J=7.7 Hz, 2H), 7.56 (t, J=7.5 Hz, 1H), 7.98 (d, J=7.7. Hz, 2H).

¹³C-NMR (major isomer only): 14.1 (CH₃), 21.9 (CH₂), 22.7 (CH₂), 27.2 (CH₂), 27.9 (CH₂), 29.2 (CH₂), 29.3 (CH₂), 29.7 (CH₂), 31.9 (CH₂), 34.1 (CH₂), 34.5 (CH₂), 69.6 (CH₂), 75.6 (CH), 104.4 (CH), 128.0 (CH), 128.5 (CH), 128.6 (CH), 130.7 (CH), 133.1 (CH), 136.9 (C), 199.4 (C).

### (i) Synthesis of (±)-3-(2-(2-(4-methylcyclohex-3-en-1yl)propyl)-1,3-dioxolan-4yl)-1-phenylpropan-1-one (Compound 9)

Following the procedure described for Compound 2, the title compound (2.53 g, 69% yield, colorless viscous liquid) was prepared from Compound 2a (2.5 g, 10.7 mmol) and 3-(4-methylcyclohex-3-en-1-yl)butyraldehyde (3.55 g, 21.4 mmol) and isolated as a mixture of diastereoisomers.

¹H-NMR: 0.89-0.95 (overlapping d, 3H), 1.16-1.36 (m, 1H), 1.37-1.57 (m, 2H), 1.62 (s, 3H), 1.60-1.84 (m, 4H), 1.85-2.05 (m, 4H), 2.05-2.16 (m, 1H), 3.06-3.25 (2H), 3.49-3.55 (m, 0.3H), 3.59 and 3.60 (overlapping dd, J=7.7, 6.3 Hz, 0.7H), 3.945 and 3.95 (overlapping dd, J=7.7, 6.8 Hz, 0.7H), 4.10-4.21 (m, 1.3 H), 4.92-496 (overlapping t, 0.7H), 5.02 and 5.03 (overlapping t, J=5.7 and 5.9 Hz, 0.3H), 5.36 (br. s, 1H), 7.46 (t, J=7.8 Hz, 2H), 7.56 (t, J=7.4 Hz, 1H), 7.98 (d, J=7.8 Hz, 2H).

¹³C-NMR: 16.5 (CH₃), 16.6 (CH₃), 23.5 (CH₃), 25.2 (CH₂), 25.3 (CH₂), 28.0 (CH₂), 28.1 (CH₂), 29.08 (CH₂), 29.13 (CH₂), 30.82 (CH₂), 30.83 (CH₂), 33.3 (CH), 33.4 (CH), 34.48 (CH₂), 34.5 (CH₂), 38.37 (CH₂), 38.42 (CH₂), 38.7 (CH), 38.9 (CH), 69.4 (CH₂), 69.5 (CH₂), 75.4 (CH), 75.5 (CH), 104.3 (CH), 104.4 (CH), 120.92 (CH), 120.95 (CH), 128.1 (CH), 128.6 (CH), 133.1 (CH), 133.9 (C), 136.9 (C), 199.48 (C), 199.5 (C).

### (j) Synthesis of (±)-3-(2-(6-methylhept-5-en-2-yl)-1,3-dioxolan-4-yl)-1-phenylpropan-1-one (Compound 10)

Following the procedure described for Compound 2, the title compound (1.84 g, 55% yield, pale-yellow liquid) was prepared from Compound 2a (2.5 g, 10.7 mmol) and 2,6-dimethyl-5-hepten-1-al (6.0 g, 42.8 mmol) and isolated as a mixture of diastereoisomers.

¹H-NMR: 0.92 (d, J=6.9 Hz, 1.8H), 0.94 (d, J=6.9 Hz, 1.2H), 1.13-1.26 (m, 1H), 1.50-1.60 (m, 1H), 1.60 (s, 3H), 1.67 (s, 3H), 1.67-1.76 (m, 1H), 1.89-2.02 (m, 2.4H), 2.02-2.13 (m, 1.6H), 3.06-3.24 (m, 2H), 3.49-3.53 (m, 0.4H), 3.55 (dd, J=7.6, 6.5 Hz, 0.3H), 3.56 (dd, J=7.7, 6.5, 0.3H), 3.96 (t, J=7.3 Hz, 0.6H), 4.10-4.18 (m, 1.4H), 4.73 (d, J=4.2 Hz, 0.3H), 4.74 (d, J=4.2 Hz, 0.3H), 4.79 (d, J=4.2 Hz, 0.2H), 4.80 (d, J=4.2 Hz, 0.2H), 5.10 (br. t, J=7.1 Hz, 1H), 7.46 (t, J=7.7 Hz, 2H), 7.56 (t, J=7.4 Hz, 1H), 7.98 (d, J=7.7 Hz, 2H).

¹³C-NMR: 13.6 (CH₃), 13.7 (CH₃), 13.72 (CH₃), 17.67 (CH₃), 17.7 (CH₃), 25.45 (CH₂), 25.5 (CH₂), 25.7 (CH₃), 27.58 (CH₂), 27.6 (CH₂), 27.7 (CH₂), 27.8 (CH₂), 31.4 (CH₂), 31.5 (CH₂), 31.6 (CH₂), 31.64 (CH₂), 34.6 (CH₂), 34.86 (CH₂), 34.88 (CH₂), 36.3 (CH), 36.4 (CH), 36.61 (CH), 36.63 (CH), 69.63 (CH₂), 69.64 (CH₂), 70.5 (CH₂), 75.4 (CH), 75.5 (CH), 106.95 (CH), 107.0 (CH), 107.9 (CH), 124.54 (CH), 124.55 (CH), 124.57 (CH), 128.1 (CH), 128.6 (CH), 131.4 (C), 131.43 (C), 131.45 (C), 131.46 (C), 133.1 (CH), 136.9 (C), 199.47 (C), 199.49 (C), 199.51 (C).

### (k) Synthesis of (E/Z)-3-(2-(4,8-dimethylnon-3-en-1yl)-1,3-dioxolan-4yl)-1-phenylpropan-1-one (Compound 11)

A solution of Compound 2a (1.00 g, 4.3 mmol), (E/Z)-5,9-dimethyldec-4-enal (2.33 g, 12.8 mmol) and pTSA (0.04 g) in toluene (20 mL) was stirred at rt for 15 h. The reaction mixture was washed with sat. aqueous Na₂CO₃ (15 mL), extracted with EtOAc (15 mL), neutralized with demineralized water (15 mL), dried (Na₂SO₄) and concentrated. Excess (E/Z)-5,9-dimethyldec-4-enal was removed by Kugelrohr distillation (120°C oven, 0.1 mbar). Flash chromatography (SiO₂, heptane/EtOAc 9:1) afforded 1.11 g (73% yield) of the title compound as a colorless oil, isolated as a mixture of diastereoisomers (dr=66:34).

¹H-NMR: 0.86 (overlapping d, J=6.7 Hz, 6H), 1.08-1.18 (m, 2H), 1.30-1.41 (m, 2H); 1.47-1.57 (m, 1H), 1.59, 166 and 1.67 (s, 3H), 1.61-1.73 (m, 2H), 1.89-2.03 (m, 3.3H), 2.06-2.16 (m, 2.7H), 3.06-3.26 (m, 2H), 3.48-3.55 (m, 0.3H), 3.60 (dd, J=7.7, 6.4 Hz, 0.7H), 3.94-3.99 (m, 0.7H), 4.11-4.22 (m, 1.3H); 4.89 (dt, J=4.8, 1.6 Hz, 0.7H); 4.98 (dt, J=4.8, 1.6 Hz, 0.3H), 5.08-5.15 (m, 1H), 7.43-7.51 (m, 2H); 7.54-7.59 (m, 1 H), 7.95-8.01 (m, 2H).

¹³C-NMR: 15.86 and 23.39 (CH₃), 22.44, 22.48, 22.53 and 22.60 (CH₂), 22.63 and 22.65 (CH₃), 25.67, 25.68, 25.71 and 25.73 (CH₂), 27.58 and 27.94 (CH₂), 27.88 and 27.92 (CH), 31.89 and 39.87 (CH₂), 34.24, 34.45, 34.51 and 34.89 (2 CH₂), 38.58, 38.60 and 38.84 (CH₂), 69.58 and 70.40 (CH₂), 75.23, 75.56 and 75.58 (CH), 103.50, 103.52, 104.46 and 104.47 (CH), 123.10, 123.13, 123.85 and 123.90 (CH), 128.05 (CH), 128.60 (CH), 133.09 and 133.11 (CH), 136.13, 136.33, 136.35, 136.84 and 136.85 (C), 199.44, 199.45, 199.49 and 199.51 (C).

### (l) Synthesis of (E/Z)-3-(2-(5-cyclohexyl-4-methylpent-4-en-2-yl)-1,3-dioxolan-4-yl)-1-phenylpropan-1-one (Compound 12)

Following the procedure described for Compound 11, the title compound (1.26 g, 80% yield, colorless viscous oil) was prepared from (E/Z)-5-cyclohexyl-2,4-dimethylpent-4-enal (2.49 g, 12.8 mmol) and isolated, after Kugelrohr distillation (140°C oven, 0.1 mbar) and column chromatography, as a mixture of diastereoisomers (dr ca. 30:30:20:20).

¹H-NMR: 0.79-0.90 (m, 3H), 0.93-1.08 (m, 2H), 1.08-1.33 (m, 2H), 1.50-1.79 (m, 9.5H), 1.81-2.26 (m, 5.5H), 3.06-3.24 (m, 2H), 3.49-3.61 (m, 1H), 3.93-4.01 (m, 0.5H), 4.09-4.20 (m, 1.5H), 4.71-4.84 (m, 1H), 4.95-5.06 (m, 1H), 7.43-7.50 (m, 2H), 7.53-7.60 (m, 1H), 7.93-8.01 (m, 2H).

¹³C-NMR: 12.97, 13.03, 13.07, 13.11, 13.36 and 13.37 (CH₃), 15.87, 15.88 and 23.48 (CH₃), 26.03, 26.07, 26.08, 26.11, 26.14 and 26.16 (3 CH₂), 27.55, 27.56, 27.59, 27.70, 27.74, 27.79 (CH₂), 33.28, 33.41, 33.49, 33.51, 33.54, 33.66, 33.85, 34.56, 34.57, 34.60, 34.84, 34.87, 41.57, 41.62, 41.70, 41.72 (4 CH₂), 34.66, 34.72, 34.79, 34.99, 35.10 and 35.15 (CH), 36.68, 36.72, 36.75 and 37.02 (CH), 69.64, 69.69, 69.73, 69.76, 70.44, 70.54 and 70.58 (CH₂), 75.46, 75.48, 75.50 and 75.56 (CH), 106.61, 106.66, 106.77, 107.46, 107.52, 107.59 and 107.70 (CH), 128.06 (CH), 128.60 (CH), 130.62, 130.64 and 130.68 (C), 133.10, 133.18 and 133.21 (CH), 133.65, 133.67, 133.72 (CH), 136.84, 136.85 and 136.87 (C), 199.48, 199.50 and 199.54 (C).

### (m) Synthesis of (±)-3-(2-methyl-2-phenyl-1,3-dioxolan-4-yl)-1-phenylpropan-1-one (Compound 13)

A toluene (50 mL) solution of Compound 2a (5 g, 21.3 mmol), acetophenone (7.7 g, 64 mmol) and pTSA (0.22 g, 1.3 mmol) was stirred at room temperature for 3 h under vacuum at 12 mbar. The mixture was diluted with diethyl ether (200 mL) and washed with sat. aqueous Na₂CO₃ and water. The organic phase was dried over MgSO₄, filtered, and concentrated. Flash chromatography of the remaining residue (SiO₂, hexane/EtOAc, 100:0 to 95:5) afforded 1.4 g (22% yield) of the title compound as a white solid (dr=67:33).

¹H-NMR: 1.63 (s, 1H), 1.66(s, 2H), 1.74-1.82 (m, 0.33H), 1.89-1.97 (m, 0.33H), 1.97-2.16 (m, 1.34H), 3.03-3.25 (m, 2H), 3.47 (t, J=8.1 Hz, 0.33H), 3.68 (dd, J=6.1, 7.8 Hz, 0.67H), 3.88 (dd, J=6.8, 7.8 Hz, 0.67H), 4.01-4.08 (m, 0.67H), 4.21 (dd, J=6.1, 8.1 Hz, 0.33H), 4.30-4.37 (m, 0.33H), 7.25-7.36 (m, 3H), 7.39-7.60 (m, 5H), 7.89-7.95 (m, 0.66H), 7.96-8.02 (m, 1.34H).

¹³C-NMR: 27.6 (CH₂), 28.2 (CH₂), 28.27 (CH₃), 28.35 (CH₃), 34.5 (CH₂), 34.9 (CH₂), 69.2 (CH₂), 69.7 (CH₂), 75.0 (CH), 76.4 (CH), 109.19 (C), 109.22 (C), 125.0 (CH), 125.2 (CH), 127.74 (CH), 127.75 (CH), 128.05 (CH), 128.07 (CH), 128.12 (CH), 128.17 (CH), 128.57 (CH), 128.6 (CH), 133.1 (CH), 136.8 (C), 136.9 (C), 143.6 (C), 144.5 (C), 199.4 (C), 199.5 (C).

### (n) Synthesis of (±)-3-(2-methyl-2-phenethyl-1,3-dioxolan-4-yl)-1-phenylpropan-1-one (Compound 14)

Following the procedure described for Compound 13, the title compound (2.2 g, 40% yield, colorless oil) was prepared from Compound 2a (4.0 g, mmol) and 4-phenyl-2-butanone (7.6 g, 51.2 mmol) and isolated as a mixture of two diastereoisomers (dr=57:43).

¹H-NMR: 1.36 (s, 1.7H), 1.42(s, 1.3H), 1.90-2.02 (m, 3H), 2.04-2.14 (m, 1H), 2.67-2.77 (m, 2H), 3.05-3.22 (m, 2H), 3.59 (t, J=7.6 Hz, 0.6H), 3.63 (t, J=7.5 Hz, 0.4H), 4.13 (dd, J=6.0, 4.6 Hz, 0.4H), 4.14 (dd, J=6.1, 4.8 Hz, 0.6H), 4.15 (m, 0.4H), 4.22-4.29 (m, 0.6H), 7.14-7.21 (m, 3H), 7.24-7.29 (m, 2H), 7.43-7.50 (m, 2H), 7.54-7.59 (m, 1H), 7.96-8.01 (m, 2H).

¹³C-NMR: 24.2 (CH₃), 25.2 (CH₃), 27.7 (CH₂), 28.0 (CH₂), 30.1 (CH₂), 30.4 (CH₂), 34.7 (CH₂), 34.8 (CH₂), 41.0 (CH₂), 41.7 (CH₂), 69.5 (CH₂), 69.8 (CH₂), 75.1 (CH), 75.8 (CH), 110.1 (C), 110.2 (C), 125.7 (CH), 128.1 (CH), 128.3 (CH), 128.33 (CH), 128.35 (CH), 128.6 (CH), 133.1 (CH), 136.8 (C), 136.84 (C), 142.1 (C), 142.2 (C), 199.4 (C), 199.5 (C).

### (o) Synthesis of (±)-3-(6-pentyl-1,4-dioxaspiro[4.4]nonan-2-yl)-1-phenylpropan-1-one (Compound 15)

Following the procedure described for Compound 13, the title compound (1.4 g, 20% yield, white solid) was prepared from Compound 2a (5.0 g, 21.3 mmol) and 2-pentylcyclopentanone (9.9 g, 64 mmol) and isolated as a mixture of four diastereoisomers.

¹H-NMR: 0.86, 0.87, 0.875 (overlapping t, J=7.0 Hz, 3H), 1.08-1.41 (m, 8H), 1.41-2.12 (m, 9H), 3.05-3.22 (m, 2H), 3.47-3.62 (overlapping dd, 1H), 3.96-4.21 (m, 2H), 7.44-7.49 (m, 2H), 7.54-7.59 (m, 1H), 7.97-8.01 (m, 2H).

¹³C-NMR: 14.11 and 14.12 (CH₃), 20.40, 20.56, 20.75 and 20.86 (CH₂), 22.67 and 22.70 (CH₂), 27.52, 27.63, 27.92, 27.96, 28.00, 28.01, 28.04, 28.20, 28.38, 28.74, 28.98 and 29.03 (3 CH₂), 29.21, 29.25, 29.30 and 29.56 (CH₂), 32.22, 32.24, 32.26 and 32.28 (CH₂), 34.62, 34.70, 34.73 and 34.86 (CH₂), 35.54, 36.02, 36.70 and 37.14 (CH₂), 46.02, 46.23, 46.69 and 46.76 (CH), 69.03, 69.34, 69.39, 69.62 (CH₂), 74.32, 74.69, 75.44 and 75.92 (CH), 118.64, 118.65, 118.83 and 119.05 (C), 128.07 and 128.08 (2 CH), 128.59 (2 CH), 133.09 (CH), 136.8, 136.88 and 136.9 (C), 199.53, 199.55, 199.63 and 199.66 (C).

### (p) Synthesis of (±)-1-phenyl-3-(2-phenyl-1,3-dioxolan-4-yl)propan-1-one (Compound 16)

Toluene (70 mL), the glycerin acetal of benzaldehyde (15.0 g, 83.2 mmol, a mixture of the 5- and 6-membered cyclic acetals), acetophenone (20.0, 166 mmol), [Ir(cod)Cl]₂ (1.12 g, 1.66 mmol), triphenylphosphine (1.31 g, 5.0 mmol), and lithium hydroxide (1.4 g, 58.3 mmol) was added to a round-bottomed flask equipped with reflux condenser and nitrogen bubbler. The dark red solution was heated at 110°C for 22 h. The mixture was diluted with diethyl ether and water and stirred vigorously for 1 h. The phases were separated, and the aqueous phase extracted with diethyl ether (2 x 150 mL). The combined organic phases were dried over MgSO₄, filtered and concentrated. The residue was heated (170°C) under vacuum (2.7 Pa) to remove excess remaining reagents. The residue was subjected to shot-path distillation (bp 130°C, 2.4 Pa) followed by flash chromatography (SiO₂, hexane/EtOAc, 100:0 to 50:50) to afford the title compound (7.2 g, 31% yield) as a colorless oil (dr=50:50).

¹H-NMR (CD₂Cl₂): 1.98-2.09 (m, 1.5H), 2.09-2.19 (m, 0.5H), 3.09-3.28 (m, 2H), 3.67 (dd, J=7.7, 6.4 Hz, 0.5H) 3.76 (dd, J=7.7, 6.4 Hz, 0.5H), 4.13 (dd, J=7.7, 6.8 Hz, 1H), 4.26-4.35 (m, 1.5H), 5.77 (s, 0.5H), 5.89 (s, 0.5H), 7.34-7.39 (m, 3H), 7.42-7.49 (m, 4H), 7.54-7.60 (m, 1H), 7.92-8.00 (m, 2H).

¹³C-NMR: 27.9 (CH₂), 28.3.0 (CH₂), 35.0 (CH₂), 35.2 (CH₂), 70.5 (CH₂), 71.1 (CH₂), 76.3 (CH), 76.8 (CH), 103.4 (CH), 104.4 (CH), 126.9 (CH), 127.1 (CH), 128.3 (CH), 128.64 (CH), 128.65 (CH), 128.96 (CH), 128.97 (CH), 129.4 (CH), 129.6 (CH), 133.4 (CH), 137.33 (C), 137.36 (C), 138.4 (C), 139.0 (C), 199.53 (C), 199.56 (C).

### (q) Synthesis of (±)-3-(2-decyl-1,3-dioxolan-4-yl)-1-(4-methoxyphenyl)propan-1-one (Compound 17)

*Part 1. Synthesis of (*±*)-3-(2,2-dimethyl-1,3-dioxolan-4yl)-1-(4-methoxyphenyl)propan-1-one* (**Compound 17a**). Toluene (25 mL), solketal (5.0 g, 37.8 mmol), para-methoxyacetophenone (8.52 g, 56.7 mmol), [Ir(cod)Cl]₂ (0.51 g, 0.75 mmol), triphenylphosphine (0.59 g, 2.25 mmol), and lithium hydroxide monohydrate (0.64 g, 26.0 mmol) was added to a 100 mL round-bottom flask equipped with reflux condenser and nitrogen bubbler. The dark red solution was heated at 110°C (oil bath) for 20 h. The mixture was diluted with diethyl ether (200 mL) and washed with water (3x 150 mL). The organic phase was dried over Na₂SO₄, filtered and concentrated. The title compound (2.65 g, 26.5% yield) was isolated by Kugelrohr distillation (170-180°C oven, 3.3 Pa) as a colorless liquid.

¹H-NMR: 1.35 (s, 3H), 1.42 (s, 3H), 1.92 (ddt, J=14.0, 8.4, 5.7 Hz, 1H), 2.05 (dddd, 14.0, 10.7, 6.6, 4.2 Hz, 1H), 2.99-3.16 (m, 2H), 3.59 (dd, J=8.0, 6.8 Hz, 1H), 3.87 (s, 3H), 4.08 (dd, J=8.0, 6.1 Hz, 1H), 4.19 (ddt, J=10.7, 6.6, 4.2 Hz, 1H), 6.93 (d, J=8.8 Hz, 2H), 7.96 (d, J=8.8 Hz, 2H).

¹³C-NMR: 25.7 (CH₃), 27.0 (CH₃), 28.1 (CH₂), 34.3 (CH₂), 55.5 (CH₂), 69.4 (CH₂), 75.3 (CH₂), 108.9 (C), 113.7 (CH), 130.0 (C), 130.3 (CH), 163.5 (C), 198.0 (C).

*Part 2.* A toluene (50 mL) solution of Compound 17a (1.5 g, 5.67 mmol), undecanal (2.9 g, 17.0 mmol) and pTSA (0.29 g, 1.7 mmol) was stirred at rt for 1 day. The mixture was diluted with diethyl ether and washed with sat. aqueous Na₂CO₃ and water. The organic phase was dried over Na₂SO₄, filtered, and concentrated. Flash chromatography (SiO₂, hexane/EtOAc, 100:0 to 88:12) afforded 1.3 g (61% yield) of the title compound (dr=59:41).

¹H-NMR: 0.87 (t, J=7.0 Hz, 3H), 1.20-1.35 (m, 14H), 1.35-1.45 (m, 2H), 1.59-1.69 (m, 2H), 1.88-1.99 (m, 1.4H), 2.05-2.13 (m, 0.6H), 3.00-3.19 (m, 2H), 3.48-3.53 (m, 0.4H), 3.58 (dd, J=7.8, 6.3 Hz, 0.6H), 3.87 (s, 3H), 3.95 (dd, J=7.7, 6.8 Hz, 0.6H), 4.10-4.20 (m, 1.4H), 4.88 (t, J=4.8 Hz, 0.6H), 4.97 (t, J=4.9 Hz, 0.4H), 6.91-6.93 (m, 1H), 6.93-6.95 (m, 1H), 7.94-7.96 (m, 1H), 7.96-7.99 (m, 1H).

¹³C-NMR: 14.1 (CH₃), 22.7 (CH₂), 24.0 (CH₂), 24.1 (CH₂), 27.8 (CH₂), 28.1 (CH₂), 29.3 (CH₂), 29.57 (CH₂), 29.59 (CH₂), 29.61 (CH₂), 29.62 (CH₂), 31.9 (CH₂), 34.13 (CH₂), 34.14 (CH₂), 34.19 (CH₂), 34.5 (CH₂), 55.5 (CH₃), 69.5 (CH₂), 70.4 (CH₂), 75.3 (CH), 75.7 (CH), 103.9 (CH), 104.9 (CH), 113.7 (CH), 129.96 (C), 129.98 (C), 130.3 (CH), 163.47 (C), 163.48 (C), 198.0 (C), 198.1 (C).

### (r) Synthesis of (±)-3-(2-decyl-1,3-dioxolan-4yl)-1-(p-tolyl)propan-1-one (Compound 18)

*Part 1. Synthesis of (*±*)-3-(2,2-dimethyl-1, 3-dioxolan-4yl)-1-(p-tolyl)propan-1-one* (**Compound 18a**). Toluene (25 mL), solketal (5.0 g, 37.8 mmol), *para-*methylacetophenone (7.6 g, 56.7 mmol), [Ir(cod)Cl]₂ (0.51 g, 0.75 mmol), triphenylphosphine (0.60 g, 2.27 mmol), and lithium hydroxide monohydrate (0.63 g, 15 mmol) was added to a 100 mL round-bottom flask equipped with reflux condenser and nitrogen bubbler. The dark red solution was heated at 110°C (oil bath) for 20 h. The mixture was filtered through a bed of Celite^{®}, diluted with ethyl acetate (200 mL) and washed with water (3x 150 mL). Flash chromatography (SiO₂, hexane/EtOAc, 95:5) afforded 4.72 g (50% yield) of the title compound as a pale-amber solid.

¹H-NMR: 1.36 (s, 3H), 1.42 (s, 3H), 1.92 (ddt, J=14.0, 8.5, 5.5 Hz, 1H), 2.05 (dddd, 14.0, 10.6, 6.5, 4.2 Hz, 1H), 2.41 (s, 3H), 3.03-3.20 (m, 2H), 3.59 (dd, J=8.0, 7.0 Hz, 1H), 4.09 (dd, J=8.0, 6.1 Hz, 1H), 4.19 (ddt, J=10.8, 6.5, 4.3 Hz, 1H), 7.26 (d, J=8.1 Hz, 2H), 7.88 (d, J=8.1 Hz, 2H).

¹³C-NMR: 21.7 (CH₃), 25.6 (CH₃), 27.0 (CH₃), 27.9 (CH₂), 34.6 (CH₂), 69.3 (CH₂), 75.3 (CH), 108.9 (C), 128.2 (CH), 129.2 (CH), 134.3 (C), 143.9 (C), 199.1 (C).

*Part 2.* A toluene (20 mL) solution of Compound 18a (1.5 g, 6.0 mmol), undecanal (3.1 g, 18.1 mmol) and pTSA (0.32 g, 1.84 mmol) was stirred at rt for 2 days. The mixture was diluted with diethyl ether and washed with sat. aqueous Na₂CO₃ and water. The organic phase was dried over Na₂SO₄, filtered, and concentrated. Flash chromatography (SiO₂, hexane/EtOAc, 100:0 to 90:10) afforded 1.5 g (69% yield) of the title compound as a white solid (dr=61:39).

¹H-NMR: 0.88 (t, J=6.9 Hz, 3H), 1.20-1.35 (m, 14H), 1.35-1.44 (m, 2H), 1.58-1.69 (m, 2H), 1.88-2.00 (m, 1.4H), 2.05-2.14 (m, 0.6H), 2.41 (s, 3H), 3.02-3.22 (m, 2H), 3.48-3.54 (m, 0.4H), 3.58 (dd, J=7.7, 6.2 Hz, 0.6H), 3.96 (dd, J=7.7, 6.8 Hz, 0.6H), 4.10-4.20 (m, 1.4H), 4.88 (t, J=4.8 Hz, 0.6H), 4.96 (t, J=4.8 Hz, 0.4H), 7.26 (d, J=8.1 Hz, 2H), 7.88 (d, J=8.1 Hz, 2H).

¹³C-NMR: 14.1 (CH₃), 21.6 (CH₂), 22.7 (CH₂), 24.0 (CH₂), 24.1 (CH₂), 27.7 (CH₂), 28.0 (CH₂), 29.3 (CH₂), 29.56 (CH₂), 29.59 (CH₂), 29.61 (CH₂), 29.62 (CH₂), 31.9 (CH₂), 34.1 (CH₂), 34.2 (CH₂), 34.4 (CH₂), 34.8 (CH₂), 69.5 (CH₂), 70.4 (CH₂), 75.3 (CH), 75.6 (CH), 103.9 (CH), 104.9 (CH), 128.18 (CH), 128.2 (CH), 129.3 (CH), 134.38 (C), 134.4 (C), 143.85 (C), 143.9 (C), 199.1 (C), 199.2 (C).

### (s) Synthesis of (±)-cis-3-(2-(4-methoxyphenyl)-1,3-dioxan-4-yl)-1-phenylpropan-1-one (Compound 19)

Toluene (10 mL), (±)-cis-(2-(4-methoxyphenyl)-1,3-dioxan-4-yl)methanol (6.0 g, 26.8 mmol, B. Herradón, S. Valverde, Tetrahedron Asymmetry, 1994, Vol. 5, pages 1479-1500), acetophenone (4.82 g, 40.1 mmol), [Ir(cod)Cl]₂ (0.37 g, 0.54 mmol), triphenylphosphine (0.42 g, 1.60 mmol), and lithium hydroxide monohydrate (0.45 g, 10.7 mmol) were added to a round-bottomed flask equipped with reflux condenser and nitrogen bubbler. The dark red solution was heated at 110°C (oil bath) for 24 h. The mixture was diluted with diethyl ether and washed with water. The organic phase was dried over MgSO₄, filtered and concentrated. The residue was subjected to flash chromatography (SiO₂, hexane/EtOAc, 100:0 to 70:30) to afford the title compound (3.1 g, 35% yield) as a white solid.

¹H-NMR (CD₂Cl₂): 1.55 (ddt, J=13.2, 2.5, 1.4 Hz, 1H), 1.75-1.85 (m, 1H), 1.91-2.00 (m, 1H), 2.00-2.08 (m, 1H), 3.08-3.20 (m, 2H), 3.77 (s, 3H), 3.86-3.95 (m, 2H), 4.20 (ddd, J=11.5, 5.1, 1.2 Hz, 1H), 5.42 (s, 1H), 6.83-6.88 (m, 2H), 7.33-7.38 (m, 2H), 7.42-7.47 (m, 2H), 7.55 (tt, J=7.4, 1.3 Hz, 1H) 7.93-7.98 (m, 2H).

¹³C-NMR (CD₂Cl₂): 30.6 (CH₂), 30.8 (CH₂), 34.3 (CH₂), 55.6 (CH₃), 67.2 (CH₂), 76.6 (CH), 101.4 (CH), 113.7 (CH), 127.7 (CH), 128.3 (CH) 128.9 (CH), 132.0 (C), 133.3 (CH), 137.5 (C), 160.2 (C), 200.0 (C).

### (t) Synthesis of (±)-cis-3-(2-(4-ethylphenyl)-1,3-dioxan-4-yl)-1-phenylpropan-1-one (Compound 20)

*Part 1. Synthesis of (*±*)-cis-(2-(4-ethylphenyl)-1,3-dioxan-4-yl)methanol* (**Compound 20a**). A dichloromethane (120 ml) solution of 1,2,4-butane triol (14.6 g, 137 mmol), the dimethyl acetal of para-ethylbenzaldehyde (16.5, 92 mmol) and Amberlyst^{®}15 resin was stirred at rt for 7 h. The solution was filtered and concentrated. The remaining residue was dissolved in diethyl ether and washed with sat. NaHCO₃. The organic phase was dried over Na₂SO₄, filtered and concentrated. Kugelrohr distillation afforded 16.5 g (81% yield) of the title compound as a colorless oil containing about 8% of the dioxolane byproduct.

¹H-NMR (CDCl₃, 400 MHz): 1.21 (t, J=7.6 Hz, 3H), 1.36-1.42 (m, 1H), 1.80-1.93 (m, 1H), 2.43 (br s, 1H), 2.63 (q, J=7.6 Hz, 2H), 3.56-3.67 (m, 2H), 3.88-3.99 (m, 2H), 4.26 (ddd, J=11.4, 5.2, 1.2 Hz, 1H), 5.49 (s, 1H), 7.16-7.22 (m, 2H), 7.37-7.42 (m, 2H).

¹³C-NMR (CDCl₃, 100.6 MHz): 15.6 (CH₃), 26.8 (CH₂), 28.7 (CH₂), 65.6 (CH₂), 66.6 (CH₂), 77.5 (CH), 101.4 (CH), 126.1 (CH), 127.8 (CH), 135.8 (C), 145.1 (C).

*Part 2.* Toluene (15 mL), Compound 20a (4.0 g, 18 mmol), acetophenone (3.24 g, 27 mmol), [Ir(cod)Cl]₂ (0.25 g, 0.36 mmol), triphenylphosphine (0.28 g, 1.1 mmol), and lithium hydroxide monohydrate (0.30 g, 7.2 mmol) were added to a round-bottomed flask equipped with reflux condenser and nitrogen bubbler. The dark red solution was heated at 110°C for 24 h. The mixture was diluted with diethyl ether and washed with water. The organic phase was dried over MgSO₄, filtered and concentrated. The residue was heated (120°C) under vacuum (2.7 Pa) to remove excess acetophenone and then subjected to flash chromatography (SiO₂, hexane/EtOAc, 100:0 to 80:20) to afford the title compound (2.7 g, 46% yield) as a white solid.

¹H-NMR: 1.21 (t, J=7.6 Hz, 3H), 1.56 (ddt, J=13.3, 2.4, 1.4 Hz, 1H), 1.81-1.91 (m, 1H), 1.95-2.04 (m, 1H), 2.05-2.14 (m, 1H), 2.63 (q, J=7.6 Hz, 2H), 3.17 (t, J=7.2 Hz, 2H), 3.89-3.98 (m, 2H), 4.25 (ddd, J=11.5, 5.0, 1.1 Hz, 1H), 5.46 (s, 1H), 7.18 (d, J=8.1 Hz, 2H), 7.39 (d, J=8.1 Hz, 2H), 7.42-7.46 (m, 2H), 7.54 (tt, J=7.4, 1.2 Hz, 1H) 7.95-7.99 (m, 2H).

¹³C-NMR: 15.6 (CH₃), 28.7 (CH₂), 30.2 (CH₂), 31.4 (CH₂), 33.8 (CH₂), 66.9 (CH₂), 76.1 (CH), 101.3 (CH), 126.0 (CH), 127.7 (CH), 128.1 (CH) 128.5 (CH), 133.0 (CH), 136.2 (C), 137.0 (C), 144.8 (C), 200.0 (C).

### (u) Synthesis of (±)-cis-3-(2-decyl-1,3-dioxan-4-yl)-1-phenylpropan-1-one (Compound 21)

A toluene (200 mL) solution of Compound 1 (8 g, 26.9 mmol), undecanal (18.4 g, 108 mmol) and pTSA (1.4 g, 8.2 mmol) was stirred at room temperature for one day. The mixture was diluted with diethyl ether (200 mL) and washed with sat. aqueous Na₂CO₃ and water. The organic phase was dried over MgSO₄, filtered, and concentrated. Excess undecanal was removed by Kugelrohr distillation (95°C oven, 2.7 Pa). Flash chromatography of the remaining residue (SiO₂, hexane/EtOAc, 100:0 to 92:8) afforded 3.9 g (40% yield) of the title compound as a white solid.

¹H-NMR: 0.87 (t, J=7.0 Hz, 3H), 1.17-1.32 (m, 14H), 1.32-1.41 (m, 2H), 1.47 (ddt, J=13.2, 2.5, 1.3 Hz, 1H), 1.56-1.62 (m, 2H), 1.66-1.76 (m, 1H), 1.86-1.94 (m, 1H), 1.98-2.06 (m, 1H), 3.13 (t, J=7.0 Hz, 2H), 3.64-3.76 (m, 2H), 4.10 (dd, J=11.5, 5.0 1H), 4.47 (t, J=5.3 Hz, 1H), 7.44-7.48 (m, 2H), 7.56 (tt, J=7.4, 1.2 Hz, 1H) 7.96-8.00 (m, 2H).

¹³C-NMR: 14.1 (CH₃), 22.7 (CH₂), 24.1 (CH₂), 29.3 (CH₂), 29.5 (CH₂), 29.56 (CH₂), 29.59 (CH₂), 29.62 (CH₂), 30.2 (CH₂), 31.5 (CH₂), 31.9 (CH₂), 33.7 (CH₂), 35.1 (CH₂), 66.5 (CH₂), 75.5 (CH), 102.2 (CH), 128.1 (CH), 128.6 (CH), 133.0 (CH), 137.0 (C), 200.1 (C).

### (v) Synthesis of (±)-cis-3-(2-decyl-1,3-dioxan-4-yl)-1-phenylpropan-1-one (Compound 22)

A toluene (150 mL) solution of Compound 1 (6 g, 20.2 mmol), 2-methylundecanal (11.2 g, 60.7 mmol) and para-toluenesulfonic acid monohydrate (1.1 g, 6.2 mmol) was stirred at room temperature for five days. The mixture was diluted with diethyl ether (200 mL) and washed with sat. aqueous Na₂CO₃ and water. The organic phase was dried over Na₂SO₄, filtered, and concentrated. The residue was subjected to flash chromatography (SiO₂, hexane/EtOAc, 100:0 to 95:5) followed by Kugelrohr distillation (230°C oven, 2.4 Pa) to afforded 3.87 g (51% yield) of the title compound as a colorless oil. (dr=56:44).

¹H-NMR: 0.88 (t, J=6.9 Hz, 3H), 0.90 (overlapping d, J=6.8 Hz, 3H), 1.04-1.16 (m, 1H), 1.16-1.37 (m, 14H), 1.45 (br d, J=13.2 Hz, 1H), 1.45-1.55 (m, 1H), 1.58-1.74 (m, 2H), 1.84-1.94 (m, 1H), 1.97-2.07 (m, 1H), 3.13 (overlapping t, J=7.2 Hz, 2H), 3.63-3.74 (m, 2H), 4.11 (dd, J=11.5, 5.1 1H), 4.26 (d, J=5.0 Hz, 1H), 7.44-7.48 (m, 2H), 7.56 (tt, J=7.5, 1.2 Hz, 1H) 7.96-8.00 (m, 2H).

¹³C-NMR: 14.11 (CH₃), 14.12 (CH₃), 14.2 (CH₃), 22.7 (CH₂), 27.0 (CH₂), 27.02 (CH₂), 29.4 (CH₂), 29.65 (CH₂), 29.66 (CH₂), 29.68 (CH₂), 29.70 (CH₂), 29.9 (CH₂), 30.0 (CH₂), 30.29 (CH₂), 30.32 (CH₂), 31.52 (CH₂), 31.56 (CH₂), 31.64 (CH₂), 31.93 (CH₂), 33.76 (CH₂), 33.78 (CH₂), 37.83 (CH), 37.86 (CH), 66.6 (CH₂), 75.43 (CH), 75.45 (CH), 105.0 (CH), 105.1 (CH), 128.1 (CH), 128.6 (CH), 133.0 (CH), 137.0 (C), 200.14 (C), 200.17 (C).

### (w) Synthesis of (±)-2-methyl-3-(2-phenethyl-1,3-dioxolan-4-yl)-1-phenylpropan-1-one (Compound 23)

A mixture of allyl alcohol (25.8 g, 444 mmol), the dimethyl acetal of propiophenone (20 g, 111 mmol, T. Rossolini, B. Ferko, D. Dixon, Organic Letters, 2019, Vol. 21, pages 6668-6673) and citric acid (0.43 g, 2.22 mmol) was added to an autoclave reactor and the reactor was placed in an oil bath heated at 165°C. The mixture was stirred and heated for 1 day. The excess allyl alcohol was removed by distillation under vacuum (2.7 Pa). The remaining residue was dissolved in diethyl ether and washed with sat. aqueous Na₂CO₃ and water. The organic phase was dried over MgSO₄, filtered, and concentrated. Kugelrohr distillation (120°C oven, 3.3 Pa) of the crude product afforded 2-methyl-1-phenyl-pent-4-en-1-one (14.3 g) as a colorless liquid.

The ketone (13.8 g, 79.2 mmol) was dissolved in methanol (100 ml) and the solution cooled in an ice bath. NaBH₄ (6.0 g, 158.4 mmol) was added portionwise. The mixture was removed from the cold bath and stirred at rt for 1 h. Water (30 ml) was added to the mixture and the methanol removed with a rotary evaporator. The remaining aqueous residue was extracted with diethyl ether. The combined organic phases were dried over MgSO₄, filtered, and concentrated to yield 2-methyl-1-phenylpent-4-en-1-ol (12.97 g) as a colorless oil that was used without further purification in the next step.

2-Methyl-1-phenylpent-4-en-1-ol was added dropwise to a dichloromethane (100 ml) solution of acetyl chloride (7.0 g), pyridine (7.0 g) and 4-dimethylaminopyridine (0.27 g) cooled in an ice bath. The solution was stirred at rt overnight. The reaction mixture was washed with 1 N HCl (25 ml) and the separated dichloromethane layer concentrated under vacuum. The residue was dissolved in diethyl ether and washed successively with 1 N HCl, water, sat. aqueous NaHCO₃ and sat. aqueous ammonium chloride. The organic phase was dried over MgSO₄, filtered, and concentrated. Kugelrohr distillation (100°C oven, 3.3 Pa) afforded 2-methyl-1-phenylpent-4-en-1-yl acetate (12.5 g) as a colorless oil.

The obtained acetate ester (11.5 g) was dissolved in dichloromethane (150 ml) and the solution cooled in an ice bath. *meta*-Chloroperbenzoic acid (16.8) was added, and the resulting slurry stirred at rt for 1 day. After adding a 20% aqueous sodium thiosulfate solution (25 ml), the mixture was stirred for 30 min and then the dichloromethane was removed with a rotary evaporator. Diethyl ether (500 ml) was added, and the solution successively washed with sat. aqueous Na₂CO₃ (3 X 30 ml) and water (2 x 25 ml). The ether phase was dried over MgSO₄, filtered, and concentrated to yield 11.7 g of the epoxide, 2-methyl-3-(oxiran-2-yl)-1-phenylpropyl acetate, as a pale-yellow, slightly viscose oil.

The epoxide (11.2 g) and anhydrous FeCl₃ (0.4 g) were dissolved in reagent grade acetone (210 ml) and the solution stirred for 3 h at rt. The acetone then was removed using a rotary evaporator and the remaining brown residue diluted with diethyl ether. This solution was washed with sat. aqueous Na₂CO₃ and water. The organic phase was dried over MgSO₄, filtered, and concentrated to yield 12.7 g of the corresponding acetonide, 3-(2,2-dimethyl-1,3-dioxolan-4-yl)-2-methyl-1-phenylpropyl acetate, as a yellow-tinted oil.

Next, a transacetalization reaction converted the acetonide into the corresponding cyclic 3-phenypropanal acetal. A toluene (100 ml) solution of the acetonide (12.42 g), 3-phenylpropanal (22.8 g) and pTSA (0.4 g) was stirred at rt for 3 h. The mixture was diluted with diethyl ether and washed with sat. aqueous Na₂CO₃ and water. The organic phase was dried over Na₂SO₄, filtered, and concentrated. Excess 3-phenylpropanal was removed by Kugelrohr distillation (120-140°C oven, 6.7 Pa). to yield the 3-phenylpropanal acetal, 2-methyl-3-(2-phenethyl-1,3-dioxolan-4-yl)-1-phenylpropyl acetate (15.2 g), as a pale-yellow oil.

The acetate group was removed by treating the above acetal (15 g) with methanol (500 ml) and K₂CO₃ (0.57 g). The solution was stirred at rt for one day. The methanol then was removed using a rotary evaporator. The remaining residue was diluted with diethyl ether and this solution was washed with brine. The organic phase was dried over MgSO₄, filtered, and concentrated to afford 2-methyl-3-(2-phenethyl-1,3-dioxolan-4-yl)-1-phenylpropan-1-ol (11.1 g) as a pale-amber, viscous oil.

Pyridinium chlorochromate (11.1 g, 50.5 mmol) was added to a stirring dichloromethane (125 ml) solution of the above alcohol (11 g) at rt. After stirring for 2 h, the reaction mixture was filtered through a pad of silica covered with a layer of Celite^{®}. The filtrate was concentrated, and the remaining residue subjected to flash chromatography (SiO₂, hexane/EtOAc, 100:0 to 80:20) yielding 10.3 g (36% overall yield from the dimethyl acetal of propriophenone) of the title compound as a colorless oil, isolated as a mixture of diastereoisomers (dr ca. 45:35:10:10).

¹H-NMR (CD₂Cl₂): 1.20-1.24 (overlapping d, J=7.0 Hz, 3H), 1.49-1.73 (m, 1H), 1.79-1.90 (m, 1H), 1.90-1.98 (m, 1H), 2.00-2.21 (m, 1H), 2.60-2.75 (m, 2H), 3.40-3.46 (m, 0.2H), 3.48-3.54 (m, 0.8H), 3.68-3.82 (m, 1H), 3.86-4.04 (m, 1.2H), 4.06-4.26 (m, 0.8H), 4.81 (t, J=4.7 Hz, 0.35H), 4.90 (t, J=4.7 Hz, 0.45H), 4.93 (t, J=4.8 Hz, 0.1H), 4.95 (t, J=4.8 Hz, 0.1H), 7.13-7.21 (m, 3H), 7.22-7.29 (m, 2H), 7.42-7.51 (m, 2H), 7.53-7.59 (m, 1H), 7.95-8.01 (m, 2H).

¹³C-NMR (CD₂Cl₂): 17.0, 17.3, 19.1 and 19.2 (CH₃), 30.45, 30.46 and 30.50 (CH₂), 36.0, 36.18 and 36.22 (CH₂), 37.36, 37.38, 37.71 and 37.96 (CH₂), 37.79, 37.82, 38.05 and 38.09 (CH), 70.29, 70.32, 70.90 and 71.04 (CH₂), 74.48, 74.70, 74.77 and 75.22 (CH), 103.27, 103.48, 104.32 and 104.39 (CH), 126.13, 126.15, and 126.16 (CH), 128.66, 128.67, 128.69, 128.72, 128.74, 128.99 and 129.02 (8 CH), 133.21, 133.24 and 133.34 (CH), 136.67, 136.77, 137.14 and 137.17 (C), 142.24 and 142.26 (C), 203.70, 203.77, 204.08 and 204.18 (C).

### (x) Synthesis of (±)-2,2-dimethyl-3-(2-phenethyl-1,3-dioxolan-4-yl)-1-phenylpropan-1-one (Compound 24)

Following the multistep procedure described for Compound 23, the title compound was prepared starting from the dimethyl acetal of isobutyrophenone and allyl alcohol. Flash chromatography (SiO₂, hexane/EtOAc, 100:0 to 80:20) afforded it as a colorless viscous oil, isolated as a mixture of diastereoisomers (dr=80:20).

¹H-NMR (CD₂Cl₂, major isomer only): 1.35 (s, 3H), 1.37 (s, 3H), 1.76-1.85 (m, 2H), 1.92 (dd, J=3.8, 14.4 Hz, 1H), 2.25 (dd, J=8.4, 14.4 Hz, 1H), 2.59 (t, J=8.2 Hz, 2H), 3.43 (t, J=7.2 Hz, 1H), 3.91 (dd, J=6.7, 7.5 Hz, 1H), 4.06 (m, 1H), 4.82 (t, J=4.70 Hz, 1H), 7.09-7.17 (m, 3H), 7.21-7.27 (m, 2H), 7.37-7.42 (m, 2H), 7.42-7.47 (m, 1H), 7.63-7.71 (m, 2H).

¹³C-NMR (CD₂Cl₂): 26.2 (CH₃), 26.3 (CH₃), 27.2 (CH₃), 27.5 (CH₃), 30.43 (CH₂), 30.45 (CH₂), 36.0 (CH₂), 36.1 (CH₂), 44.5 (CH₂), 44.6 (CH₂), 46.9 (C), 47.0 (C), 70.8 (CH₂), 71.4 (CH₂), 73.6 (CH), 74.4 (CH), 103.3 (CH), 104.4 (CH), 126.1 (CH), 128.1 (CH), 128.3 (CH), 128.4 (CH), 128.63 (CH), 128.65 (CH), 128.70 (CH), 128.72 (CH), 131.0 (CH), 131.1 (CH), 139.5 (C), 139.6 (C), 142.2 (C), 208.5 (C), 208.7 (C).

### (y) Synthesis of (±)-3-(2-phenethyl-1,3-dioxolan-4-yl)-1-(5,6,7,8-tetrahydronaphthalen-2-yl)propan-1-one (Compound 25)

Following the multistep procedure described for Compound 23, the title compound was prepared starting from the dimethyl acetal of 1-(5,6,7,8-tetrahydronaphthalen-2-yl)ethan-1-one (Florantone^{®}, origin: Takasago Corporation, Tokyo, Japan) and allyl alcohol. Flash chromatography (SiO₂, hexane/EtOAc, 100:0 to 80:20) afforded it as a white solid, isolated as a mixture of diastereoisomers (dr=65:35).

¹H-NMR (CD₂Cl₂): 1.75-1.83 (m, 4H), 1.86-1.98 (m, 3.35H), 2.00-2.08 (m, 0.65H), 2.68-2.76 (m, 2H), 2.77-2.84 (m, 4H), 2.98-3.16 (m, 2H), 3.47-3.53 (m, 0.35H), 3.58 (dd, J=7.8, 6.3 Hz, 0.65H), 3.94 (dd, J=7.7, 6.8 Hz, 0.65H), 4.10-4.19 (m, 1.35H), 4.91 (t, J=4.6 Hz, 0.65H), 5.00 (t, J=4.8 Hz, 0.35H), 7.11-7.21 (m, 4H), 7.22-7.28 (m, 2H), 7.63-7.68 (m, 2H).

¹³C-NMR (CD₂Cl₂): 23.3 (CH₂), 23.4 (CH₂), 28.1 (CH₂), 28.5 (CH₂), 29.8 (CH₂), 30.0 (CH₂), 30.4 (CH₂), 30.5 (CH₂), 34.8 (CH₂), 35.0 (CH₂), 36.1 (CH₂), 36.3 (CH₂), 70.0 (CH₂), 70.8 (CH₂), 75.8 (CH), 76.2 (CH), 103.5 (CH), 104.3 (CH), 125.3 (CH), 126.1 (CH), 128.68 (CH), 128.69 (CH), 128.7 (CH), 129.2 (CH), 129.6 (CH), 134.9 (C), 137.9 (C), 142.3 (C), 143.5 (C), 199.49 (C), 199.52 (C).

### (z) Synthesis of (±)-3-(5-methyl-2-phenethyl-1,3-dioxolan-4-yl)-1-phenylpropan-1-one (Compound 26)

Following the multistep procedure described for Compound 23, the title compound was prepared starting from the dimethyl acetal of acetophenone and 3-buten-2-ol. Flash chromatography (SiO₂, hexane/EtOAc, 100:0 to 80:20) afforded it as a colorless oil, isolated as a single diastereoisomer.

¹H-NMR (CD₂Cl₂): 1.22 (d, J=6.4 Hz, 3H), 1.76-1.85 (m, 1H), 1.85-1.91 (m, 1H), 1.91-1.97 (m, 2H), 2.73 (t, J=8.2 Hz, 2H), 3.07 (ddd, J=6.6, 8.6, 15.2 Hz, 1H), 3.21 (ddd, J=5.3, 8.6, 14.0 Hz, 1H), 4.01 (ddd, J=3.3, 6.5, 9.7 Hz, 1H), 4.17 (pentet, J=6.5 Hz, 1H), 4.90 (t, J=4.6 Hz, 1H), 7.13-7.22 (m, 3H), 7.23-7.28 (m, 2H), 7.44-7.50 (m, 2H), 7.54-7.59 (m, 1H), 7.95-8.00 (m, 2H).

¹³C-NMR (CD₂Cl₂): 15.5 (CH₃), 25.1 (CH₂), 30.5 (CH₂), 35.6 (CH₂), 36.6 (CH₂), 74.9 (CH), 78.1 (CH), 103.0 (CH), 126.1 (CH), 128.3 (CH), 128.67 (CH), 128.74 (CH), 128.9 (CH), 133.3 (CH), 137.55 (C), 142.4 (C), 199.9 (C).

### (aa) Synthesis of (±)-3-(5,5-dimethyl-2-phenethyl-1,3-dioxolan-4-yl)-1-phenylpropan-1-one (Compound 27)

Following the multistep procedure described for Compound 23, the title compound was prepared starting from the dimethyl acetal of acetophenone and 2-methyl-3-buten-2-ol. Flash chromatography (SiO₂, hexane/EtOAc, 100:0 to 90:10) afforded it as a colorless oil (dr=80:20)

¹H-NMR (CD₂Cl₂, 600 MHz, major isomer): 1.17 (s, 3H), 1.28 (s, 3H), 1.77-1.96 (m, 4H), 2.72 (t, J=8.2 Hz, 2H), 3.10 (ddd, J=6.6, 8.8, 15.3 Hz, 1H), 3.25 (ddd, J=5.1, 8.8, 13.9 Hz, 1H), 3.63 (dd, J=2.7, 10.7 Hz, 1H), 4.96 (t, J=4.7 Hz, 1H), 7.13-7.18 (m, 1H), 7.18-7.22 (m, 2H), 7.23-7.28 (m, 2H), 7.45-7.50 (m, 2H), 7.56-7.59 (m, 1H), 7.96-8.00 (m, 2H).

¹³C-NMR (CD₂Cl₂, 150 MHz, major isomer): 23.7 (CH₃), 24.7 (CH₃), 25.6 (CH₂), 30.5 (CH₂), 36.2 (CH₂), 37.0 (CH₂), 79.9 (C), 84.6 (CH), 101.7 (CH), 126.1 (CH), 128.3 (CH), 128.67 (CH), 128.74 (CH), 129.0 (CH), 133.4 (CH), 137.4 (C), 142.4 (C), 199.7 (C).

### Example 2

### Kinetics of the light-induced degradation of compounds according to formula (I)

Compounds according to formula (I) (ca. 50 mg) and DMSO (serving as internal standard, ca. 5 mg) were weighed into a volumetric flask and filled up to 5 mL with CD₃CN. The solution was poured into a home-made Pyrex^{®} glass cell and thermostatted at 25°C. The sample was then irradiated with a xenon lamp at 7.0 mW cm⁻² (ca. 90000 lux). Aliquots of the sample (750 µL) were pipetted off before irradiation (first data point) and, after switching on the lamp, every 30 min for 150 min (data points 2 to 6). The samples were analyzed by quantitative ¹H-NMR spectroscopy. The degradation of Compounds 1, 2, 13 and 16 according to formula (I) was followed by integration of specific protons of the starting compound (e.g. the acetal proton in case of R² = H) with respect to the DMSO signal. The formation of the aldehyde, ketone and the phenyl ketone derivative was also followed with respect to the DMSO signal. The data in Figures 1 and 2 demonstrates a rapid response of the compounds according to formula (I) after exposure to light source. The photolabile cyclic acetals or ketals according to the present invention were almost completely degraded after irradiation with the xenon lamp for 2 h while forming the corresponding phenyl ketone derivative of formula (III) together with an aldehyde of formula R¹CHO or a ketone of formula (R¹)(R²)C=O (according to formula (II)).

### Example 3

### Dynamic headspace analysis of the release of a perfuming ingredient from the invention's compounds of formula (I) incorporated into a consumer product (fabric softener)

A fabric softener base with the following final composition has been prepared:

| | |
|---|---|
| Stepantex^{®} VL90 A (origin: Stepan) | 16.5% by weight |
| Calcium chloride (10% aq. solution) | 0.6% by weight |
| Water | 82.9% by weight |

In a beaker, a solution of the photosensitive cyclic acetal or ketal derivative of formula (I) described in Example 1 (0.078 mmol) in acetone (0.6 mL) was added to the fabric softener (5.4 g). After homogenization, a part of the sample (1.8 g) was dispersed in demineralized cold tap water (600 mL). One cotton sheet (EMPA cotton test cloth Nr. 221, origin: Eidgenössische Materialprüfanstalt), pre-washed with an unperfumed detergent powder and cut to ca. 12 x 12 cm sheets, ca. 3.2 g) was added and agitated manually for 3 min, left standing for 2 min, then wrung out by hand, and weighed to obtain a constant quantity of residual water (ca. 7.0 g). A reference samples consisting of an equimolar amount of the corresponding aldehyde or ketone to be released (0.078 mmol) in acetone (0.6 mL) was added to the fabric softener (5.4 g) and analyzed the same way. The cotton sheets (one with the photosensitive cyclic acetal or ketal derivative and one with the corresponding fragrance to be released) were line-dried for 24 h in the dark. The cotton sheets were then analyzed. For the measurements, the sheets with the photosensitive cyclic acetal or ketal derivative were put into a headspace sampling cell (ca. 160 mL inner volume) and irradiated with a xenon lamp (Solarbox 1500 from CO.FO.ME.GRA Srl. at about 7-8 mW/cm², ca. 90000 lux), whereas the sheet with the free fragrance was put into the headspace sampling cell exposed to natural indoor daylight. The headspace sampling cells were thermostatted at 25°C and exposed to a constant air flow of ca. 200 mL/min. The air was filtered through active charcoal and aspirated through a saturated solution of NaCl (to ensure a constant humidity of the air of ca. 75%). The system was equilibrated during 15 min while adsorbing the volatiles on a waste poly(2,6-diphenyl-p-phenylene oxide (Tenax^{®} TA, 100 mg) cartridge. Then, six times consecutively, the volatiles were adsorbed for 10 min on a clean Tenax^{®} cartridge and for 20 min on a waste Tenax^{®} cartridge. Altogether 6 data points were collected during 175 min. The waste cartridges were discarded; the other cartridges were desorbed on a Perkin Elmer TurboMatrix ATD desorber coupled to an Agilent Technologies 7890A gas chromatograph equipped with a HP-1 capillary column (30 m, i.d. 0.32 mm, film 0.25 µm) and a FID detector. The volatiles were eluted with helium (1 mL/min) using a temperature gradient starting at 80°C and going to 260°C at 15°C/min. Headspace concentrations (in ng/L air) were obtained by external standard calibrations using five different concentrations of the fragrance to be released in ethanol. Each calibration solution (0.1 µL) was injected onto a clean Tenax^{®} cartridge, which was desorbed and analyzed under the same conditions. The results obtained for the release of the different fragrances after 175 min are summarized in Table 1. All values are average values of at least two measurements.

**Table 1: Headspace concentrations of active aldehydes or ketones released from invention's compounds of formula (I) upon exposure to a xenon lamp as compared to the corresponding reference sample. Values correspond to the headspace concentrations measured after sampling for 175 min.**

| | | | |
|---|---|---|---|
| Acetophenone [ng/L] released from Compound 1 | Benzaldehyde [ng/L] released from Compound 1 | Benzaldehyde [ng/L] measured for reference | Factor of increase for Benzaldehyde |
| 683.7 | 328.7 | 24.5 | ×13 |
| Acetophenone [ng/L] released from Compound 2 | Undecanal [ng/L] released from Compound 2 | Undecanal [ng/L] measured for reference | Factor of increase for Undecanal |
| 1054.7 | 478.9 | 20.0 | ×24 |
| Acetophenone [ng/L] released from Compound 3 | 3-Phenylbutanal [ng/L] released from Compound 3 | 3-Phenylbutanal [ng/L] measured for reference | Factor of increase for 3-Phenylbutanal |
| 1414.3 | 308.8 | 36.6 | ×8 |
| Acetophenone [ng/L] released from Compound 4 | 2-Methylundecanal [ng/L] released from Compound 4 | 2-Methylundecanal [ng/L] measured for reference | Factor of increase for 2-Methylundecanal |
| 981.5 | 1059.2 | 23.5 | ×45 |
| Acetophenone [ng/L] released from Compound 5 | 10-Undecenal [ng/L] released from Compound 5 | 10-Undecenal [ng/L] measured for reference | Factor of increase for 10-Undecenal |
| 310.7 | 881.4 | 30.7 | ×29 |
| Acetophenone [ng/L] released from Compound 6 | 2,4-Dimethyl-3-cyclohexene-1-carbaldehyde [ng/L] released from Compound 6 | 2,4-Dimethyl-3-cyclohexene-1-carbaldehyde [ng/L] measured for reference | Factor of increase for 2,4-Dimethyl-3-cyclohexene-1-carbaldehyde |
| 808.6 | 365.3 | 4.4 | ×83 |
| Acetophenone [ng/L] released from Compound 7 | 3-(4,4-Dimethylcyclohexyl)-propanal [ng/L] released from Compound 7 | 3-(4,4-Dimethylcyclohexyl)-propanal [ng/L] measured for reference | Factor of increase for 3-(4,4-Dimethylcyclohe xyl)propanal |
| 809.6 | 646.7 | 66.4 | ×10 |
| Acetophenone [ng/L] released from Compound 8 | 4-Dodecenal [ng/L] released from Compound 8 | 4-Dodecenal [ng/L] measured for reference | Factor of increase for 4-Dodecenal |
| 807.6 | 288.9 | 24.4 | ×12 |
| Acetophenone [ng/L] released from Compound 9 | 3-(4-Methylcyclohex-3-en-1-yl)butanal [ng/L] released from Compound 9 | 3-(4-Methylcyclohex-3-en-1-yl)butanal [ng/L] measured for reference | Factor of increase for 3-(4-Methylcyclohex-3-en-1-yl)butanal |
| 742.0 | 225.3 | 2.2 | ×102 |
| Acetophenone [ng/L] released from Compound 11 | (E/Z)-5,9-Dimethyldec-4-enal [ng/L] released from Compound 11 | (E/Z)-5,9-Dimethyldec-4-enal [ng/L] measured for reference | Factor of increase for (E/Z)-5,9-dimethyldec-4-enal |
| 303.0 | 10.2 | 2.1 | ×5 |
| Acetophenone [ng/L] released from Compound 12 | (E/Z)-5-Cyclohexyl-2,4-dimethylpent-4-enal [ng/L] released from Compound 12 | (E/Z)-5-Cyclohexyl-2,4-dimethylpent-4-enal [ng/L] measured for reference | Factor of increase for (E/Z)-5-cyclohexyl-2,4-dimethylpent-4-enal |
| 326.3 | 4.1 | 1.2 | ×3 |
| Acetophenone [ng/L] released from Compound 13 | 2 Equiv. of acetophenone are released from Compound 13 | Acetophenone [ng/L] measured for reference | Factor of increase for acetophenone |
| 531.9 | - | 22.0 | ×24 |
| Acetophenone [ng/L] released from Compound 14 | 4-Phenylbutan-2-one [ng/L] released from Compound 14 | 4-Phenylbutan-2-one [ng/L] measured for reference | Factor of increase for 4-phenylbutan-2-one |
| 376.4 | 36.7 | 2.1 | ×17 |
| Acetophenone [ng/L] released from Compound 15 | 2-Pentylcyclopentanone [ng/L] released from Compound 15 | 2-Pentylcyclopentanone [ng/L] measured for reference | Factor of increase for 2-pentylcyclopentanone |
| 253.4 | 134.1 | 2.8 | ×48 |
| Acetanisole [ng/L] released from Compound 17 | Undecanal [ng/L] released from Compound 17 | Undecanal [ng/L] measured for reference | Factor of increase for undecanal |
| 1.4 | 51.0 | 7.0 | ×7 |
| 1-(4-Methylphenyl)ethan-1-one [ng/L] released from Compound 18 | Undecanal [ng/L] released from Compound 18 | Undecanal [ng/L] measured for reference | Factor of increase for undecanal |
| 180.0 | 55.2 | 6.7 | ×8 |
| Acetophenone [ng/L] released from Compound 19 | 4-Methoxybenzaldehyde [ng/L] released from Compound 19 | 4-Methoxybenzaldehyde [ng/L] measured for reference | Factor of increase for 4-methoxybenzaldehyde |
| 257.9 | 11.9 | 4.9 | ×2 |
| Acetophenone [ng/L] released from Compound 20 | 4-Ethylbenzaldehyde [ng/L] released from Compound 20 | 4-Ethylbenzaldehyde [ng/L] measured for reference | Factor of increase for 4-ethylbenzaldehyde |
| 468.8 | 491.9 | 17.1 | ×29 |
| Acetophenone [ng/L] released from Compound 21 | Undecanal [ng/L] released from Compound 21 | Undecanal [ng/L] measured for reference | Factor of increase for undecanal |
| 253.2 | 42.8 | 4.9 | ×9 |
| Acetophenone [ng/L] released from Compound 22 | 2-Methylundecanal [ng/L] released from Compound 22 | 2-Methylundecanal [ng/L] measured for reference | Factor of increase for 2-Methylundecanal |
| 413.1 | 143.2 | 3.2 | ×45 |
| Propiophenone [ng/L] released from Compound 23 | 3-Phenylpropanal [ng/L] released from Compound 23 | 3-Phenylpropanal [ng/L] measured from reference | Factor of increase for 3-phenylpropanal |
| 288.2 | 86.2 | 13.9 | ×6 |
| 2-Methyl-1-phenylpropan -1-one [ng/L] released from Compound 24 | 3-Phenylpropanal [ng/L] released from Compound 24 | 3-Phenylpropanal [ng/L] measured from reference | Factor of increase for 3-phenylpropanal |
| 26.9 | 66.1 | 13.9 | ×5 |
| 1-(5,6,7,8-Tetrahydro-naphthalen-2-yl)ethan-1-one [ng/L] released from Compound 25 | 3-Phenylpropanal [ng/L] released from Compound 25 | 3-Phenylpropanal [ng/L] measured from reference | Factor of increase for 3-phenylpropanal |
| 0.3 | 75.7 | 13.9 | ×5 |
| Acetophenone [ng/L] released from Compound 26 | 3-Phenylpropanal [ng/L] released from Compound 26 | 3-Phenylpropanal [ng/L] measured from reference | Factor of increase for 3-phenylpropanal |
| 483.2 | 91.2 | 13.9 | ×7 |
| Acetophenone [ng/L] released from Compound 27 | 3-Phenylpropanal [ng/L] released from Compound 27 | 3-Phenylpropanal [ng/L] measured from reference | Factor of increase for 3-phenylpropanal |
| 354.5 | 15.5 | 13.9 | ×1 |

The data clearly show a considerable improvement in long-lastingness for the light-induced fragrance release from the invention's compound of formula (I) with respect to the free reference fragrance. The data in Table 1 focus on the release of active carbonyl compounds of formula (II) resulting from the second step of the light-induced two-step mechanism outlined before. Generally, a very large increase in headspace concentrations was observed for the release of acetophenone (compound of formula (III), which is released from the invention's compound of formula (I) in the first step of the light-induced two-step sequence) with respect to the corresponding free acetophenone (reference).

### Example 4

### Dynamic headspace analysis of the release of a perfuming ingredient from the invention's compounds of formula (I) incorporated into a consumer product (all-purpose cleaner)

An all-purpose cleaner (APC) formulation with the following final composition has been prepared:

| | |
|---|---|
| Neodol^{®} 91-8 (origin: Shell Chemicals) | 5.0 % by weight |
| Marlon^{®} A 375 (origin: Hüls AG) | 4.0 % by weight |
| Sodium cumolsulfonate | 2.0 % by weight |
| Kathon^{®} CG (origin: Rohm and Haas) | 0.2 % by weight |
| Water | 88.8 % by weight |

In a flask, one of the invention's compounds of formula (I) (0.0369 mmol) was dissolved in ethanol (0.1 mL). Then the APC formulation (3.0 g) was added, and the sample shaken gently. An aliquot of these samples (1 mL) was pipetted off and diluted with demineralized tap water (9 mL). A film of this solution (0.75 mL) was pipetted onto a porous ceramic plate (ca. 5 × 10 cm) and left standing at room temperature in the dark. Similarly, a reference sample consisting of an unmodified aldehyde or ketone (0.0369 mmol) instead of one of the invention's compounds of formula (I) in ethanol (0.1 mL) was prepared and processed the same way.

After one day, each of the ceramic plates was placed inside a headspace sampling cell (ca. 625 mL) and exposed to a constant air flow of ca. 200 mL/min. The air was filtered through active charcoal and aspirated through a saturated aqueous solution of NaCl (to ensure a constant humidity of the air of ca. 75%). The headspace sampling cells were then exposed to a UVA lamp (centered at 370 nm) at a light energy of ca. 2.2 mW/cm². The headspace system was equilibrated for 15 min by adsorbing the volatiles onto a waste Tenax^{®} cartridge. Then the volatiles were alternately adsorbed for 10 min onto a clean Tenax^{®} cartridge and for 20 min onto a waste Tenax^{®} cartridge (6×). The waste cartridges were discarded; the clean cartridges were desorbed and analyzed as described in Example 3. All measurements were performed at least twice. The average headspace concentrations of aldehydes and/or ketones released from the compounds of formula (I) as prepared in Example 1 or from the reference sample after 115 min of sampling above the porous ceramic plates are listed in Table 2. Table 2 also indicates the factors of increase of the aldehyde or ketone released from the invention's compounds of formula (I) with respect to the reference sample.

**Table 2: Headspace concentrations of active aldehydes or ketones released from invention's compounds of formula (I) upon exposure to a UVA lamp as compared to the corresponding reference sample. Values correspond to the headspace concentrations measured after sampling for 115 min.**

| | | |
|---|---|---|
| 2-Methylundecanal [ng/L] released from Compound 4 | 2-Methylundecanal [ng/L] measured for reference | Factor of increase for 2-methylundecanal |
| 6.9 | 0.3 | ×23 |
| Acetophenone [ng/L] released from Compound 4 | Acetophenone [ng/L] measured for reference | Factor of increase for acetophenone |
| 200.9 | 3.0 | ×67 |
| 4-Phenylbutan-2-one [ng/L] released from Compound 14 | 4-Phenylbutan-2-one [ng/L] measured for reference | Factor of increase for 4-phenyl-butan-2-one |
| 28.9 | 0.3 | ×96 |
| Acetophenone [ng/L] released from Compound 14 | Acetophenone [ng/L] measured for reference | Factor of increase for acetophenone |
| 579.9 | 3.0 | ×193 |
| Undecanal [ng/L] released from Compound 18 | Undecanal [ng/L] measured for reference | Factor of increase for undecanal |
| 21.0 | 13.6 | ×1.5 |
| 1-(4-Methyl-phenyl)ethan-1-one [ng/L] released from Compound 18 | 1-(4-Methyl-phenyl)ethan-1-one [ng/L] measured for reference | Factor of increase for 1-(4-methylphenyl)ethan-1-one |
| 197.4 | 0.6 | ×330 |
| 2-Methylundecanal [ng/L] released from Compound 22 | 2-Methylundecanal [ng/L] measured for reference | Factor of increase for 2-methylundecanal |
| 74.4 | 0.3 | ×248 |
| Acetophenone [ng/L] released from Compound 22 | Acetophenone [ng/L] measured for reference | Factor of increase for acetophenone |
| 448.8 | 3.0 | ×150 |

The data clearly show a considerable improvement in long-lastingness for the light-induced fragrance release from the invention's compound of formula (I) with respect to the free reference fragrance.

### Example 5

### Preparation of a perfume oil

A non-limiting example of a typical perfume oil is prepared by admixing the following perfuming co-ingredients:

| **Ingredients** | **weight%** |
|---|---|
| Ethyl 2-methylbutanoate | 0.16 |
| Hexyl acetate | 0.37 |
| Limonene | 1.67 |
| 2,6-Dimethyl-7-octen-2-ol | 0.94 |
| 2-Phenylethanol | 2.15 |
| Linalool | 0.73 |
| (2RS,4SR/4RS)-4-Methyl-2-(2-methyl-1-propen-1-yl)tetrahydro-2H-pyran | 0.30 |
| Ethyl 2-methyl-1,3-dioxolane-2-acetate | 0.32 |
| Benzyl acetate | 2.46 |
| Allyl heptanoate | 0.38 |
| alpha-Terpineol | 0.88 |
| 3,7-Dimethyl-6-octen-1-ol | 0.55 |
| 4-Methoxybenzaldehyde | 1.00 |
| (E)-4-Methyl-3-decen-5-ol | 0.37 |
| [cis/trans-4-(2-Propanyl)cyclohexyl]methanol | 0.47 |
| 1-Methoxy-4-[(1E)-1-propen-1-yl]benzene | 0.15 |
| (1RS,2RS/2SR)-2-(2-Methyl-2-propanyl)cyclohexyl acetate | 1.95 |
| 1,1-Dimethyl-2-phenylethyl acetate | 0.95 |
| Tricyclo[5.2.1.0^{2,}~]dec-3/4-en-8-yl acetate | 3.34 |
| Allyl 3-cyclohexylpropanoate | 0.26 |
| 3-(4-Isopropylphenyl)-2-methylpropanal (3E)-3-Methyl-4-(2,6,6-trimethyl-2-cyclohexen-1-yl)-3-buten-2-one and | 8.18 |
| (1E)-1-(2,6,6-trimethyl-2-cyclohexen-1-yl)-1-penten-3-one | 1.13 |
| 2-Phenoxyethyl 2-methylpropanoate | 5.38 |
| Tricyclo[5.2.1.0(2,6)]dec-3/4-en-8-yl propanoate | 2.32 |
| 5-Heptyldihydro-2(3H)-furanone | 2.30 |
| 2/3-Methylbutyl salicylate | 1.42 |
| (3Z)-3-Hexen-1-yl salicylate | 0.31 |
| 1-(2,3,8,8-Tetramethyl-1,3,4,5,6,7-hexahydronaphthalen-2-yl)ethanone | 16.03 |
| Hexyl 2-hydroxybenzoate | 5.04 |
| (2E)-2-Benzylideneoctanal | 21.22 |
| (-)-(3aR,5aS,9aS,9bR)-3a,6,6,9a-Tetramethyldodecahydronaphtho[2,1-b]furan | 0.27 |
| Habanolide^{®} | 4.78 |
| Exaltolide^{®} | 3.82 |
| Benzyl 2-hydroxybenzoate | 3.01 |
| Dipropylene glycol | 5.39 |
| | Total: 100 |

### Example 6

### Preparation of a liquid detergent formulation comprising an invention's compound of formula (I)

A typical unperfumed liquid detergent formulation is listed in Table 3. A perfumed liquid detergent is prepared by adding, under gentle shaking, the perfume oil of Example 5 (0.3 to 0.8% by weight relative to the total weight of the liquid detergent) and at least one of the invention's compounds of formula (I) (0.05 to 0.5% by weight relative to the total weight of the liquid detergent) into the unperfumed liquid detergent formulation of Table 3.

**Table 3: Composition of a typical unperfumed liquid detergent formulation**

| **Ingredients** | **Amount [wt%]** |
|---|---|
| Sodium C₁₄₋₁₇ alkyl sec. sulfonate ⁽¹⁾ | 7.0 |
| Fatty acids, C₁₂₋₁₈ and C₁₈-unsaturated ⁽²⁾ | 7.5 |
| C_{12/14} fatty alcohol polyglycol ether with 7 mol EO ⁽³⁾ | 17.0 |
| Triethanolamine | 7.5 |
| Propylene glycol | 11.0 |
| Citric acid | 6.5 |
| Potassium hydroxyde | 9.5 |
| Properase^{®} L ⁽⁴⁾ | 0.2 |
| Puradax^{®} EG L ⁽⁴⁾ | 0.2 |
| Purastar^{®} ST L ⁽⁴⁾ | 0.2 |
| Acrylates/Steareth-20 methacrylate structuring crosspolymer ⁽⁵⁾ | 6.0 |
| Deionized water | 27.4 |

| | |
|---|---|
| ⁽¹⁾ Hostapur^{®} SAS 60; origin: Clariant ⁽²⁾ Edenor^{®} K 12-18; origin: Cognis ⁽³⁾ Genapol^{®} LA 070; origin: Clariant ⁽⁴⁾ Origin: Genencor International ⁽⁵⁾ Aculyn^{®} 88; origin: Dow Chemicals | |

### Example 7

### Preparation of a solid detergent comprising an invention's compound of formula (I)

The chassis of a model powder detergent base comprises sodium sulfate, sodium carbonate, sodium dodecylbenzensulfonate, sodium silicate, zeolite, C₁₂₋₁₅ pareth-7, bentonite, perborate, TAED, citric acid, sodium acrylic acid/MA co-polymer, sodium carbonate peroxide, tetrasodium etidronate, sodium chloride, sodium bicarbonate, cellulose gum, disodium anilinomorpholinotriazinylaminostilbenesulfonate, phenylpropyl dimethicone, enzyme, dye.

A typical unperfumed model powder detergent base is composed as listed in Table 4. A perfumed solid detergent is prepared by adding under gentle shaking the perfume oil of Example 5 (0.3 to 0.6% by weight, relative to the total weight of the solid detergent) and at least one of the invention's compounds of formula (I) (0.15% by weight, relative to the total weight of the solid detergent).

**Table 4: Composition of a typical unperfumed powder detergent**

| **Ingredients** | **Amount [wt%]** |
|---|---|
| Anionic surfactant | 5-25 % |
| Non-ionic surfactant | 2-15% |
| Builder | 20-50% |
| Percarborate | 5-20% |
| TAED | 1-8% |
| Polymer | 3-10% |
| Optical brightener | 0.1-0.5% |
| Enzyme, Dye | < 2% |

### Example 8

### Preparation of a bleach-free solid detergent comprising an invention's compound of formula (I)

Typical bleach-free powder detergent formulations are composed of sodium sulfate, sodium carbonate, sodium dodecylbenzensulfonate, sodium silicate, zeolite, C₁₂₋₁₅ pareth-7, bentonite, citric acid, sodium acrylic acid/MA co-polymer, sodium carbonate peroxide, tetrasodium etidronate, sodium chloride, sodium bicarbonate, cellulose gum, disodium anilinomorpholinotriazinylaminostilbenesulfonate, phenylpropyl dimethicone, enzyme, dye.

A typical unperfumed model powder detergent base is composed as listed in Table 5. A perfumed bleach-free solid detergent is prepared by adding under gentle shaking the perfume oil of Example 5 (0.3 to 0.6% by weight, relative to the total weight of the bleach-free solid detergent) and at least one of the invention's compounds of formula (I) (0.15% by weight, relative to the total weight of the bleach-free solid detergent).

**Table 5: Composition of a typical unperfumed bleach-free powder detergent**

| **Ingredients** | **Amount [wt%]** |
|---|---|
| Anionic surfactant | 5-20 % |
| Non-ionic surfactant | 3-12% |
| Builder | 20-65% |
| Polymer | 3-10% |
| Optical brightener | 0.1-0.5% |
| Enzyme, Dye | < 1% |

### Example 9

### Preparation of a hand dishwash formulation comprising an invention's compound of formula (I)

A typical hand dishwash formulation is listed in Table 6. Water, sodium hydroxide and diethanolamide are mixed. Then linear alkylbenzene sulfonic acid is added. After neutralization, the remaining ingredients are added. The pH is checked (7-8) and adjusted if necessary. A perfumed hand dishwash is prepared by adding under gentle shaking the perfume oil of Example 5 (0.4 to 0.8% by weight, relative to the total weight of the hand dishwash formulation) and at least one of the invention's compounds of formula (I) (0.02 to 0.5% by weight relative to the total weight of the unperfumed formulation) into the unperfumed hand dishwash formulation of Table 6.

**Table 6: Composition of a typical unperfumed hand dishwash formulation**

| **Ingredients** | **Amount [wt%]** |
|---|---|
| Linear alkylbenzene sulfonic acid ⁽¹⁾ | 20.0 |
| Diethanolamine ⁽²⁾ | 3.5 |
| Sodium hydroxide (50%) ⁽³⁾ | 3.4 |
| Secondary alcohol ethoxolate ⁽⁴⁾ | 2.5 |
| Sodium xylene sulfonate | 6.3 |
| Deionized water | 64.3 |

| | |
|---|---|
| ⁽¹⁾ Biosoft^{®} S-118; origin: Stepan ⁽²⁾ Ninol^{®} 40-CO; origin: Stepan ⁽³⁾ Stepanate^{®} SXS; origin: Stepan ⁽⁴⁾ Tergitol^{®} 15-S-9; origin: Dow Chemicals | |

### Example 10

### Preparation of a transparent isotropic shampoo formulation comprising an invention's compound of formula (I)

A typical unperfumed transparent isotropic shampoo formulation is listed in Table 7. The unperfumed shampoo formulation is prepared by dispersing Polyquaternium-10 in water. The remaining ingredients of Phase A are mixed separately by addition of one after the other while mixing well after each adjunction. This pre-mix is added to the Polyquaternium-10 dispersion and mixed for another 5 min. Then, the premixed Phase B and the premixed Phase C are added (Monomuls^{®} 90L-12 is heated to melt in Texapon^{®} NSO IS) while agitating. Phase D and Phase E are added while agitating. The pH is adjusted with a citric acid solution to 5.5-6.0 to give the unperfumed shampoo formulation listed in Table 7. The perfumed shampoo formulation is obtained by adding, under gentle shaking, the perfume oil of Example 5 (0.1 to 0.8% by weight relative to the total weight of the unperfumed shampoo formulation) and at least one of the compounds of formula (I) (0.05 to 0.5% by weight relative to the total weight of the unperfumed shampoo formulation) into the unperfumed shampoo formulation listed in Table 7.

**Table 7: Composition of a typical unperfumed transparent isotropic shampoo formulation**

| **Phase** | **Ingredients** | **Amount [wt%]** |
|---|---|---|
| A | Deionized water | 44.4 |
| | Polyquaternium-10 ⁽¹⁾ | 0.3 |
| | Glycerin 85% ⁽²⁾ | 1.0 |
| | DMDM Hydantoin ⁽³⁾ | 0.2 |
| B | Sodium laureth sulfate ⁽⁴⁾ | 28.0 |
| | Cocamidopropyl betaine ⁽⁵⁾ | 3.2 |
| | Disodium cocoamphodiacetate ⁽⁶⁾ | 4.0 |
| | Ethoxy (20) stearyl alcohol ⁽⁷⁾ | 1.0 |
| C | Sodium laureth sulfate ⁽⁴⁾ | 3.0 |
| | Glyceryl laureate ⁽⁸⁾ | 0.2 |
| D | Deionized water | 1.0 |
| | Sodium methylparaben ⁽⁹⁾ | 0.1 |
| E | Sodium chloride (10% aqueous solution) | 15.0 |
| | Citric acid (10% aqueous solution to pH 5.5-6.0) | q.s. |

| | | |
|---|---|---|
| ⁽¹⁾ Ucare^{®} Polymer JR-400; origin: Noveon ⁽²⁾ Origin: Brenntag Schweizerhall AG ⁽³⁾ Glydant^{®}; origin: Lonza ⁽⁴⁾ Texapon^{®} NSO IS; origin: Cognis ⁽⁵⁾ Tego^{®} Betain F 50; origin: Evonik ⁽⁶⁾ Amphotensid GB 2009; origin: Zschimmer & Schwarz ⁽⁷⁾ Brij^{®} S20; origin: Croda ⁽⁸⁾ Monomuls^{®} 90 L-12; origin: Gruenau GmbH ⁽⁹⁾ Nipagin Monosodium; origin: NIPA | | |

### Example 11

### Preparation of a pearly shampoo formulation comprising an invention's compound of formula (I)

A typical unperfumed pearly shampoo formulation is listed in Table 8. The unperfumed shampoo formulation is prepared by dispersing Tetrasodium EDTA, Guar hydroxypropyltrimonium chloride and Polyquaternium-10 in water. NaOH (10% aqueous solution, Phase B) is added once Phase A is homogeneous. Then, the premixed Phase C is added, and the mixture heated to 75°C. Phase D ingredients are added and mixed until the mixture is homogeneous. The mixture is cooled. At 45°C, Phase E ingredients are added while mixing. The final viscosity is adjusted with NaCl (25% aqueous solution) and a pH of 5.5-6.0 is adjusted with NaOH (10% aqueous solution). A perfumed pearly shampoo formulation is obtained by adding, under gentle shaking, the perfume oil of Example 5 (0.1 to 0.8% by weight relative to the total weight of the unperfumed shampoo formulation) and at least one of the compounds of formula (I) (0.05 to 0.5% by weight relative to the total weight of the unperfumed shampoo formulation) into the unperfumed pearly shampoo formulation listed in Table 8.

**Table 8: Composition of a typical unperfumed pearly shampoo formulation**

| **Phase** | **Ingredients** | **Amount [wt%]** |
|---|---|---|
| A | Deionized water | 45.97 |
| | Tetrasodium EDTA ⁽¹⁾ | 0.05 |
| | Guar hydroxypropyl-trimonium chloride ⁽²⁾ | 0.05 |
| | Polyquaternium-10 ⁽³⁾ | 0.075 |
| B | NaOH (10% aqueous solution) | 0.30 |
| C | Ammonium lauryl sulfate ⁽⁴⁾ | 34.00 |
| | Ammonium laureth sulfate ⁽⁵⁾ | 9.25 |
| | Cocamidopropyl betaine ⁽⁶⁾ | 2.00 |
| | Dimethicone (&) C₁₂₋₁₃ pareth-4 (&) C₁₂₋₁₃ pareth-23 (&) salicylic acid ⁽⁷⁾ | 2.50 |
| D | Cetyl alcohol ⁽⁸⁾ | 1.20 |
| | Cocamide MEA ⁽⁹⁾ | 1.50 |
| | Glycol distearate ⁽¹⁰⁾ | 2.00 |
| E | Methylchloroisothiazolinone & methylisothiazolinone ⁽¹¹⁾ | 0.10 |
| | D-Panthenol 75% ⁽¹²⁾ | 0.10 |
| | Deionized water | 0.30 |
| F | Sodium chloride (25% aqueous solution) | 0.60 |

| | | |
|---|---|---|
| ⁽¹⁾ EDETA^{®} B Powder; origin: BASF ⁽²⁾ Jaguar^{®} C14 S; origin: Rhodia ⁽³⁾ Ucare^{®} Polymer JR-400; origin: Noveon ⁽⁴⁾ Sulfetal^{®} LA B-E; origin: Zschimmer & Schwarz ⁽⁵⁾ Zetesol^{®} LA; origin: Zschimmer & Schwarz ⁽⁶⁾ Tego^{®} Betain F 50; origin: Evonik ⁽⁷⁾ Xiameter^{®} MEM-1691; origin: Dow Corning ⁽⁸⁾ Lanette^{®} 16; origin: BASF ⁽⁹⁾ Comperlan^{®} 100; origin: Cognis ⁽¹⁰⁾ Cutina^{®} AGS; origin: Cognis ⁽¹¹⁾ Kathon^{®} CG; origin: Rohm & Haas ⁽¹²⁾ D-Panthenol; origin: Roche | | |

### Example 12

### Preparation of a rinse-off hair conditioner formulation comprising an invention's compound of formula (I)

A typical unperfumed rinse-off hair conditioner formulation is listed in Table 9. The unperfumed rinse-off hair conditioner formulation is prepared by mixing the ingredients of Phase A until a uniform mixture was obtained. Tylose^{®} is allowed to completely dissolve. Then the mixture is heated to 70-75°C. The ingredients of Phase B are combined and melted at 70-75°C. Then the ingredients of Phase B are added to Phase A with good agitation, and the mixing is continued until that the mixture has a temperature of 60°C. Then, the ingredients of Phase C are added while agitating and keeping mixing until the mixture cooled to 40°C. The pH is adjusted with a citric acid solution to 3.5-4.0. A perfumed rinse-off hair conditioner formulation is obtained by adding, under gentle shaking, the perfume oil of Example 5 (0.2 to 1.0% by weight relative to the total weight of the unperfumed conditioner formulation) and at least one of the compounds of formula (I) (0.05 to 0.5% by weight relative to the total weight of the unperfumed conditioner formulation) into the unperfumed rinse-off hair conditioner formulation listed in Table 9.

**Table 9: Composition of a typical unperfumed rinse-off hair conditioner formulation**

| **Phase** | **Ingredients** | **Amount [wt%]** |
|---|---|---|
| A | Deionized water | 81.8 |
| | Behentrimonium chloride ⁽¹⁾ | 2.5 |
| | Hydroxyethylcellulose ⁽²⁾ | 1.5 |
| B | Cetearyl alcohol ⁽³⁾ | 4.0 |
| | Glyceryl stearate (and) PEG-100 stearate ⁽⁴⁾ | 2.0 |
| | Behentrimonium metho-sulfate (and) cetyl alcohol (and) butylene glycol ⁽⁵⁾ | 4.0 |
| | Ethoxy (20) stearyl alcohol ⁽⁶⁾ | 1.0 |
| C | Amodimethicone (and) Trideceth-12 (and) Cetrimonium chloride ⁽⁷⁾ | 3.0 |
| | Chlorhexidine digluconate (20% aqueous solution) (8) | 0.2 |
| D | Citric acid (10% aqueous solution tol pH 3.5-4.0) | q.s. |

| | | |
|---|---|---|
| ⁽¹⁾ Genamin^{®} KDMP; origin: Clariant ⁽²⁾ Tylose^{®} H10 Y G4; origin: Shin Etsu ⁽³⁾ Lanette^{®} O; origin: BASF ⁽⁴⁾ Arlacel^{®} 165; origin: Croda ⁽⁵⁾ Incroquat^{®} Behenyl TMS-50-PA- (MH); origin: Croda ⁽⁶⁾ Brij^{®} S20; origin: Croda ⁽⁷⁾ Xiameter^{®} MEM-949; origin: Dow Corning ⁽⁸⁾ Origin: Alfa Aesar | | |

### Example 13

### Preparation of a structured shower gel formulation comprising an invention's compound of formula (I)

A typical unperfumed structured shower gel formulation is listed in Table 10. A perfumed structured shower gel is prepared by adding, under gentle shaking, the perfume oil of Example 5 (0.1 to 1.5% by weight relative to the total weight of the structured shower gel) and at least one of the invention's compounds of formula (I) (0.05 to 0.5% by weight relative to the total weight of the structured shower gel) into the unperfumed structured shower gel formulation of Table 10.

**Table 10: Composition of a typical unperfumed structured shower gel formulation**

| **Ingredients** | **Amount [wt%]** |
|---|---|
| Deionized water | 49.35 |
| Tetrasodium EDTA ⁽¹⁾ | 0.05 |
| Acrylates co-polymer ⁽²⁾ | 6.00 |
| Sodium C₁₂₋₁₅ pareth sulfate ⁽³⁾ | 35.00 |
| Sodium hydroxide (20% aqueous solution) | 1.00 |
| Cocamidopropyl betaine ⁽⁴⁾ | 8.00 |
| Methylchloroisothiazolinone and methylisothiazolinone ⁽⁵⁾ | 0.10 |
| Citric acid (40% aqueous solution) | 0.50 |

| | |
|---|---|
| ⁽¹⁾ EDETA B powder; origin: BASF ⁽²⁾ Carbopol Aqua SF-1 polymer; origin: Noveon ⁽³⁾ Zetesol AO 328 U; origin: Zschimmer & Schwarz ⁽⁴⁾ Tego Betain F 50; origin: Goldschmidt ⁽⁵⁾ Kathon^{®} CG; origin: Rohm & Haas | |

### Example 14

### Preparation of a transparent shower gel formulation comprising an invention's compound of formula (I)

A typical unperfumed transparent shower gel formulation is listed in Table 11. A perfumed transparent shower gel is prepared by adding, under gentle shaking, the perfume oil of Example 5 (0.5 to 1.5% by weight relative to the total weight of the transparent shower gel) and at least one of the invention's compounds of formula (I) (0.05 to 0.5% by weight relative to the total weight of the transparent shower gel) into the unperfumed transparent shower gel formulation of Table 11.

**Table 11: Composition of a typical unperfumed transparent shower gel formulation**

| **Ingredients** | **Amount [wt%]** |
|---|---|
| Deionized water | 52.40 |
| Tetrasodium EDTA ⁽¹⁾ | 0.10 |
| Sodium benzoate | 0.50 |
| Propylene glycol | 2.00 |
| Sodium C₁₂₋₁₅ pareth sulfate ⁽²⁾ | 35.00 |
| Cocamidopropyl betaine ⁽³⁾ | 8.00 |
| Polyquaternium-7 ⁽⁴⁾ | 0.20 |
| Citric acid (40% aqueous solution) | 1.00 |
| Sodium chloride | 0.80 |

| | |
|---|---|
| ⁽¹⁾ EDETA B powder; origin: BASF ⁽²⁾ Zetesol AO 328 U; origin: Zschimmer & Schwarz ⁽³⁾ Tego Betain F 50; origin: Goldschmidt ⁽⁴⁾ Merquat^{®} 550; origin: Lubrizol | |

### Example 15

### Preparation of a milky shower gel formulation comprising an invention's compound of formula (I)

A typical unperfumed milky shower gel formulation is listed in Table 12. A perfumed milky shower gel is prepared by adding, under gentle shaking, the perfume oil of Example 5 (0.1 to 1.5% by weight relative to the total weight of the milky shower gel) and at least one of the invention's compounds of formula (I) (0.05 to 0.5% by weight relative to the total weight of the milky shower gel) into the unperfumed milky shower gel formulation of Table 12.

**Table 12: Composition of a typical unperfumed milky shower gel formulation**

| **Ingredients** | **Amount [wt%]** |
|---|---|
| Deionized water | 50.95 |
| Tetrasodium EDTA ⁽¹⁾ | 0.05 |
| Sodium benzoate | 0.50 |
| Glycerin (86% aqueous solution) | 3.50 |
| Sodium laureth sulfate ⁽²⁾ | 27.00 |
| Polyquaternium-7 ⁽³⁾ | 1.00 |
| Coco-betaine ⁽⁴⁾ | 6.00 |
| PEG-120 Methyl glucose trioleate ⁽⁵⁾ | 1.00 |
| Citric acid (40% aqueous solution) | 1.00 |
| Glycol distearate & laureth-4 & cocamidopropyl betaine ⁽⁶⁾ | 3.00 |
| Sodium chloride (20% aqueous solution) | 5.00 |
| PEG-40 hydrogenated castor oil ⁽⁷⁾ | 1.00 |

| | |
|---|---|
| ⁽¹⁾ EDETA^{®} B powder; origin: BASF ⁽²⁾ Texapon^{®} NSO IS; origin: Cognis ⁽³⁾ Merquat^{®} 550; origin: Lubrizol ⁽⁴⁾ Dehyton^{®} AB-30; origin: Cognis ⁽⁵⁾ Glucamate^{®} LT; origin: Lubrizol ⁽⁶⁾ Euperlan^{®} PK 3000 AM; origin: Cognis ⁽⁷⁾ Cremophor^{®} RH 40; origin: BASF | |

## Claims

1. A compound of formula 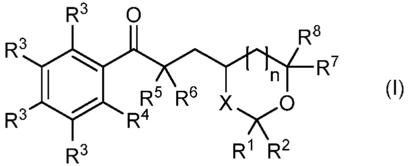
in the form of any one of its stereoisomers or a mixture thereof, and wherein
n is 0 or 1;
X is an oxygen atom or a NR⁹ group wherein R⁹ is a hydrogen atom or a methyl group;
R¹ represents a C₁₋₁₈ hydrocarbon group, optionally comprising one to three oxygen atoms and/or one to two nitrogen atoms and/or one sulfur atom; R² represents a hydrogen atom or a R¹ group; or R¹ and R², when taken together, form a C₅₋₁₆ cycloalkyl, C₅₋₁₆ cycloalkenyl, C₄₋₁₄ heterocycloalkyl or C₄₋₁₄ heterocycloalkenyl group, each optionally substituted with one or more of a C₁₋₁₅ alkyl, C₂₋₁₅ alkenyl, C₁₋₁₅ alkoxy, C₃₋₁₅ cycloalkyl, C₅₋₁₅ cycloalkenyl, C₆₋₁₀ aryl and/or C₆₋₁₀ aryloxy group, each optionally substituted with one or more of a C₁₋₈ alkyl, C₁₋₈ alkoxy, carboxylic acid and/or C₁₋₄ carboxylic ester group, wherein the heteroatom represents one or more of an oxygen atom;
R³ and R⁴ represent, independent of each other, a hydrogen atom, a C₁₋₆ alkoxy group or a C₁₋₁₂ alkyl group, optionally substituted by a hydroxy, C₁₋₆ alkoxy or oxo group, or, two adjacent R³ groups, when taken together, are a C₃₋₈ linear alkanediyl group optionally substituted by one or more of a hydroxy, C₁₋₃ alkyl and/or C₁₋₃ alkoxy group;
R⁵ represents a hydrogen atom or a C₁₋₆ hydrocarbon group, or R⁴ and R⁵, when taken together, represent a C₁₋₄ linear, branched or cyclic alkanediyl group, optionally comprising one oxygen atom;
R⁶ represents a hydrogen atom or a methyl group;
R⁷ and R⁸ represent, independent of each other, a hydrogen atom or a C₁₋₆ alkyl group; and
the groups R¹ and R² have in total at least 4 carbon atoms.

2. The compound according to claim 1, wherein R⁷ and R⁸ represent, independent of each other, a hydrogen atom or a methyl group; preferably a hydrogen atom.

3. The compound according to any one of claims 1 to 2, wherein R⁶ represents a hydrogen atom.

4. The compound according to any one of claims 1 to 3, wherein R⁵ represents a hydrogen atom or a C₁₋₄ linear or branched alkyl group or a C₂₋₄ linear or branched alkenyl group; preferably R⁵ represents a hydrogen atom.

5. The compound according to any one of claims 1 to 4, wherein X is an oxygen atom and wherein n is 0.

6. The compound according to any one of claims 1 to 5, wherein R³ and R⁴ represent, independent of each other, a hydrogen atom, a C₁₋₃ alkoxy group or a C₁₋₆ alkyl group, optionally substituted by a hydroxy, C₁₋₃ alkoxy or oxo group, or, two adjacent R³ groups, when taken together, are a C₃₋₄ linear alkanediyl group optionally substituted by one or more of a hydroxy and/or C₁₋₃ alkyl group.

7. The compound according to any one of claims 1 to 6, wherein R¹ represents a C₁₋₁₂ hydrocarbon group, optionally comprising one to three oxygen atoms; or R¹ and R², when taken together, form a C₅₋₁₆ cycloalkyl or C₅₋₁₆ cycloalkenyl group, each optionally substituted with one or more of a C₁₋₈ alkyl, C₂₋₈ alkenyl, C₁₋₈ alkoxy, C₃₋₈ cycloalkyl, C₅₋₈ cycloalkenyl, C₆ aryl and/or C₆ aryloxy group, each optionally substituted with one or more of a C₁₋₆ alkyl, C₁₋₆ alkoxy, carboxylic acid and/or C₁₋₄ carboxylic ester group.

8. The compound according to any one of claims 1 to 7, wherein the compound of formula (I) is selected from the group consisting of 1-phenyl-3-(2-phenyl-1,3-dioxan-4-yl)propan-1-one, 3-(2-decyl-1,3-dioxolan-4-yl)-1-phenylpropan-1-one, 1-phenyl-3-(2-(2-phenylpropyl)-1,3-dioxolan-4-yl)propan-1-one, 1-phenyl-3-(2-(undecan-2-yl)-1,3-dioxolan-4-yl)propan-1-one, 3-(2-(dec-9-en-1-yl)-1,3-dioxolan-4-yl)-1-phenylpropan-1-one, 3-(2-(2,4-dimethylcyclohex-3-en-1-yl)-1,3-dioxolan-4-yl)-1-phenylpropan-1-one, 3-(2-(2-(4,4-dimethylcyclohex-1-en-1-yl)ethyl)-1,3-dioxolan-4-yl)-1-phenylpropan-1-one, 1-phenyl-3-(2-(undec-3-en-1-yl)-1,3-dioxolan-4-yl)propan-1-one, 3-(2-(2-(4-methylcyclohex-3-en-1-yl)propyl)-1,3-dioxolan-4-yl)-1-phenylpropan-1-one, 3-(2-(6-methylhept-5-en-2-yl)-1,3-dioxolan-4-yl)-1-phenylpropan-1-one, (E/Z)-3-(2-(4,8-dimethylnon-3-en-1-yl)-1,3-dioxolan-4-yl)-1-phenylpropan-1-one, (E/Z)-3-(2-(5-cyclohexyl-4-methylpent-4-en-2-yl)-1,3-dioxolan-4-yl)-1-phenylpropan-1-one, 3-(2-methyl-2-phenyl-1,3-dioxolan-4-yl)-1-phenylpropan-1-one, 3-(2-methyl-2-phenethyl-1,3-dioxolan-4-yl)-1-phenylpropan-1-one, 3-(6-pentyl-1,4-dioxaspiro[4.4]nonan-2-yl)-1-phenylpropan-1-one, 1-phenyl-3-(2-phenyl-1,3-dioxolan-4-yl)propan-1-one, 3-(2-decyl-1,3-dioxolan-4-yl)-1-(4-methoxyphenyl)propan-1-one, 3-(2-decyl-1,3-dioxolan-4-yl)-1-(p-tolyl)propan-1-one, 3-(2-(4-methoxyphenyl)-1,3-dioxan-4-yl)-1-phenylpropan-1-one, 3-(2-(4-ethylphenyl)-1,3-dioxan-4-yl)-1-phenylpropan-1-one, 3-(2-decyl-1,3-dioxan-4-yl)-1-phenylpropan-1-one, 3-(2-decyl-1,3-dioxan-4-yl)-1-phenylpropan-1-one, 2-methyl-3-(2-phenethyl-1,3-dioxolan-4-yl)-1-phenylpropan-1-one, 2,2-dimethyl-3-(2-phenethyl-1,3-dioxolan-4-yl)-1-phenylpropan-1-one, 3-(2-phenethyl-1,3-dioxolan-4-yl)-1-(5,6,7,8-tetrahydronaphthalen-2-yl)propan-1-one, 3-(5-methyl-2-phenethyl-1,3-dioxolan-4-yl)-1-phenylpropan-1-one and 3-(5,5-dimethyl-2-phenethyl-1,3-dioxolan-4-yl)-1-phenylpropan-1-one, in the form of any one of its stereoisomers.

9. Use of a compound of formula (I) as defined in any one of claims 1 to 8 as perfuming ingredient to provide a long-lasting odor.

10. A method to confer, enhance, improve or modify the odor properties of a perfuming composition the air surrounding the perfuming composition, a surface or a perfumed article, comprising adding to the composition, the air, or article, or contacting or treating the surface with an effective amount of at least one compound of formula (I) as defined in claims 1 to 8.

11. A perfuming composition comprising
i) at least one compound of formula (I), as defined in any one of claims 1 to 8;
ii) at least one ingredient selected from the group consisting of a perfumery carrier and a perfumery base; and
iii) optionally at least one perfumery adjuvant.

12. A perfumed consumer product comprising at least one compound of formula (I), as defined in any one of claims 1 to 8 or a perfuming composition as defined in claim 11.

13. The perfumed consumer product according to claim 12, wherein the perfumery consumer product is a perfume, a fabric care product, a body-care product, a cosmetic preparation, a skin-care product, an air care product or a home care product.

14. The perfumed consumer product according to claim 13, wherein the perfumery consumer product is a fine perfume, a splash or eau de parfum, a cologne, a shave or after-shave lotion, a liquid or solid detergent optionally in the form of a pod or tablet, a fabric softener, a liquid or solid scent booster, a dryer sheet, a fabric refresher, an ironing water, a paper, a bleach, a carpet cleaner, a curtain-care product, a shampoo, a leave-on or rinse-off hair conditioner, a coloring preparation, a color care product, a hair shaping product, a dental care product, a disinfectant, an intimate care product, a hair spray, a skin cream or lotion, a vanishing cream, a deodorant or antiperspirant, a hair remover, a tanning or sun product, a nail product, a skin cleansing, a makeup, a soap, a shower or bath mousse, an oil or gel, a foot/hand care product, a hygiene product, an air freshener, a "ready to use" powdered air freshener, a mold remover, a furniture care product, a wipe, a dish detergent or hard-surface detergent, a leather care product, a car air-freshener, a polish, a wax or a plastic cleaner.

15. A method to release from a precursor compound, compounds selected from
the group consisting of
a) a carbonyl compound of formula 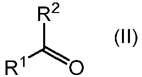 in the form of any one of its stereoisomers or a mixture thereof, wherein R¹ represents a C₁-₁₈ hydrocarbon group, optionally comprising one to three oxygen atoms and/or one to two nitrogen atoms and/or one sulfur atom; R² represents a hydrogen atom or a R¹ group; or R¹ and R², when taken together, form a C₅₋₁₆ cycloalkyl, C₅₋₁₆ cycloalkenyl, C₄₋₁₄ heterocycloalkyl or C₄₋₁₄ heterocycloalkenyl group, each optionally substituted with one or more of a C₁₋₁₅ alkyl, C₂₋₁₅ alkenyl, C₁₋₁₅ alkoxy, C₃₋₁₅ cycloalkyl, C₅₋₁₅ cycloalkenyl, C₆₋₁₀ aryl and/or C₆₋₁₀ aryloxy group, each optionally substituted with one or more of a C₁₋₈ alkyl, C₁₋₈ alkoxy, carboxylic acid and/or C₁₋₄ carboxylic ester group, wherein the heteroatom represents one or more of an oxygen atom;
b) a ketone of formula 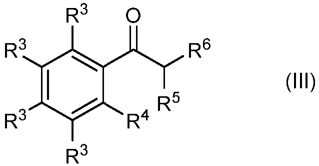
in the form of any one of its stereoisomers or a mixture thereof, and wherein R³ and R⁴ represent, independent of each other, a hydrogen atom, a C₁₋₆ alkoxy group or a C₁₋₁₂ alkyl group, optionally substituted by a hydroxy, C₁₋₆ alkoxy or oxo group, or, two adjacent R³ groups, when taken together, are a C₃₋₈ linear alkanediyl group optionally substituted by one or more of a hydroxyl, C₁₋₃ alkyl and/or C₁₋₃ alkoxy group; R⁵ represents a hydrogen atom or a C₁₋₆ hydrocarbon group, or R⁴ and R⁵, when taken together, represent a C₁₋₄ linear, branched or cyclic alkanediyl group, optionally comprising one oxygen atom; R⁶ represents a hydrogen atom or a methyl group;
wherein the precursor compound is a compound of formula (I) 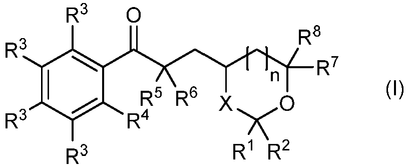
in the form of any one of its stereoisomers or a mixture thereof, and wherein R¹, R², R³, R⁴ , R⁵ and R⁶ have the same meaning as defined above; n is 0 or 1; X is an oxygen atom or a NR⁹ group wherein R⁹ is a hydrogen atom or a methyl group; and R⁷ and R⁸ represent, independent of each other, a hydrogen atom or a C₁₋₆ alkyl group;
by exposing the precursor compound of formula (I) to light and to an environment wherein the compound is hydrolyzed.

## Patentansprüche

1. Verbindung der Formel 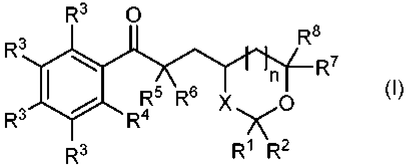
in Form eines ihrer Stereoisomere oder eines Gemischs davon und wobei n für 0 oder 1 steht;
X für ein Sauerstoffatom oder eine NR⁹-Gruppe steht, wobei R⁹ für ein Wasserstoffatom oder eine Methylgruppe steht; R¹ für eine C₁₋₁₈-Kohlenwasserstoffgruppe steht, die gegebenenfalls ein bis drei Sauerstoffatome und/oder ein bis zwei Stickstoffatome und/oder ein Schwefelatom umfasst; R² für ein Wasserstoffatom oder eine R¹-Gruppe steht; oder R¹ und R² zusammengenommen eine C₅₋₁₆-Cycloalkyl-, C₅₋₁₆-Cycloalkenyl-, C₄₋₁₄-Heterocycloalkyl- oder C₄₋₁₄-Heterocycloalkenylgruppe bilden, wobei jede dieser Gruppen gegebenenfalls durch eines oder mehrere von einer C₁₋₁₅-Alkyl-, C₂₋₁₅-Alkenyl-, C₁₋₁₅-Alkoxy-, C₃₋₁₅-Cycloalkyl-, C₅₋₁₅-Cycloalkenyl-, C₆₋₁₀-Aryl- und/oder C₆₋₁₀-Aryloxygruppe substituiert ist, wobei jede dieser Gruppen gegebenenfalls durch eines oder mehrere von einer C₁₋₈-Alkyl-, C₁₋₈-Alkoxy-, Carbonsäure- und/oder C₁₋₄-Carbonsäureestergruppe substituiert ist, wobei das Heteroatom für eines oder mehrere von einem Sauerstoffatom steht;
R³ und R⁴ unabhängig voneinander für ein Wasserstoffatom, eine C₁₋₆-Alkoxygruppe oder eine C₁₋₁₂-Alkylgruppe, die gegebenenfalls durch eine Hydroxy-, C₁₋₆-Alkoxy- oder Oxogruppe substituiert ist, stehen oder zwei benachbarte R³-Gruppen zusammengenommen eine lineare C₃₋₈-Alkandiylgruppe sind, die gegebenenfalls durch eines oder mehrere von einer Hydroxy-, C₁₋₃-Alkyl- und/oder C₁₋₃-Alkoxygruppe substituiert ist;
R⁵ für ein Wasserstoffatom oder eine C₁₋₆-Kohlenwasserstoffgruppe steht oder R⁴ und R⁵ zusammengenommen für eine lineare, verzweigte oder cyclische C₁₋₄-Alkandiylgruppe stehen, die gegebenenfalls ein Sauerstoffatom umfasst;
R⁶ für ein Wasserstoffatom oder eine Methylgruppe steht; R⁷ und R⁸ unabhängig voneinander für ein Wasserstoffatom oder eine C₁₋₆-Alkylgruppe stehen; und
die Gruppen R¹ und R² insgesamt mindestens 4 Kohlenstoffatome aufweisen.

2. Verbindung nach Anspruch 1, wobei R⁷ und R⁸ unabhängig voneinander für ein Wasserstoffatom oder eine Methylgruppe, vorzugsweise ein Wasserstoffatom, stehen.

3. Verbindung nach einem der Ansprüche 1 bis 2, wobei R⁶ für ein Wasserstoffatom steht.

4. Verbindung nach einem der Ansprüche 1 bis 3, wobei R⁵ für ein Wasserstoffatom oder eine lineare oder verzweigte C₁₋₄-Alkylgruppe oder eine lineare oder verzweigte C₂₋₄-Alkenylgruppe steht; vorzugsweise R⁵ für ein Wasserstoffatom steht.

5. Verbindung nach einem der Ansprüche 1 bis 4, wobei X für ein Sauerstoffatom steht und wobei n für 0 steht.

6. Verbindung nach einem der Ansprüche 1 bis 5, wobei R³ und R⁴ unabhängig voneinander für ein Wasserstoffatom, eine C₁₋₃-Alkoxygruppe oder eine C₁₋₆-Alkylgruppe, die gegebenenfalls durch eine Hydroxy-, C₁₋₃-Alkoxy- oder Oxogruppe substituiert ist, stehen oder zwei benachbarte R³-Gruppen zusammengenommen eine lineare C₃₋₄-Alkandiylgruppe sind, die gegebenenfalls durch eines oder mehrere von einer Hydroxy- und/oder C₁₋₃-Alkylgruppen substituiert ist.

7. Verbindung nach einem der Ansprüche 1 bis 6, wobei R¹ für eine C₁₋₁₂-Kohlenwasserstoffgruppe steht, die gegebenenfalls ein bis drei Sauerstoffatome umfasst; oder R¹ und R² zusammengenommen eine C₅₋₁₆-Cycloalkyl- oder C₅₋₁₆-Cycloalkenylgruppe bilden, wobei jede dieser Gruppen gegebenenfalls durch eines oder mehrere von einer C₁₋₈-Alkyl-, C₂₋₈-Alkenyl-, C₁₋₈-Alkoxy, C₃₋₈-Cycloalkyl, C₅₋₈-Cycloalkenyl, C₆-Aryl- und/oder C₆-Aryloxygruppe substituiert ist, wobei jede dieser Gruppen gegebenenfalls durch eines oder mehrere von einer C₁₋₆-Alkyl-, C₁₋₆-Alkoxy-, carbonsäure- und/oder C₁₋₄-Carbonsäureestergruppe substituiert ist.

8. Verbindung nach einem der Ansprüche 1 bis 7, wobei die Verbindung der Formel (I) aus der Gruppe bestehend aus 1-Phenyl-3-(2-phenyl-1,3-dioxan-4-yl)propan-1-on, 3-(2-Decyl-1,3-dioxolan-4-yl)-1-phenylpropan-1-on, 1-Phenyl-3-(2-(2-phenylpropyl)-1,3-dioxolan-4-yl)propan-1-on, 1-Phenyl-3-(2-(undecan-2-yl)-1,3-dioxolan-4-yl)propan-1-on, 3-(2-(Dec-9-en-1-yl)-1,3-dioxolan-4-yl)-1-phenylpropan-1-on, 3-(2-(2,4-Dimethylcyclohex-3-en-1-yl)-1,3-dioxolan-4-yl)-1-phenylpropan-1-on, 3-(2-(2-(4,4-Dimethylcyclohex-1-en-1-yl)ethyl)-1,3-dioxolan-4-yl)-1-phenylpropan-1-on, 1-Phenyl-3-(2-(undec-3-en-1-yl)-1,3-dioxolan-4-yl)propan-1-on, 3-(2-(2-(4-Methylcyclohex-3-en-1-yl)propyl)-1,3-dioxolan-4-yl)-1-phenylpropan-1-on, 3-(2-(6-Methylhept-5-en-2-yl)-1,3-dioxolan-4-yl)-1-phenylpropan-1-on, (E/Z)-3-(2-(4,8-Dimethylnon-3-en-1-yl)-1,3-dioxolan-4-yl)-1-phenylpropan-1-on, (E/Z)-3-(2-(5-Cyclohexyl-4-methylpent-4-en-2-yl)-1,3-dioxolan-4-yl)-1-phenylpropan-1-on, 3-(2-Methyl-2-phenyl-1,3-dioxolan-4-yl)-1-phenylpropan-1-on, 3-(2-Methyl-2-phenethyl-1,3-dioxolan-4-yl)-1-phenylpropan-1-on, 3-(6-Pentyl-1,4-dioxaspiro[4.4]nonan-2-yl)-1-phenylpropan-1-on, 1-Phenyl-3-(2-phenyl-1,3-dioxolan-4-yl)propan-1-on, 3-(2-Decyl-1,3-dioxolan-4-yl)-1-(4-methoxyphenyl)propan-1-on, 3-(2-Decyl-1,3-dioxolan-4-yl)-1-(p-tolyl)propan-1-on, 3-(2-(4-Methoxyphenyl)-1,3-dioxan-4-yl)-1-phenylpropan-1-on, 3-(2-(4-Ethylphenyl)-1,3-dioxan-4-yl)-1-phenylpropan-1-on, 3-(2-Decyl-1,3-dioxan-4-yl)-1-phenylpropan-1-on, 3-(2-Decyl-1,3-dioxan-4-yl)-1-phenylpropan-1-on, 2-Methyl-3-(2-phenethyl-1,3-dioxolan-4-yl)-1-phenylpropan-1-on, 2,2-Dimethyl-3-(2-phenethyl-1,3-dioxolan-4-yl)-1-phenylpropan-1-on, 3-(2-Phenethyl-1,3-dioxolan-4-yl)-1-(5,6,7,8-tetrahydronaphthalen-2-yl)propan-1-on, 3-(5-Methyl-2-phenethyl-1,3-dioxolan-4-yl)-1-phenylpropan-1-on und 3-(5,5-Dimethyl-2-phenethyl-1,3-dioxolan-4-yl)-1-phenylpropan-1-on in Form eines ihrer Stereoisomere ausgewählt ist.

9. Verwendung einer Verbindung der Formel (I) gemäß einem der Ansprüche 1 bis 8 als Parfümierungsbestandteil zur Bereitstellung eines lang anhaltenden Geruchs.

10. Verfahren zum Verleihen, Verstärken, Verbessern oder Modifizieren der Geruchseigenschaft einer Parfümierungszusammensetzung, der die Parfümierungszusammensetzung umgebenden Luft, einer Oberfläche oder eines parfümierten Artikels, umfassend das Zugeben einer wirksamen Menge mindestens einer Verbindung der Formel (I) gemäß den Ansprüchen 1 bis 8 zu der Zusammensetzung, der Luft bzw. dem Artikel oder das Inkontaktbringen oder Behandeln der Oberfläche mit einer wirksamen Menge mindestens einer Verbindung der Formel (I) gemäß den Ansprüchen 1 bis 8.

11. Parfümierungszusammensetzung, umfassend
i) mindestens eine Verbindung der Formel (I) gemäß einem der Ansprüche 1 bis 8;
ii) mindestens einen Bestandteil, der aus der Gruppe bestehend aus einem Parfümträger und einer Parfümgrundlage ausgewählt ist; und
iii) gegebenenfalls mindestens ein Parfümadjuvans.

12. Parfümiertes Konsumprodukt, umfassend mindestens eine Verbindung der Formel (I) gemäß einem der Ansprüche 1 bis 8 oder einer Parfümierungszusammensetzung gemäß Anspruch 11.

13. Parfümiertes Konsumprodukt nach Anspruch 12, wobei es sich bei dem Konsumprodukt um ein Parfüm, ein Textilpflegeprodukt, ein Körperpflegeprodukt, eine Kosmetikzubereitung, ein Hautpflegeprodukt, ein Luftpflegeprodukt oder ein Haushaltspflegeprodukt handelt.

14. Parfümiertes Konsumprodukt nach Anspruch 13, wobei es sich bei dem Parfüm-Konsumprodukt um ein Feinparfüm, ein Splash oder Eau de Parfum, ein Eau de Cologne, eine Rasier- oder After-Shave-Lotion, ein flüssiges des festes Waschmittel, gegebenenfalls in Form eines Pods oder einer Tablette, einen Textilweichmacher, einen flüssigen oder festen Duftverstärker, ein Trocknertuch, einen Textilauffrischer, ein Bügelwasser, ein Papier, ein Bleichmittel, einen Teppichreiniger, ein Vorhangpflegeprodukt, ein Shampoo, einen Conditioner, der im Haar verbleibt oder ausgespült wird, ein Färbepräparat, ein Farbpflegeprodukt, ein Haarformungsprodukt, ein Zahnpflegeprodukt, ein Desinfektionsmittel, ein Intimpflegeprodukt, ein Haarsprays, eine Hautcreme oder -lotion, eine Abdeckcreme, ein Deodorant oder Antitranspirant, einen Haarentferner, ein Bräunungs- oder Sonnenprodukt, ein Nagelprodukt, ein Hautreinigungsmittel, ein Make-up, eine Seife, einen Dusch- oder Badeschaum, ein Dusch- oder Badeöl oder -gel, ein Fuß-/Handpflegeprodukt, ein Hygieneprodukt, einen Lufterfrischer, einen gebrauchsfertigen pulverförmigen Lufterfrischer, einen Schimmelentferner, ein Möbelpflegeprodukt, ein Wischtuch, ein Geschirrspülmitteln oder Reinigungsmittel für harte Oberflächen, ein Lederpflegeprodukt, einen Lufterfrischer fürs Auto, eine Politur, einen Wachs oder einen Kunststoffreiniger handelt.

15. Verfahren zum Freisetzen von Verbindungen aus einer Vorläuferverbindung, wobei die Verbindungen ausgewählt sind aus der Gruppe bestehend aus
a) einer Carbonylverbindung der Formel 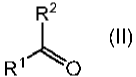 in Form eines ihrer Stereoisomere oder eines Gemischs davon, wobei R¹ für eine C₁₋₁₈-Kohlenwasserstoffgruppe steht, die gegebenenfalls ein bis drei Sauerstoffatome und/oder ein bis zwei Stickstoffatome und/oder ein Schwefelatom umfasst; R² für ein Wasserstoffatom oder eine R¹-Gruppe steht; oder R¹ und R² zusammengenommen eine C₅₋₁₆-Cycloalkyl-, C₅₋₁₆-Cycloalkenyl-, C₄₋₁₄-Heterocycloalkyl- oder C₄₋₁₄-Heterocycloalkenylgruppe bilden, wobei jede dieser Gruppen gegebenenfalls durch eines oder mehrere von einer C₁₋₁₅-Alkyl-, C₂₋₁₅-Alkenyl-, C₁₋₁₅-Alkoxy-, C₃₋₁₅-Cycloalkyl-, C₅₋₁₅-Cycloalkenyl-, C₆₋₁₀-Aryl- und/oder C₆₋₁₀-Aryloxygruppe substituiert ist, wobei jede dieser Gruppen gegebenenfalls durch eines oder mehrere von einer C₁₋₈-Alkyl-, C₁₋₈-Alkoxy-, Carbonsäure- und/oder C₁₋₄-Carbonsäureestergruppe substituiert ist, wobei das Heteroatom für eines oder mehrere von einem Sauerstoffatom steht;
b) einem Keton der Formel 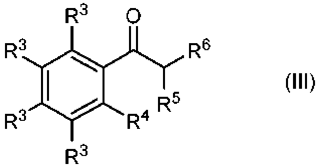 in Form eines ihrer Stereoisomere oder eines Gemischs davon und wobei R³ und R⁴ unabhängig voneinander für ein Wasserstoffatom, eine C₁₋₆-Alkoxygruppe oder eine C₁₋₁₂-Alkylgruppe, die gegebenenfalls durch eine Hydroxy-, C₁₋₆-Alkoxy- oder Oxogruppe substituiert ist, stehen oder zwei benachbarte R³-Gruppen zusammengenommen eine lineare C₃₋₈-Alkandiylgruppe sind, die gegebenenfalls durch eines oder mehrere von einer Hydroxyl-, C₁₋₃-Alkyl- und/oder C₁₋₃-Alkoxygruppe substituiert ist; R⁵ für ein Wasserstoffatom oder eine C₁₋₆-Kohlenwasserstoffgruppe steht oder R⁴ und R⁵ zusammengenommen für eine lineare, verzweigte oder cyclische C₁₋₄-Alkandiylgruppe stehen, die gegebenenfalls ein Sauerstoffatom umfasst; R⁶ für ein Wasserstoffatom oder eine Methylgruppe steht;
wobei es sich bei der Vorläuferverbindung um eine Verbindung der Formel (I) 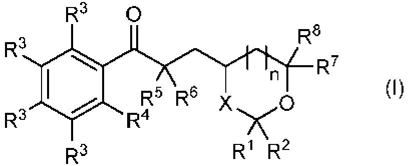 in Form eines ihrer Stereoisomere oder eines Gemischs davon handelt und wobei R¹, R², R³, R⁴, R⁵ und R⁶ die gleiche Bedeutung wie oben definiert haben; n für 0 oder 1 steht; X für ein Sauerstoffatom oder eine NR⁹-Gruppe steht, wobei R⁹ für ein Wasserstoffatom oder eine Methylgruppe steht; und R⁷ und R⁸ unabhängig voneinander für ein Wasserstoffatom oder eine C₁₋₆-Alkylgruppe stehen; indem man die Vorläuferverbindung der Formel (I) Licht und einer Umgebung, in der die Verbindung hydrolysiert wird, aussetzt.

## Revendications

1. Composé de formule 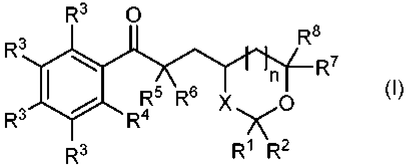
sous la forme de l'un quelconque de ses stéréoisomères ou d'un mélange de ceux-ci et dans lequel n vaut 0 ou 1 ;
X est un atome d'oxygène ou un groupe NR⁹, R⁹ étant un atome d'hydrogène ou un groupe méthyle ;
R¹ représente un groupe hydrocarboné en C₁₋₁₈, comprenant éventuellement un à trois atomes d'oxygène et/ou un à deux atomes d'azote et/ou un atome de soufre ; R² représente un atome d'hydrogène ou un groupe R¹ ; ou R¹ et R², pris ensemble, forment un groupe cycloalkyle en C₅₋₁₆, cycloalcényle en C₅₋₁₆, hétérocycloalkyle en C₄₋₁₄ ou hétérocycloalcényle en C₄₋₁₄, chacun éventuellement substitué par un ou plusieurs groupes parmi les groupes alkyle en C₁₋₁₅, alcényle en C₂₋₁₅, alcoxy en C₁₋₁₅, cycloalkyle en C₃₋₁₅, cycloalcényle en C₅₋₁₅, aryle en C₆₋₁₀ et/ou aryloxy en C₆₋₁₀, chacun éventuellement substitué par un ou plusieurs groupes parmi les groupes alkyle en C₁₋₈, alcoxy en C₁₋₈, acide carboxylique et/ou ester en C₁₋₄ d'acide carboxylique, l'hétéroatome représentant un ou plusieurs atomes parmi un atome d'oxygène ;
R³ et R⁴ représentent, chacun indépendamment de l'autre, un atome d'hydrogène, un groupe alcoxy en C₁₋₆ ou un groupe alkyle en C₁₋₁₂, éventuellement substitués par un groupe hydroxy, alcoxy en C₁₋₆ ou oxo, ou deux groupes R³ adjacents, pris ensemble, sont un groupe alcanediyle en C₃₋₈ linéaire éventuellement substitué par un ou plusieurs groupes parmi les groupes hydroxy, alkyle en C₁₋₃ et/ou alcoxy en C₁₋₃ ;
R⁵ représente un atome d'hydrogène ou un groupe hydrocarboné en C₁₋₆ ou R⁴ et R⁵, pris ensemble, représentent un groupe alcanediyle en C₁₋₄ linéaire, ramifié ou cyclique, comprenant éventuellement un atome d'oxygène ;
R⁶ représente un atome d'hydrogène ou un groupe méthyle ; R⁷ et R⁸ représentent, chacun indépendamment de l'autre, un atome d'hydrogène ou un groupe alkyle en C₁₋₆ ; et
les groupes R¹ et R² ont au total au moins 4 atomes de carbone.

2. Composé selon la revendication 1, dans lequel R⁷ et R⁸ représentent, chacun indépendamment de l'autre, un atome d'hydrogène ou un groupe méthyle ; de préférence un atome d'hydrogène.

3. Composé selon l'une quelconque des revendications 1 à 2, dans lequel R⁶ représente un atome d'hydrogène.

4. Composé selon l'une quelconque des revendications 1 à 3, dans lequel R⁵ représente un atome d'hydrogène ou un groupe alkyle en C₁₋₄ linéaire ou ramifié ou un groupe alcényle en C₂₋₄ linéaire ou ramifié ; de préférence R⁵ représente un atome d'hydrogène.

5. Composé selon l'une quelconque des revendications 1 à 4, dans lequel X est un atome d'oxygène et dans lequel n vaut 0.

6. Composé selon l'une quelconque des revendications 1 à 5, dans lequel R³ et R⁴ représentent, chacun indépendamment de l'autre, un atome d'hydrogène, un groupe alcoxy en C₁₋₃ ou un groupe alkyle en C₁₋₆, éventuellement substitués par un groupe hydroxy, alcoxy en C₁₋₃ ou oxo, ou deux groupes R³ adjacents, pris ensemble, sont un groupe alcanediyle en C₃₋₄ linéaire éventuellement substitué par un ou plusieurs groupes parmi les groupes hydroxy et/ou alkyle en C₁₋₃.

7. Composé selon l'une quelconque des revendications 1 à 6, dans lequel R¹ représente un groupe hydrocarboné en C₁₋₁₂, comprenant éventuellement un à trois atomes d'oxygène ; ou R¹ et R², pris ensemble, forment un groupe cycloalkyle en C₅₋₁₆ ou cycloalcényle en C₅₋₁₆, chacun éventuellement substitué par un ou plusieurs groupes parmi les groupes alkyle en C₁₋₈, alcényle en C₂₋₈, alcoxy en C₁₋₈, cycloalkyle en C₃₋₈, cycloalcényle en C₅₋₈, aryle en C₆ et/ou aryloxy en C₆, chacun éventuellement substitué par un ou plusieurs groupes parmi les groupes alkyle en C₁₋₆, alcoxy en C₁₋₆, acide carboxylique et/ou ester en C₁₋₄ d'acide carboxylique.

8. Composé selon l'une quelconque des revendications 1 à 7, le composé de formule (I) étant choisi dans le groupe constitué par la 1-phényl-3-(2-phényl-1,3-dioxan-4-yl)propan-1-one, la 3-(2-décyl-1,3-dioxolan-4-yl)-1-phénylpropan-1-one, la 1-phényl-3-(2-(2-phénylpropyl)-1,3-dioxolan-4-yl)propan-1-one, la 1-phényl-3-(2-(undécan-2-yl)-1,3-dioxolan-4-yl)propan-1-one, la 3-(2-(déc-9-én-1-yl)-1,3-dioxolan-4-yl)-1-phénylpropan-1-one, la 3-(2-(2,4-diméthylcyclohex-3-én-1-yl)-1,3-dioxolan-4-yl)-1-phénylpropan-1-one, la 3-(2-(2-(4,4-diméthylcyclohex-1-én-1-yl)éthyl)-1,3-dioxolan-4-yl)-1-phénylpropan-1-one, la 1-phényl-3-(2-(undéc-3-én-1-yl)-1,3-dioxolan-4-yl)propan-1-one, la 3-(2-(2-(4-méthylcyclohex-3-én-1-yl)propyl)-1,3-dioxolan-4-yl)-1-phénylpropan-1-one, la 3-(2-(6-méthylhept-5-én-2-yl)-1,3-dioxolan-4-yl)-1-phénylpropan-1-one, la (E/Z)-3-(2-(4,8-diméthylnon-3-én-1-yl)-1,3-dioxolan-4-yl)-1-phénylpropan-1-one, la (E/Z)-3-(2-(5-cyclohexyl-4-méthylpent-4-én-2-yl)-1,3-dioxolan-4-yl)-1-phénylpropan-1-one, la 3-(2-méthyl-2-phényl-1,3-dioxolan-4-yl)-1-phénylpropan-1-one, la 3-(2-méthyl-2-phénéthyl-1,3-dioxolan-4-yl)-1-phénylpropan-1-one, la 3-(6-pentyl-1,4-dioxaspiro[4.4]nonan-2-yl)-1-phénylpropan-1-one, la 1-phényl-3-(2-phényl-1,3-dioxolan-4-yl)propan-1-one, la 3-(2-décyl-1,3-dioxolan-4-yl)-1-(4-méthoxyphényl)propan-1-one, la 3-(2-décyl-1,3-dioxolan-4-yl)-1-(p-tolyl)propan-1-one, la 3-(2-(4-méthoxyphényl)-1,3-dioxan-4-yl)-1-phénylpropan-1-one, la 3-(2-(4-éthylphényl)-1,3-dioxan-4-yl)-1-phénylpropan-1-one, la 3-(2-décyl-1,3-dioxan-4-yl)-1-phénylpropan-1-one, la 3-(2-décyl-1,3-dioxan-4-yl)-1-phénylpropan-1-one, la 2-méthyl-3-(2-phénéthyl-1,3-dioxolan-4-yl)-1-phénylpropan-1-one, la 2,2-diméthyl-3-(2-phénéthyl-1,3-dioxolan-4-yl)-1-phénylpropan-1-one, la 3-(2-phénéthyl-1,3-dioxolan-4-yl)-1-(5,6,7,8-tétrahydronaphtalén-2-yl)propan-1-one, la 3-(5-méthyl-2-phénéthyl-1,3-dioxolan-4-yl)-1-phénylpropan-1-one et la 3-(5,5-diméthyl-2-phénéthyl-1,3-dioxolan-4-yl)-1-phénylpropan-1-one, sous la forme de l'un quelconque de ses stéréoisomères.

9. Utilisation d'un composé de formule (I) tel que défini dans l'une quelconque des revendications 1 à 8 en tant qu'ingrédient parfumant pour fournir une odeur de longue durée.

10. Procédé pour conférer, rehausser, améliorer ou modifier les propriétés odorantes d'une composition parfumante, de l'air entourant la composition parfumante, d'une surface ou d'un article parfumé, comprenant l'ajout à la composition, à l'air ou à l'article d'une quantité efficace d'au moins un composé de formule (I) tel que défini dans les revendications 1 à 8 ou la mise en contact ou le traitement de la surface avec celle-ci.

11. Composition parfumante comprenant
i) au moins un composé de formule (I), tel que défini dans l'une quelconque des revendications 1 à 8 ;
ii) au moins un ingrédient choisi dans le groupe constitué par un vecteur de parfumerie et une base de parfumerie ; et
iii) éventuellement au moins un adjuvant de parfumerie.

12. Produit de consommation parfumé comprenant au moins un composé de formule (I) tel que défini dans l'une quelconque des revendications 1 à 8 ou une composition parfumante telle que définie dans la revendication 11.

13. Produit de consommation parfumé selon la revendication 12, le produit de consommation de parfumerie étant un parfum, un produit d'entretien des tissus, un produit de soin du corps, une préparation cosmétique, un produit de soin de la peau, un produit d'assainissement de l'air ou un produit d'entretien ménager.

14. Produit de consommation parfumé selon la revendication 13, le produit de consommation de parfumerie étant un parfum fin, un splash ou une eau de parfum, une eau de Cologne, une lotion de rasage ou d'après-rasage, un détergent liquide ou solide éventuellement sous la forme d'un pod ou d'une tablette, un assouplissant des tissus, un booster de parfum liquide ou solide, une feuille pour sèche-linge, un rafraîchisseur des tissus, une eau de repassage, un papier, un produit de blanchiment, un nettoyant pour tapis, un produit d'entretien des rideaux, un shampooing, un après-shampooing avec ou sans rinçage, une préparation colorante, un produit de soin de la couleur, un produit de mise en forme des cheveux, un produit de soin dentaire, un désinfectant, un produit de soin intime, une laque, une crème ou lotion pour la peau, une crème de jour, un déodorant ou anti-transpirant, un produit épilatoire, un produit bronzant ou de protection solaire, un produit pour les ongles, un produit nettoyant pour la peau, un maquillage, un savon, une mousse, une huile ou un gel pour la douche ou le bain, un produit de soin des pieds/mains, un produit d'hygiène, un rafraîchisseur d'air, un rafraîchisseur d'air en poudre « prêt à l'emploi », un produit éliminant les moisissures, un produit d'entretien des meubles, une lingette, un détergent pour la vaisselle ou un détergent pour les surfaces dures, un produit d'entretien du cuir, un parfum d'ambiance pour voiture, un cirage, une cire ou un produit nettoyant pour les plastiques.

15. Procédé pour libérer d'un composé précurseur des composés choisis dans le groupe constitué par
a) un composé carbonyle de formule 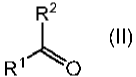 sous la forme de l'un quelconque de ses stéréoisomères ou d'un mélange de ceux-ci, R¹ représentant un groupe hydrocarboné en C₁₋₁₈, comprenant éventuellement un à trois atomes d'oxygène et/ou un à deux atomes d'azote et/ou un atome de soufre ; R² représentant un atome d'hydrogène ou un groupe R¹ ; ou R¹ et R², pris ensemble, formant un groupe cycloalkyle en C₅₋₁₆, cycloalcényle en C₅₋₁₆, hétérocycloalkyle en C₄₋₁₄ ou hétérocycloalcényle en C₄₋₁₄, chacun éventuellement substitué par un ou plusieurs groupes parmi les groupes alkyle en C₁₋₁₅, alcényle en C₂₋₁₅, alcoxy en C₁₋₁₅, cycloalkyle en C₃₋₁₅, cycloalcényle en C₅₋₁₅, aryle en C₆₋₁₀ et/ou aryloxy en C₆₋₁₀, chacun éventuellement substitué par un ou plusieurs groupes parmi les groupes alkyle en C₁₋₈, alcoxy en C₁₋₈, acide carboxylique et/ou ester en C₁₋₄ d'acide carboxylique, l'hétéroatome représentant un ou plusieurs atomes parmi un atome d'oxygène ;
b) une cétone de formule 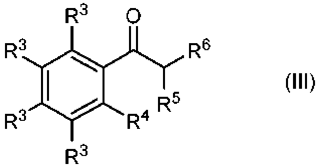 sous la forme de l'un quelconque de ses stéréoisomères ou d'un mélange de ceux-ci, R³ et R⁴ représentant, chacun indépendamment de l'autre, un atome d'hydrogène, un groupe alcoxy en C₁₋₆ ou un groupe alkyle en C₁₋₁₂, éventuellement substitués par un groupe hydroxy, alcoxy en C₁₋₆ ou oxo, ou deux groupes R³ adjacents, pris ensemble, étant un groupe alcanediyle en C₃₋₈ linéaire éventuellement substitué par un ou plusieurs groupes parmi les groupes alkyle en C₁₋₃ et/ou alcoxy en C₁₋₃ ; R⁵ représentant un atome d'hydrogène ou un groupe hydrocarboné en C₁₋₆ ou R⁴ et R⁵, pris ensemble, représentant un groupe alcanediyle en C₁₋₄ linéaire, ramifié ou cyclique, comprenant éventuellement un atome d'oxygène ; R⁶ représentant un atome d'hydrogène ou un groupe méthyle ;
dans lequel le composé précurseur est un composé de formule (I) 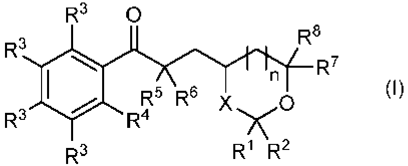 sous la forme de l'un quelconque de ses stéréoisomères ou d'un mélange de ceux-ci, R¹, R², R³, R⁴, R⁵ et R⁶ ayant la même signification que celle définie ci-dessus ; n valant 0 ou 1 ; X étant un atome d'oxygène ou un groupe NR⁹, R⁹ étant un atome d'hydrogène ou un groupe méthyle ; et R⁷ et R⁸ représentant, chacun indépendamment de l'autre, un atome d'hydrogène ou un groupe alkyle en C₁-6 ;
par exposition du composé précurseur de formule (I) à de la lumière et à un environnement dans lequel le composé est hydrolysé.
